# EUROPEAN PATENT SPECIFICATION

(11) **EP 3 867 640 B1**
(45) Date of publication and mention of the grant of the patent: **07.06.2023**
(21) Application number: 19786586.8
(22) Date of filing: 16.10.2019
(51) Int. Cl.: G01N 33/50

(54) **METHOD FOR IDENTIFYING A CHEMICAL COMPOUND OR AGENT INDUCING UBIQUITINATION OF A PROTEIN OF INTEREST**
VERFAHREN ZUR IDENTIFIZIERUNG EINER CHEMISCHEN VERBINDUNG ODER EINES MITTELS, DAS DIE UBIQUITINIERUNG EINES INTERESSIERENDEN PROTEINS INDUZIERT
PROCÉDÉ D'IDENTIFICATION D'UN COMPOSÉ CHIMIQUE OU D'UN AGENT INDUISANT L'UBIQUITINATION D'UNE PROTÉINE D'INTÉRÊT

(30) Priority: 16.10.2018 EP 18200834
(43) Date of publication of application: 25.08.2021
(73) Proprietor: CeMM - Forschungszentrum für Molekulare Medizin GmbH, 1090 Wien (AT)
(72) Inventor: WINTER, Georg, 1140 Wien (AT); MAYOR RUIZ, Cristina, 1090 Wien (AT)
(74) Representative: Maiwald GmbH
(86) International application number: PCT/EP2019/078125
(87) International publication number: WO 2020/079103

(56) References cited:
- EP-A1- 3 000 479
- GROISMAN REGINA ET AL: "The ubiquitin ligase activity in the DDB2 and CSA complexes is differentially regulated by the COP9 signalosome in response to DNA damage", CELL, ELSEVIER, AMSTERDAM, NL, vol. 113, no. 3, 2 May 2003 (2003-05-02), pages 357-367, XP002544420, ISSN: 0092-8674, DOI: 10.1016/S0092-8674(03)00316-7
- EMIL BULATOV ET AL: "Targeting Cullin-RING E3 ubiquitin ligases for drug discovery: structure, assembly and small-molecule modulation", BIOCHEMICAL JOURNAL, vol. 467, no. 3, 17 April 2015 (2015-04-17), pages 365-386, XP055647005, ISSN: 0264-6021, DOI: 10.1042/BJ20141450
- TING HAN ET AL: "Anticancer sulfonamides target splicing by inducing RBM39 degradation via recruitment to DCAF15", SCIENCE, vol. 356, no. 6336, 16 March 2017 (2017-03-16), page eaal3755, XP55646954, ISSN: 0036-8075, DOI: 10.1126/science.aal3755
- AKINJIYAN FAVOUR A ET AL: "Lead discovery and chemical biology approaches targeting the ubiquitin proteasome system", BIOORGANIC & MEDICINAL CHEMISTRY LETTERS, vol. 27, no. 20, 30 August 2017 (2017-08-30), pages 4589-4596, XP085203758, ISSN: 0960-894X, DOI: 10.1016/J.BMCL.2017.08.058
- CHE YE ET AL: "Inducing protein-protein interactions with molecular glues", BIOORGANIC & MEDICINAL CHEMISTRY LETTERS, PERGAMON, AMSTERDAM, NL, vol. 28, no. 15, 19 April 2018 (2018-04-19), pages 2585-2592, XP085433384, ISSN: 0960-894X, DOI: 10.1016/J.BMCL.2018.04.046

## Description

### FIELD

The present invention relates to a method for identifying, obtaining and/or testing a compound or agent, in particular a chemical compound, able to induce ubiquitination of a protein of interest. Further, but not part of the invention, the present disclosure relates to a chemical compound or agent identified and/or tested by the method of the present invention and medical uses of these compounds or agents. Furthermore, but not part of the invention, the present disclosure relates to a method for treating diseases, like cancer, said method for treating comprising administering the chemical compound or agent identified and/or obtainable by the method of the present invention. Moreover, here described but not part of the invention is an isolated eukaryotic cell manipulated to comprise enhanced cullin-RING ubiquitin ligase (CRL) activity, in particular to comprise constantly neddylated CRLs.

### BACKGROUND

Protein degradation plays a central role in many cellular functions such as for cell maintenance and normal function. Accordingly, degradation of proteins, like proteins associated with cellular functions, e.g., maintenance function, has implications for the cell's proliferation, differentiation, and death. While inhibitors of proteins can block or reduce protein activity in a cell, protein degradation in a cell can also reduce activity by removing the target protein. Utilizing a cell's protein degradation pathway can, therefore, provide means for reducing or removing potentially undesired protein activity. One of the cell's major degradation pathways is known as the ubiquitin-proteasome pathway. The ubiquitin-proteasome pathway is a critical pathway that regulates key regulator proteins and degrades misfolded and abnormal proteins, wherein such proteins are marked for degradation by the proteasome by covalent attachment of ubiquitin to the protein.

In particular, the ubiquitin-proteasome pathway mediates the turnover of a wide range of proteins with exquisite specificity within cells. The ubiquitin conjugation on target proteins is mediated by an enzymatic cascade comprised by an E1 ubiquitin-activating enzyme, an E2 ubiquitin-conjugating enzyme and an E3 ubiquitin ligase that attach ubiquitin to the target protein (Hershko et al., Nat. Med. 6, 1073-1081 (2000); Komander et al., Annu. Rev. Biochem. 81, 203-229 (2012)). The specificity of a target protein to be degraded is mediated by the E3 ligase, conferring a highly selective degradation of proteins. Within the E3 ligase family, cullin-RING E3 ubiquitin ligases (CRLs) are the largest subfamily, responsible for around 20% of the protein turnover (Deshaies and Joazeiro; Annu. Rev.Biochem. 78, 399-434 (2009); Petroski and Deshaies; Nat. Rev. Mol. Cell Biol. 6, 9-20 (2005)). CRLs are modular complexes built around a cullin scaffold, which associate with an adaptor protein, a substrate receptor (SR) and a RING protein that recruits the E2 enzyme (Zheng, Nature 416, 703-709 (2002)). In particular, the SR associated with the CRL binds the target protein to be degraded, for example the CRL may bind the target indirecty via a substrate or compound that is directly bound to the SR and said target protein, thus forming a ternary complex comprising the SR of the CRL, the substrate or compound and the target protein. In mammals, more than 200 SRs are known to associate with one of eight cullins and assemble a CRL specific for a single or small collection of target proteins (Bennett et al., Cell 143, 951-965 (2010)).

Critical steps in the regulation of cullin-RING E3 ubiquitin ligases (CRLs) are (1) the activation induced by the covalent attachment of the protein denoted as NEDD8 to the cullin scaffold (neddylation), and (2) the subsequent deneddylation, i.e. dissociation of the NEDD8 from the CRL and association of the CRLs with CAND1 leading to remodeling of the CRL complexes which impairs substrate exchange upon binding to the CRL (Deshaies et al., Subcell Biochem. 54, 41-56 (2010); Pierce et al., Cell 153, 206-215 (2013)). The activating neddylation is mainly catalyzed by NAE1 and UBE2M, and induces a conformational change that enables the transfer of ubiquitin from the E2 enzyme to the target protein, wherein the target protein has been recruited to the substrate receptor either by a particular conformation, a posttranslational modification, or also by a small molecule or metabolite. Specifically, the target protein is recruited by the substrate or the small molecule degrader by binding to the target protein and binding to the SR associated with the CRL forming a ternary complex comprising the substrate, the target protein and the SR assembled with the CRL. As outlined above, since neddylation of CRL is catalyzed by NAE1, UBE2M and others, most CLRs are not neddylated in UBE2M-deficient cells and therefore remain inactive. Thus, UBE2M deficient cells were shown to be resistant to degradation of target proteins that are recruited by small-molecule degraders to the CRL subtypes CRL4^{CRBN}, CRL4^{DCAF15}, or CRL2^{VHL}, wherein CRBN, DCAF15 and VHL refer to the SR that is associated with the respective CRL. The deneddylation reaction is catalysed by the COP9 signalosome (CSN) (Cope and Deshaies; Cell 114, 663-671 (2003); Wei and Deng; Annu. Rev. Cell Dev. Biol. 19, 261-286 (2003); Wolf et al., Nat. Cell Biol. 5, 1029-1033 (2003)) after which CRLs become available for CAND1-mediated substrate receptor exchange. Therefore, CSN is a central component of this dynamic cycle of CRLs allowing their dismantlement and remodeling. Although CSN inhibits CRL activity *in vitro* (Zhou et al., Mol. Cell 11, 927-938 (2003)), it is required for competent CRL activity *in vivo* (Cope and Deshaies; Cell 114, 663-671 (2003)).

The ubiquitin-proteasome pathway system is used for selective protein degradation. For example, heterobifunctional compounds are composed of a target protein-binding moiety and an E3 ubiquitin ligase binding moiety and may induce proteasome-mediated degradation of a target protein/ protein of interest (POI) via recruitment of the POI by the compound to the E3 ubiquitin ligase and subsequent ubiquitiniation of the POI by the E3 ubiquitin ligase. These heterobifunctional compounds offer the possibility of temporal control over protein expression and/or protein activity. Thus, such compounds are capable of inducing the inactivation of a POI upon addition to cells or administration to an animal or human, and could be therefore useful as biochemical reagents and lead to a new paradigm for the treatment of diseases, e.g. by removing pathogenic or oncogenic proteins. Further examples of compounds that may induce selective protein degradation comprise molecular glues, which refer to compounds which can bind the target protein and the E3 ligase ubiquitin ligase through cooperative binding, i.e. these compounds can only bind both, the target protein and the E3 ligase ubiquitin ligase simultaneously in contrast to heterobifunctional proteins, which can also bind either the target protein or the E3 ubiquitin ligase separately.

Applying such compounds for selective target protein degradation is an emerging pharmacologic strategy with immense interest from industry. Thereby, optimization of these compounds is a largely empirical process that is supported by recombinant binding and dimerization data. Methods for the identification of such compounds in the prior art are based on *in vitro* binding affinity assays measuring the affinity of a certain compound to the POI or the E3 ubiquitin ligase, but these *in vitro* assays (i.e. previously employed test systems which are not based on cells and/or cell systems) are not informative for cellular target degradation *in vivo* by an E3 ubiquitin ligase. While *in vitro* assays measuring ternary complex formation with recombinant E3 ligase, compound and target protein are certainly more informative, they also only hold limited predictive value for cellular degradation activity *in vivo* by the E3 ligase given that physiological processes in cells such as cellular uptake, compartmentalization, and compound stability are not considered by *in vitro* assays. Moreover, in assays for identification of compounds able to induce degradation of the POI, the addressed POI is often used in a truncated form, e.g. just the bound domain. Hence, the truncated POI may not be bound to its cognate cellular binding partners and effectors, and post-translational modification of the POI may be impaired. Thus, binding of compounds may be impaired if the POI is not present in its physiological form so that identification of the compounds able induce degradation of the POI maybe hampered.

Besides *in vitro* assays, *in vivo* assays in cells used for the identification of compounds able to induce degradation of a POI typically report on protein abundance of the POI. Despite the detection of POI by methods based on immunoblots, further different strategies for detection of such POI are available, typically by fusing the POI to luciferase or to a fluorescent protein and detecting the POI by the level of luminescence or fluorescence. However, tagging and labeling of the POI may be specific for each POI and thus these cellular assays need to be built individually for each POI to be addressed. Moreover, these assays suffer from the fact that they are measuring the endpoint (protein degradation), while being agnostic to upstream processes such as a lack of cellular permeabilization of the compound, a lack of binding of the compound to either the POI or the E3, and finally (the most frequently observed reason), a lack of productive ternary complex formation comprising the POI, the substrate and the SR of the E3 ubiquitin ligase. Other cellular assays report on compound-induced ternary complex formation (either via nluc complementation, or via BRET), but these assays are labour intensive since transient transfection of a modified POI is required. Further, the assays need to be developled and may need to be optimized for every POI-E3 pair, and also require relatively large tags to be introduced (25-35 kDa), which can obstruct productive E3 ligase formation. Furthermore, those assays do not report on whether or not the dimerization occurs with a SR that is fully incorporated into a productive CRL, or with a monomeric SR, or an SR-adaptor dimer, such the dimer comprising association of CRBN and DDB1 without the association of the respective CRL.

Further reference is made to Groisman et al., Cell 113, 357-367 (2003), Bulatov et al., Biochem. J. 467, 365-386 (2015), EP 3 000 479 A1, Han et al., Science 356, eaal37555 (2017), Favour et al., Bioorganic & Medicinal chemistry letters 27, 4589-4596 (2017), and Ye et al., Bioorganic & Medicinal chemistry letters 28, 2585-2592 (2018).

In view of the above, the technical problem underlying the present invention is the provision of means and methods for identifying chemical and/or biochemical compounds or agents that are able to induce ubiquitination of a target protein/protein of interest (POI), in particular a target protein/POI desired to be degraded in a cell, like a diseased cell.

The solution to this technical problem is provided by the embodiments as defined herein below and as characterized in the claims.

### SUMMARY

The invention relates to the embodiments as defined in the claims. The disclosure relates to the following:
A method for identifying a chemical compound or agent able to induce ubiquitination of a protein of interest
said method comprising:
(i) contacting a chemical compound or agent of interest with a eukaryotic cell comprising and/or expressing the protein of interest and manipulating said cell to comprise enhanced cullin-RING ubiquitin ligase (CRL) activity;
(ii) determining the level of at least one member of an E3 ligase complex present in the cell;
   wherein said chemical compound or agent is identified/determined to induce ubiquitination of the protein of interest if the level of the at least one member of the E3 ligase complex is increased compared to the level determined in the absence of said chemical compound or agent of interest. Said "protein of interest" is also defined as a "target protein", in particular a target protein to be degraded via an ubiquitination. The term "protein of interest" and/or "target protein" as used in this cotext also comprises a plurality of proteins of interest/target proteins. This is also illustrated in the appended examples.

Preferably, the compound/agent to be indentified with the means and methods of the present invention is a chemical compound.

In one embodiment of the present invention, the method as characterized herein above and in the detailed description is a method wherein in step (i) said cullin-RING ubiquitin ligase (CRL) activity is enhanced compared to the cullin-RING ubiquitin ligase (CRL) activity in a control cell that is not manipulated to comprise enhanced CRL activity. Furthermore, it is also an embodiment of the present invention that the inventive method as characterized herein above is a method wherein said (eukaryotic) cell manipulated to comprise enhanced cullin-RING ubiquitin ligase (CRL) activity is a cell that is manipulated to comprise constantly neddylated cullin-RING ubiquitin ligases/CRLs and/or that is manipulated to comprise constant neddylation of cullin-RING ubiquitin ligases/CRLs. As used herein, the term "constantly neddylated" and/or "constant neddylation" means/is equivalent to the term "hyperneddylated" and/or "hyperneddylation". In context of this invention, the terms "constantly neddylated" or "constant neddylation", and "hyperneddylated" or "hyperneddylation" are considered to be equivalent. These terms are known to the person skilled in the art and relate to increased intracellular levels of neddylated cullins (*i.e.* eukaryotic cullins, like CUL1, CUL2, CUL3, CUL4A, CUL4B, CUL5, CUL6 (also comprising for example CUL6 from Drosophila or C elegans), CUL7, in particular human CUL4A, human CUL4B, human CUL1, human CUL2, human CUL3, human CUL5 and/or human CUL7, and in particular human CUL4A and/or human CUL4B).

As is also detailed herein below and as is illustrated in the appended examples, in context of this "neddylation" means and refers to a type of protein modification process by which the ubiquitin-like protein NEDD8 is conjugated to the CRL through E1 activating enzyme (NAE; a heterodimer of NAE1 and UBA3 subunit), E2 conjugating enzyme (Ubc12, UBE2M) and E3 ligase (Gong et al. J. Biol. Chem. 2013; 274: 1203612042). This modification, namely the "neddylation", activates the E3 ligase activity of cullin-RING ubiquitin ligases/CRLs by promoting substrate ubiquitination. "Increased neddylation"/"constant neddylaion"/"hyperneddylation" as used herein refers to a quantitatively and/or qualitatively higher neddylation. Also this is detailed herein below and illustrated in the appended examples. The person skilled in the art is aware that constant neddylation may also lead to destabilization and/or auto-degradation of at least one member of the cullin-RING ubiquitin ligase/CRL, for example, a ligase substrate receptor.

As mentioned above, in context of the present invention "enhanced cullin-RING ubiquitin ligase activity"/ "enhanced CRL activity" means that said cullin-RING ubiquitin ligase activity/CRL activity is enhanced compared to the cullin-RING ubiquitin ligase activity/CRL activity in a control cell that is not manipulated to comprise enhanced cullin-RING ubiquitin ligase activity/CRL activity. Said control cell is preferably of the the same cell type as the cell which was manipulated to comprise enhanced CRL activity. In context of this invention said control cell is also designated as "wildtype cell". Accordingly, and in context of this invention, the present invention relates to a specific *in vivo* method for identifying/determining/obtaining a (chemical) compound or agent, i.e. a compound to be assessed for its potential to induce and/or stimulate the ubiquitination of a protein of interest, wherein in step (i) of the method recited herein above said cullin-RING ubiquitin ligase activity/CRL activity is enhanced compared to the CRL activity in a control cell that is not manipulated to comprise enhanced CRL activity. In this context and in context of this invention, the term *"in vivo"* relates to a method to be applied on isolated cells and/or cell lines as further defined herein below. The term *"in vivo"* in context of the methods of this invention does not rely to a method to be practiced on humans, i.e. a living human individual. Accordingly, the method(s) provided herein is/are method that is based on method wherein preferably isolated cells/cell lines are used and employed and, therefore, the herein claimed methods can also be characterized as an *in vitro* method. This is also evident form the appended examples and the disclosure herein below.

In accordance with the above, the present invention relates in particular to a method for identifying a compound or agent able to induce ubiquitination of a protein of interest (target protein),
said method comprising:
(i) contacting a chemical compound or agent of interest with a eukaryotic cell comprising and/or expressing said protein of interest (target protein) and manipulating said cell to comprise enhanced cullin-RING ubiquitin ligase (CRL) activity,
(ii) determining the level of at least one member of an E3 ligase complex present in the cell;

wherein in step (i) said cullin-RING ubiquitin ligase (CRL) activity is enhanced compared to the cullin-RING ubiquitin ligase (CRL) activity in a control cell that is not manipulated to comprise enhanced CRL activity; and
wherein the chemical compound or agent is determined to induce ubiquitination of the protein of interest (target protein) when the level of the at least one member of the E3 ligase complex is increased (in said cell) compared to the level determined in the absence of the chemical compound or agent.

Most preferably, the compound or agent to be identified with the herein disclosed inventive methods is a chemical compound. Yet, it is to be understood, the term "compound or agent" may also comprise biological and/or biological molecules. Furthermore, the term "identifying" or "to identify" in context of the method of the present invention also comprises "testing" and/or "obtaining" compounds that are able of inducing ubiquitination of (a) protein(s) of interest/target protein(s).

Also disclosed herein but not part of the invention are eukarotic cells manipulated to comprise enhanced cullin-RING ubiquitin ligase (CRL) activity. Furthermore, described herein but not part of the invention are compounds identified/determined and/or obtained with the inventive method whereby these compounds are able to induce ubiquitination of a protein of interest. These compounds are in particular useful as medicaments, for example in the treatment of diseases and/or disorders wherein it is desired to degrade proteins of interest/target proteins via ubiquitination. Accordingly, described herein but not part of the invention are methods of treating such diseases or disorders, said methods comprising the administration to an individual in need of such a treatment with the (chemical) compound or agent identified as capable of inducing ubiquitination of a protein of interest/target protein with the methods of the present invention.

### The figures show:

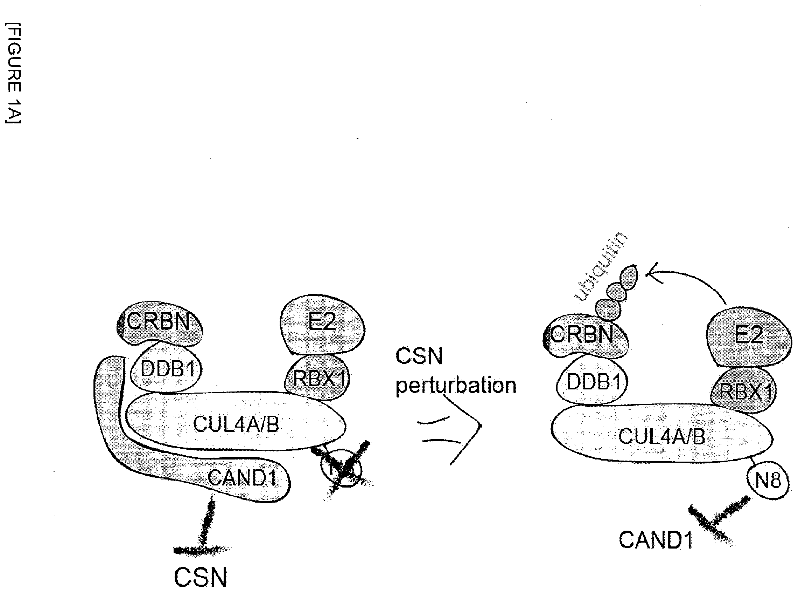

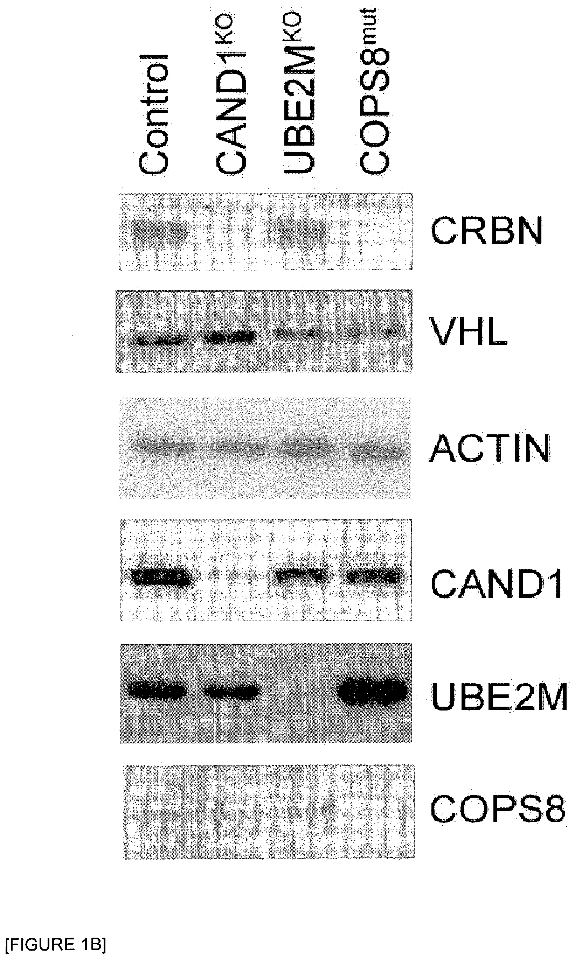

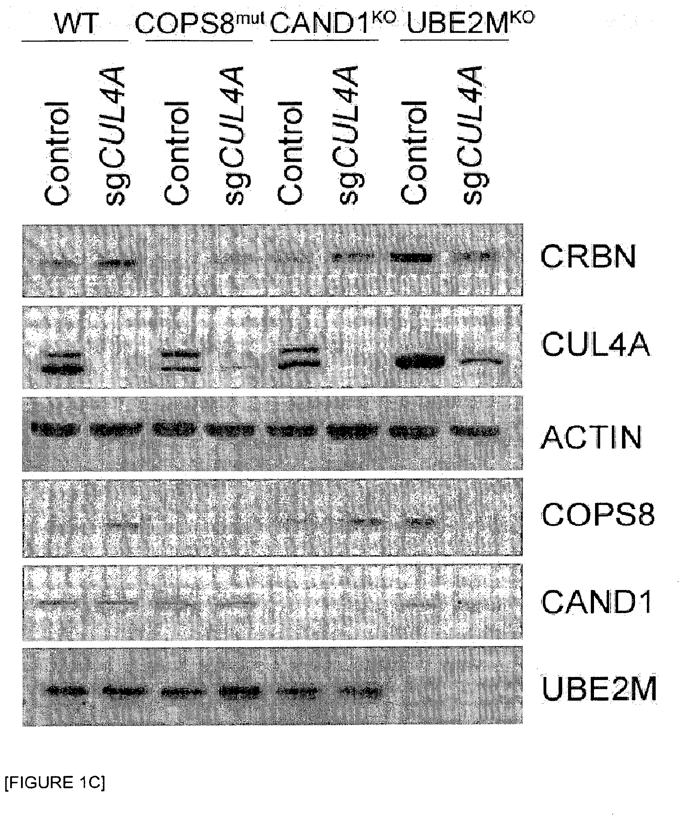

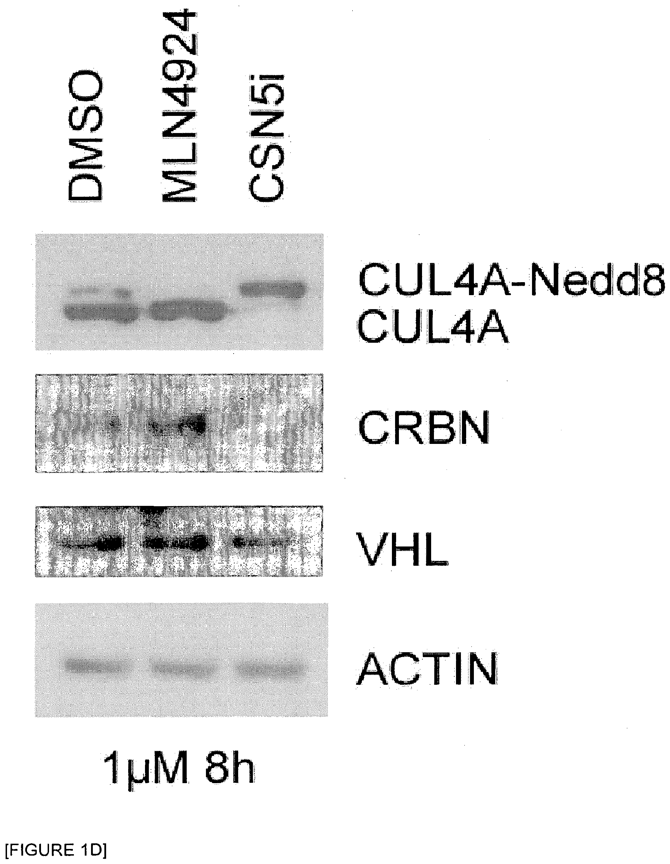

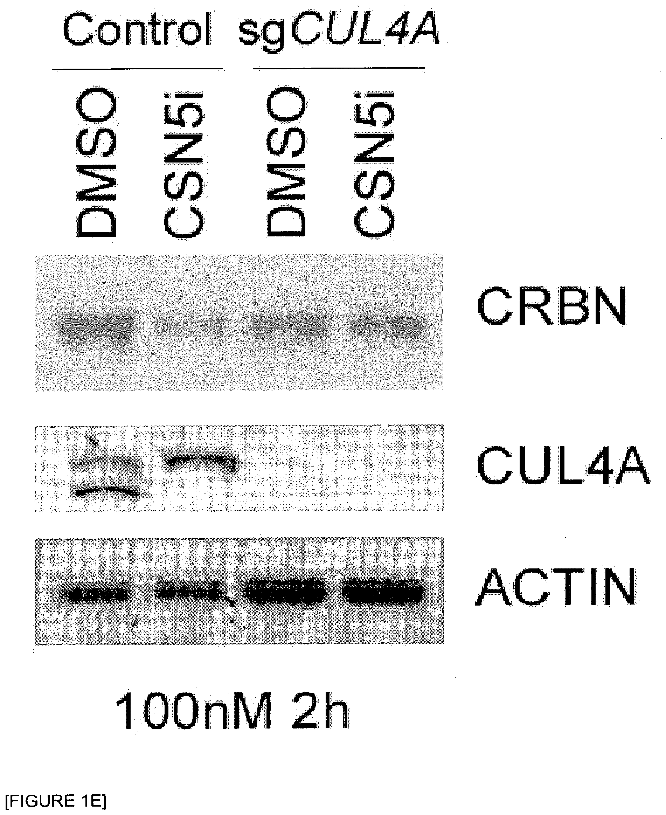

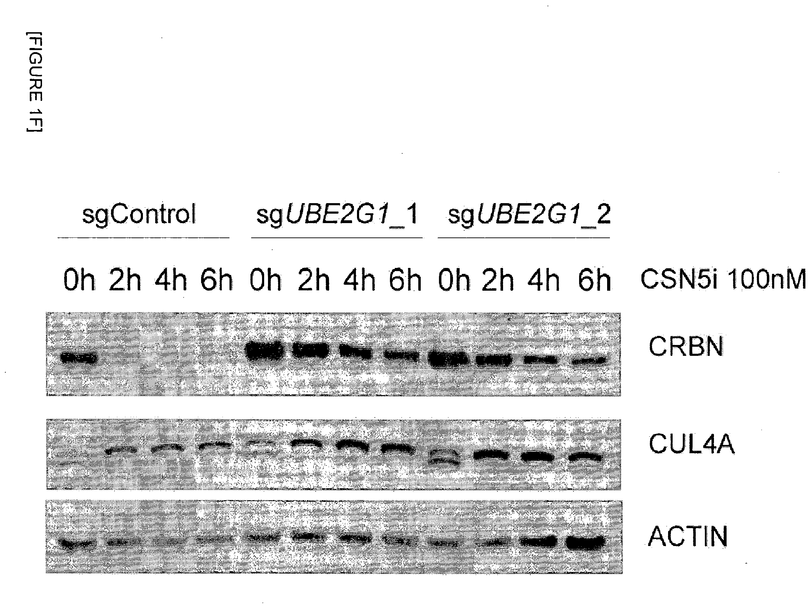

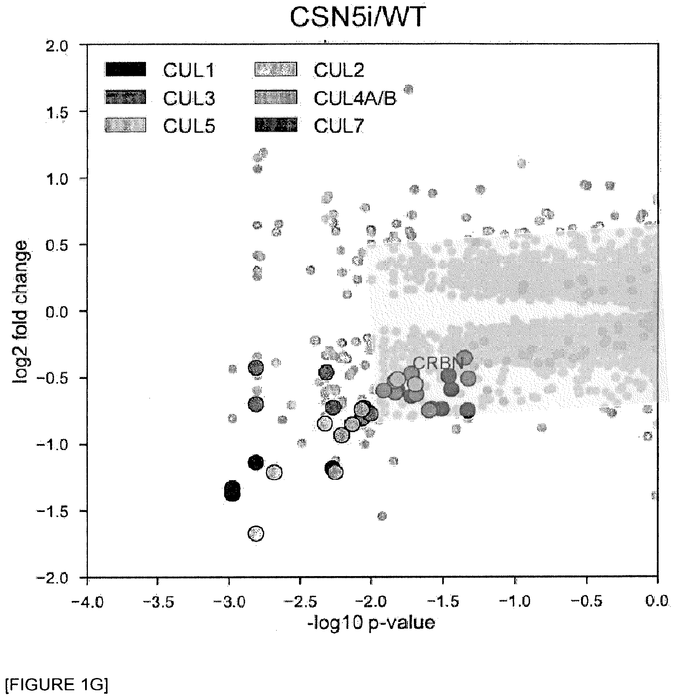

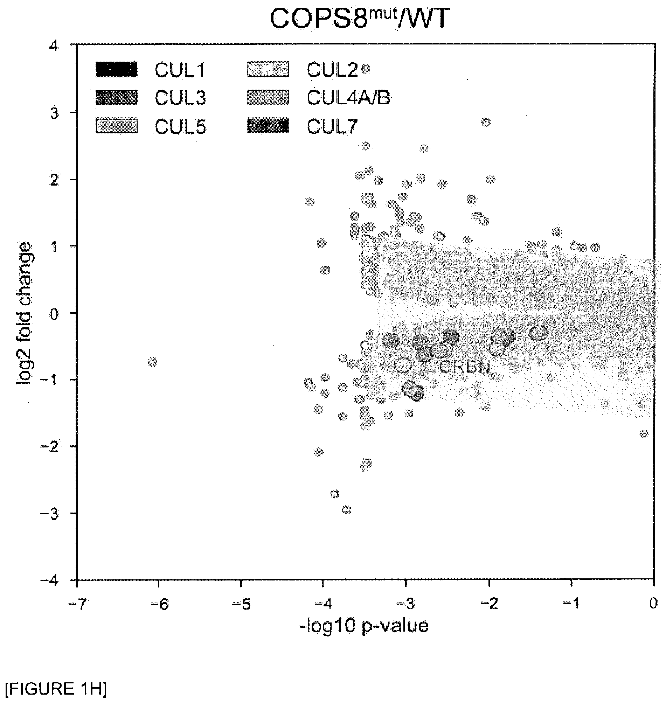

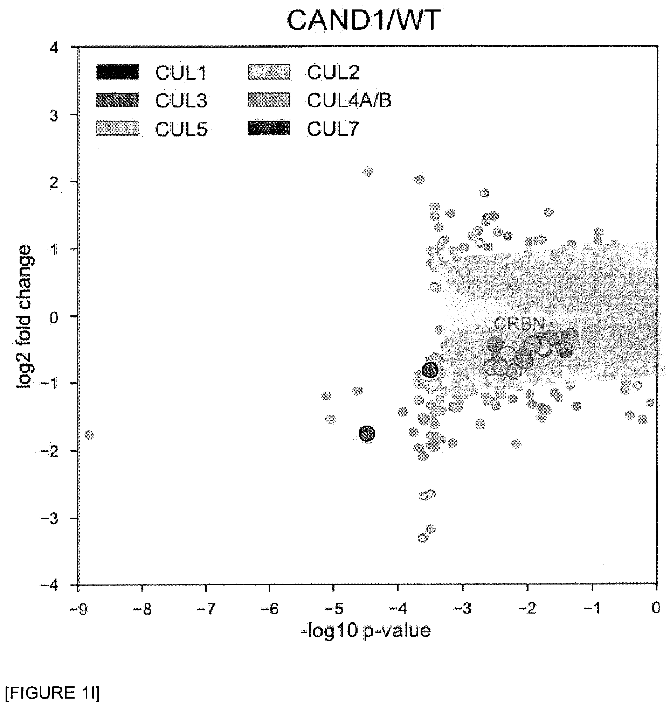

- FIGURE 1:: A. Schematic of the deneddylated (left) and the neddylated (right) CUL4A/B complex bound to the substrate receptor CRBN via the adaptor protein DDB1. CAND1 interacts with the deneddylated form of CUL4A/B and enables subsequent substrate receptor exchange (left). Perturbation of the CSN traps the CRL in the neddylated state by blocking binding of CAND1 and induces autodegradation of the substrate receptor (i.e. CRBN by ubiquitination via the E2 ubiquitin-conjugating enzyme bound to the CUL4A/B via RBX1).
B. Western Blot (WB) of the level of CRBN, VHL, actin, CAND1, UBE2M and COPS8 in control (wild-type KBM7 cells), CAND1 knock-out cells, UBE2M mutant and COPS8 mutant cells. The level of CRBN, the SR of the CRL4^{CRBN} E3 ligase, is downregulated in COPS8 mutant cells and less prominent downregulated in CAND1 knock-out cells. VHL levels, the SR of the CRL2^{VHL} E3 ligase, are not downregulated in control (wild-type cells), CAND1 knock-out cells, UBE2M knock-out cells and COPS8 mutant cells.
C. Western Blot (WB) of the level of CRBN, CUL4A, actin, COPS8, CAND1 and UBE2M in control (wild-type) KBM7 cells, COPS8 mutant cells, CAND1 knock-out cells, UBE2M mutant cells with and without treatment of sgRNA targeting CUL4A (sgCUL4A). Downregulation of CRBN is rescued by the treatment with sgRNA targeting CUL4A.
D. Western Blot (WB) of the level of CUL4A-NEDD8, CUL4A, CRBN, VHL and actin in cells treated with DMSO, MLN4924 or CSN5i-3. Downregulation of CRBN is recapitulated by the treatment with CSN5i-3.
E. Western Blot (WB) of the level of CRBN, CUL4A and actin in DMSO and CSN5i-3 treated cells with and without treatment of sgRNA targeting CUL4A. Downregulation of CRBN is rescued in the CUL4A deficient cells (cells treated with sgRNA targeting CUL4A) treated with CSN5i-3.
F. Western Blot (WB) of the level of CRBN, CUL4A and actin in control (wild-type) cells, UBE2G1_1 and UBE2G1_2 cells all treated with sgRNA targeting UBE2G1 and 100 nM CSN5i-3 after 0, 2, 4 and 6 hours. Downregulation of CRBN after treatment with CSN5i-3 is rescued in cells treated with two sgRNAs targeting UBE2G1.
G. Log₂ fold change in abundance of [8359 proteins] including the SRs related to CUL1, CUL2, CUL3, CU4A/B, CUL5 and CUL7 versus -log₁₀ p-value comparing KBM7 cells treated with CSN5i-3 to KBM7 cells treated with vehicle (DMSO).
H. Log₂ fold change in abundance of [8359 proteins] including the SRs related to CUL1, CUL2, CUL3, CU4A/B, CUL5 and CUL7 versus -log₁₀ p-value comparing COPS8^{mut} KBM7 cells to wild type (WT) KBM7 cells.
I. Log₂ fold change in abundance of [8359 proteins] including the SRs related to CUL1, CUL2, CUL3, CU4A/B, CUL5 and CUL7 versus -log₁₀ p-value comparing CAND1 knock-out cells to wild type (WT) cells.

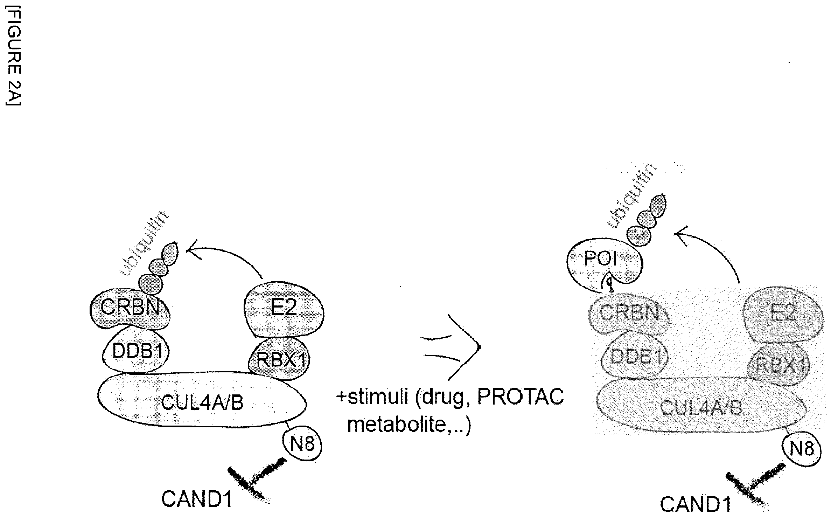

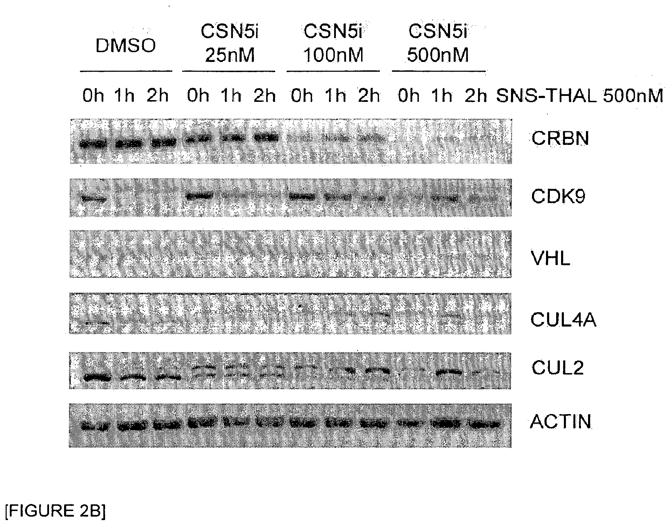

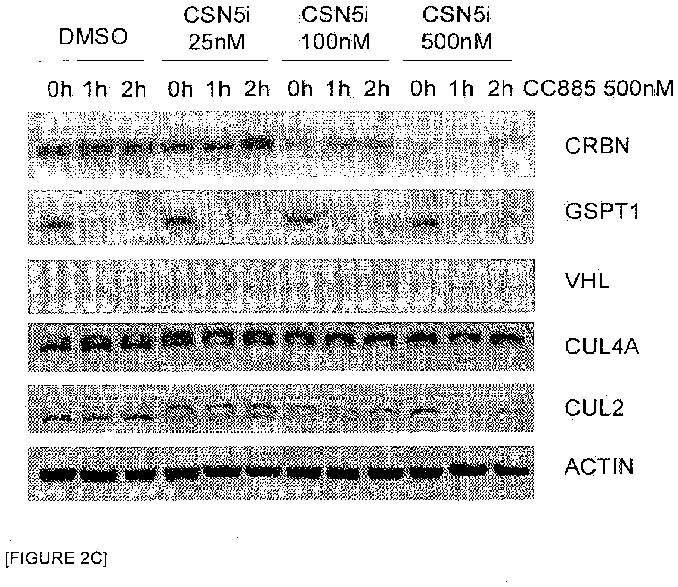

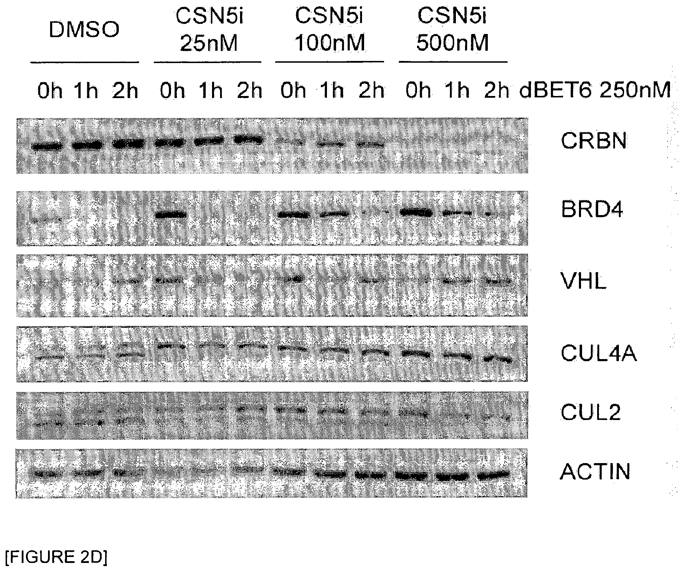

- FIGURE 2:: A. Schematic of the constantly neddylated/hyperneddylated CUL4A/B complex bound to the substrate receptor CRBN via the adaptor protein DDB1 (left). Perturbation of the CSN traps the CUL4A/B in the neddylated state and induces autodegradation of the substrate receptor CRBN by ubiquitination via the E2 ubiquitin-conjugating enzyme bound to the CUL4A/B via RBX1. Schematic of the stabilization of the CRBN upon chemical compound or agent availability (right). The chemical compound or agent recruits a POI to the CRL, wherein the chemical compound or agent delineates the autodegradation of the CRBN by shielding the CRBN from the E2-mediated ubiquitin transfer. Conjugation of ubiquitin to the POI by E2 ubiquitin-conjugating enzyme confers degradation of the POI.
B. Western Blot (WB) of the level of CRBN (substrate receptor of CUL4A), CDK9 (POI), VHL (substrate receptor of CUL2), CUL4A, CUL2 and actin in wild type cells pretreated with DMSO, 25nM, 100nM or 500nM CSN5i-3. Cells were treated with 500nM of the compound SNS-THAL-032 at the indicated exposure times. CRBN was stabilized upon SNS-THAL-032 treatment in cells treated with DMSO, 25 nM and 100 nM CSN5i-3.
C. Western Blot (WB) of the level of CRBN (substrate receptor of CUL4), GSPT1 (POI), VHL, CUL4A, CUL2 and actin in wild type cells pretreated with DMSO, 25nM, 100nM and 500nM CSN5i-3. Cells were treated with 500nM of the compound CC-885 at the indicated exposure times. CRBN was stabilized upon CC-885 treatment in cells treated with DMSO, 25 nM, 100 nM and 500 nM CSN5i-3.
D. Western Blot (WB) of the level of CRBN, BRD4 (POI), VHL, CUL4A, CUL2 and actin in wild type cells pretreated with DMSO, 25nM, 100nM and 500nM CSN5i-3. Cells were treated with 500nM of the compound dBET6 at the indicated exposure times. CRBN was stabilized upon dBET6 treatment in cells treated with DMSO, 25 nM and 100 nM CSN5i-3.
- FIGURE 3:: Western Blot (WB) of the level of CRBN, CUL4A, GSPT1 (POI) and actin in wild type cells pretreated with DMSO or 125 nM of the molecular glue CC-885 for 15 or 30 minutes before cells were treated with 100 nM CSN5i-3 (chemical inhibition of CSN). CRBN was stabilized upon CC-885 treatment in the presence of CSN5i-3 (lines 4 and 6 of the Western Blot).
- FIGURE 4:: Comparison of heterobifunctional compounds such as PROTAC^{®}s versus molecular glue in a CRL.
- FIGURE 5:: Schematic representation of destabilization of the substrate receptor CRBN and stabilization upon neosubstrate recruitment via the molecular glue.
- FIGURE 6:: Overview of two approaches for the identification of novel molecular glues and novel CRLs that can be hijacked.
- FIGURE 7:: Dose-ranging cellular viability assays in isogenic human leukemia cells (KBM7) with truncations or focal deletions in CAND1, COPS8 or UBE2M. Compared are the CRBN-based PROTAC^{®} dBET6, the DCAF15-based MG indisulam and the BET inhibitor JQ1.
- FIGURE 8:: Dynamically regulated Induction of SR auto-degradation. For a set of SRs, auto-degradation could only be initiated in starved conditions, suggesting that in non-starved setting, these SRs might have been occupied by endogenous substrate.
- FIGURE 9:: Schematic depiction of the workflow of strategy to find novel MGs via time-resolved measuring of differential auto-degradation.
- FIGURE 10:: Targeting strategy for knocking-in HiBit-HA sequence at the endogenous DCAF15 locus.
- FIGURE 11:: Schematic explanation employed (HiBit-based) luciferase assays (either lytic detection where luciferase complementation is achieved by adding recombinant protein, or live-cell where the luciferase complementation is achieved by expressing the luciferase complementation protein LgBit in cells). Live cell imaging can be conducted by knocking in the HiBit sequence (see Fig10) at the endogenous locus, or by ectopically overexpressing HiBit-DCAF15 fusion protein via a viral vector (see Fig 12).
- FIGURE 12:: Map of lentiviral vector for ectopic overexpression of HiBit-HA-DCAF15 fusion. Vector is employed for virus production in 293T cells with standard packaging plasmids. Viral particles are harvested and used to transduce target cells of interest.
- FIGURE 13:: DCAF15 destabilization after CSN5i-3 treatment. Quantified in live cells that have HiBit-HA Knockin at the DCAF15 locus.
- FIGURE 14:: DCAF15 destabilization after CSN5i-3 treatment. Quantified via lytic detection in cells that have HiBit-HA Knockin at the DCAF15 locus.
- FIGURE 15:: DCAF15 re-stabilization in (HiBit-knock in) live cells upon treatment with CSN5i-3 and the known DCAF15 binder indisulam compared to cells treated with CSN5i-3 and DMSO (vehicle control).
- FIGURE 16:: DCAF15 destabilization after CSN5i-3 treatment. Quantified via lytic detection (as described above) in cells that have ectopic overexpression of HiBit-HA-DCAF15.
- FIGURE 17:: DCAF15 re-stabilization in live cells upon treatment with CSN5i-3 and the known DCAF15 binder indisulam as well as a novel DCAF15 binder found via this method (dCEMM.1), compared to cells treated with CSN5i-3 and DMSO (vehicle control).
- FIGURE 18:: Results of the high-throughput screen. The curves show the relative luciferase signal (approximating DCAF15 levels) after individual drug treatments at 10 uM as a function of time. As expected, a significant stabilization of DCAF15 over time after cellular treatment with the known DCAF15-based molecular glue indisulam was observed. While the vast majority of tested compounds did not yield a measurable DCAF15 stabilization (200 inactive compounds displayed, all in light gray), a significant stabilization with two test compounds here named "dCeMM.1" and "dCeMM.2" was observed.
- FIGURE 19:: Chemical structure of indisulam and the two identified hits.
- FIGURE 20:: Western Blot analysis of RBM39- and actin loading control levels after 12 or 16 h treatment of KBM7 wildtype cells ("WT") that have not been engineered to carry additional mutations, or after treatment with KBM7 cells that were engineered to be deficient for DCAF15 via CRISPR/Cas9 technology. dCeMM.1 led to a significant destabilization of RBM39 levels that was comparable to destabilization achieved with indisulam. As predicted from a glue-type molecule, compound effects are dependent on DCAF15 expression.
- FIGURE 21:: Western Blot analysis of RBM39- and actin loading control levels after 10 h treatment of 293T cells that are deficient for DCAF15 ("DCAF15 KO"), or after treatment of cells that have been reconstituted to express nLUC-tagged DCAF15 (the same cells that have been used for the initial screen as outlined in Figure 18). dCeMM-2 led to a significant destabilization of RBM39 levels that was comparable to destabilization achieved with indisulam. As predicted from a glue-type molecule, compound effects are dependent on DCAF15 expression.
- FIGURE 22:: Five known degraders all depend on the enzyme UBE2M for their cytotoxic activity in the leukemic cell line KBM7.
- FIGURE 23:: Initial hits in the differential viability screen where 2000 small molecules were tested for their anti-proliferative activity in KBM7 wildtype cells ("WT") and KBM7 cells mutant for UBE2M.
- FIGURE 24:: (A) Cellular viability of KBM7 cells (WT or UBE2M mutant) after treatment with dCeMM.1. (72 hour treatment) (B) cellular viability of KBM7 cells (WT or DCAF15 knockout) after dose-ranging treatment with dCeMM.1 (72 hour treatment).

### DETAILED DESCRIPTION

The present invention provides a method for identifying a compound or agent able to induce ubiquitination of a protein of interest (target protein),
the method comprising:
   (i) contacting a chemical compound or agent of interest with a eukaryotic cell comprising and/or expressing the protein of interest (target protein) and manipulating said cell to comprise enhanced cullin-RING ubiquitin ligase (CRL) activity;
   (ii) determining the level of at least one member of an E3 ligase complex present in the cell;
wherein said chemical compound or agent is identified to induce ubiquitination of the protein of interest/target protein if the level of the at least one member of the E3 ligase complex is increased compared to the level determined in the absence of said chemical compound or agent of interest.

Preferably, said "compound or agent" to be indentified is a chemical compound. This term also comprises a biological compound. As used in context of this invention, the term "identifying" or "to identify" in context of the method of the present invention also comprises "testing" and/or "obtaining" compounds that are able of inducing ubiquitination of (a) proteins of interest/target protein(s). Furthermore, in context of the means and methods of this invention, the term "protein of interest" or "target protein" to be ubiquitinated (and thereby finally degraded) also relates to a plurality of proteins , i.e. also to "proteins of interest" and to "target proteins".

In one embodiment of the present invention, the method as characterized herein above and in the detailed description is a method wherein in step (i) said cullin-RING ubiquitin ligase (CRL) activity is enhanced compared to the cullin-RING ubiquitin ligase (CRL) activity in a control cell that is not manipulated to comprise enhanced CRL activity.

Accordingly, the present invention also relates to a method for identifying a compound or agent able to induce ubiquitination of a protein of interest (target protein), said method comprising:
(i) contacting a chemical compound or agent of interest with a eukaryotic cell comprising and/or expressing said protein of interest (target protein) and manipulating said cell to comprise enhanced cullin-RING ubiquitin ligase (CRL) activity,
(ii) determining the level of at least one member of an E3 ligase complex present in the cell;
   wherein in step (i) said cullin-RING ubiquitin ligase (CRL) activity is enhanced compared to the cullin-RING ubiquitin ligase (CRL) activity in a control cell that is not manipulated to comprise enhanced CRL activity; and
   wherein the chemical compound or agent is determined to induce ubiquitination of the protein of interest (target protein) the level of the at least one member of the E3 ligase complex is increased (in said cell) compared to the level determined in the absence of the chemical compound or agent (i.e. in a negative control wherein the compound or agent is not contacted with/administered to said eukaryotic cell and/or to a corresponding control cell/cell line).

Accordingly, the present invention also relates, in one embodiment of the present invention, to a method wherein in step (i) of the method described herein above said cullin-RING ubiquitin ligase (CRL) activity is enhanced compared to the cullin-RING ubiquitin ligase (CRL) activity in a control cell that is not manipulated to comprise enhanced CRL activity.

Furthermore, it is also an embodiment of the present invention that the inventive method as characterized herein above is a method wherein said (eukaryotic) cell manipulated to comprise enhanced cullin-RING ubiquitin ligase (CRL) activity is a cell that is manipulated to comprise constantly neddylated cullin-RING ubiquitin ligases/CRLs and/or that is manipulated to comprise constant neddylation of cullin-RING ubiquitin ligases/CRLs.

As used herein, the term "constantly neddylated" and/or "constant neddylation" means/is equivalent to the term "hyperneddylated" and/or "hyperneddylation". In context of this invention, the terms "constantly neddylated" or "constant neddylation", and "hyperneddylated" or "hyperneddylation" are considered to be equivalent. These terms are known to the person skilled in the art and relate to increased intracellular levels of neddylated cullins (i.e. eukaryotic cullins, like CUL1, CUL2, CUL3, CUL4A, CUL4B, CUL5, CUL 6 [also comprising CUL6 from e.g. Drosophia or C. elegans], CUL7, in particular human CUL4A, human CUL4B, human CUL1, human CUL2, human CUL3, human CUL5 and/or human CUL7, and in most preferred embodiments human CUL4A and/or human CUL4B). As is also detailed herein below and as is illustrated in the appended examples, in context of this "neddylation" means and refers to a type of protein modification process by which the ubiquitin-like protein NEDD8 is conjugated to the CRL through E1 activating enzyme (NAE; a heterodimer of NAE1 and UBA3 subunit), E2 conjugating enzyme (Ubc12, UBE2M) and E3 ligase (Gong et al. J. Biol. Chem. 2013; 274: 1203612042). This modification, namely the "neddylation", activates the E3 ligase activity of cullin-RING ubiquitin ligases/CRLs by promoting substrate ubiquitination. "Increased neddylation"/"constant neddylaion"/"hyperneddylation" as used herein refers to a quantitatively and/or qualitatively higher neddylation. Also this is detailed herein below and illustrated in the appended examples. The person skilled in the art is aware that constant neddylation may also lead to destabilization and/or auto-degradation of at least one member of the cullin-RING ubiquitin ligase/CRL, for example, a ligase substrate receptor.

In context of the methods of the present invention and as also evident for the skilled person, the term "enhanced cullin-RING ubiquitin ligase activity"/ "enhanced CRL activity" means that said cullin-RING ubiquitin ligase activity/CRL activity is enhanced compared to the cullin-RING ubiquitin ligase activity/CRL activity in a control cell that is not manipulated to comprise enhanced cullin-RING ubiquitin ligase activity/CRL activity. Said control cell is preferably the of the same cell type as the cell which was manipulated to comprise enhanced CRL activity. In context of this invention said control cell is also designated as "wildtype cell". Accordingly, the present invention relates to an (*in vivo*) method for identifying/determining a chemical compound or agent, i.e. a compound to be asssed for its potential to induce and/or stimulate the ubiquitination of a protein of interest, wherein in step (i) of the method recited herein above said cullin-RING ubiquitin ligase activity/CRL activity is enhanced compared to the CRL activity in a control cell that is not manipulated to comprise enhanced CRL activity. It is of note that in context of this invention, the terms "enhanced cullin-RING ubiquitin ligase activity"/ "enhanced CRL activity" also comprise "augmented cullin-RING ubiquitin ligase activity"/ "augmented CRL activity". Details on this enhancement/augmentation are provided herein below and said enhancement/ augmentation is also illustrated in the appended Examples.

In accordance with the above, the present invention relates to a method for identifying a (chemical) compound or agent able to induce ubiquitination of a protein of interest/target protein,
said method comprising:
   (i) contacting said (chemical) compound or agent with a eukaryotic cell and manipulating said cell to comprise enhanced cullin-RING ubiquitin ligase (CRL) activity compared to the CRL activity in the absence of manipulating the cell to comprise enhanced CRL activity, wherein said cell expresses the protein of interest/target protein;
   (ii) determining the level of at least one member of an E3 ligase complex present in the cell;
wherein the chemical compound or agent is determined to induce ubiquitination of the protein of interest/target protein if the level of the at least one member of the E3 ligase complex is increased compared to the level determined in the absence of the chemical compound or agent.

The method(s) of the present invention is/are to be considered as a method which can be carried out *in vitro* as well as *in vivo.* It is understood by the person skilled in the art that the term *"in vivo"* in context of the present invention is limited to the use of this method on isolated cells and isolated tissues as well as to the use of the claimed method on ethical animal test systems, like xenografts in test animals or on transgenic test animals, like transgenic mice or rats. As is also evident form the disclosure content of this invention as well as from the appended examples, the methods for identifying a chemical compound or agent able to induce ubiquitination of a protein of interest as disclosed here are restricted to the use of these methods "in vitro" and to "in vivo" uses which do not comprise the "in vivo" use on human beings and/or on animals, besides selected test and/or laboratory animals as recited herein above. Such test animals or also transgenic animals may comprise, mice, rats and the like but may also comprise test animals/transgenics like Drosphila or C.elegans (both for example comprising CUL6). Preferably, the methods of the present invention are to be used on and with (a) cell line(s)/cellular systems which are based on established cell lines. Accordingly, the present invention provides for (living) test cell systems/ (living) cellular systems useful in methods for identifying, testing, obtaining and/or screening compounds/agents able to induce ubiquitination of proteins of interest/target proteins.

As disclosed herein and mentioned above, the terms "compound or agent" or "chemical compound or agent" may also comprise biological and/or biological molecules. Said "compound or agent" to be identified in accordance with the method of this invention may be suspected to be able to induce ubiquitination of a protein of interest and the method of the present invention is, accordingly, to be used to verify and/or test the capacity of said compound to induce ubiquitination of a protein of interest/target proteins. The term "compound or agent" also comprises a plurality and/or mixture of "compounds or agents".

Again, in all methods of the present invention, the term "identifying" or "to identify" also comprises "testing" and/or "obtaining" compounds that are able of inducing ubiquitination of a protein of interest. Therefore,e, the term "identifying" or "to identify" in context of the method(s) of the present invention also comprises "testing" and/or "obtaining" compounds that are able of inducing ubiquitination of (a) proteins of interest/target protein(s).

The present invention provides an assay for identifying a (chemical) compound or agent able to induce ubiquitination of a protein of interest. As is evident from the the disclosure herein and from the appended examples and figures, the method for identifying a chemical compound or agent able to induce ubiquitination of a protein of interest as provided herein comprises the determination of the level of an at least one member of the E3 ligase complex in a eukaryotic cell manipulated to comprise enhanced CRL activity in the absence and presence of the chemical compound or agent. The enhanced CRL activity in the cell is induced by genetical or chemical manipulation of the cell as described below. In the absence of the chemical compound or agent, the enhanced CRL activity of the cell leads to ubiquitination and degradation of the at least one member of the E3 ligase complex by the CRL, a process denoted as autodegradation and exemplified in the appended examples. The term "auto-ubiquitination" refers to the E3 ligase mediated ubiqutination of at least one member of said E3 ligase complex. In other words, by autoubiquitination of the at least one member of the E3 ligase is meant that at least one member of the E3 ligase complex is ubiquitinated by said E3 ligase complex. Particularly, at least one ubiquitin group of the E3 ligase complex may be transferred to the at least one member of said E3 ligase complex. Upon ubiquitination of the at least one member of the E3 ligase complex, the E3 ligase complex may degrade said at least one member of the E3 ligase complex. In other words, the E3 ligase complex "self-ubiquitinates" at least one member of said E3 ligase, thereby destabilizing said at least one member which may result in degradation of said at least one member. This degradation of at least one member of the E3 ligase complex by the same E3 ligase complex also refers to "autodegradation" of the at least one member of the E3 ligase complex.

Such at least one member of the E3 ligase complex, i.e. at least one member of the E3 ligase complex to be ubiquitinated and/or autodegradaded, may be at at least one member of the E3 ligase complex as described herein and in context of the present invention. Said at least one member of the E3 ligase complex may be at least one member which is in molecular proximity of the E3 ligase and which is able to be ubiquitinated by the E3 ligase and/or is degradable by the E3 ligase. For example, the at least one member of the E3 ligase complex may include but is not limited to a substrate receptor or an adaptor protein of the E3 ligase complex. As illustrated in the appended Example, the at least one member of the E3 ligase complex that is autoubiquitinated may be a substrate receptors, such as DCAF15 or cereblon.

A chemical compound or agent contacted with the cell may be able to induce molecular proximity between a component of a CRL E3 ligase complex and a protein of interest which may be bound to the chemical compound or agent or which may be part of a ternary complex. As shown in the examples, it has been surprisingly found that a chemical compound/biochemical compound or agent bound to the protein of interest may be able to impair the autodegradation of the at least one member of the E3 ligase complex by hijacking the at least one member of the E3 ligase complex, e.g. by proximity or binding of the chemical compound or agent contacted with the protein of interest to the at least one member of the E3 ligase complex. Thus, as shown in the examples, the autodegradation of the at least one member of the E3 ligase complex may be impaired in that the protein of interest may be ubiquitinated by the CRL rather than the at least one member of the E3 ligase complex.

Thus, in context of the present invention it has been surprisingly found that determining and correlating the level of the at least one member of the E3 ligase complex in the absence and presence of the chemical compound or agent (to be identified/tested/evaluated in context of the the methods of the present invention) can be used to identify if the chemical compound or agent is able to induce ubiquitination of the protein of interest. In particular, if the level of the at least one member of the E3 ligase complex is increased compared to the level determined in the absence of the chemical compound or agent, it is indicative that the autodegradation of the at least one member of the E3 ligase complex is impaired in the presence of the chemical compound or agent. In this case, the chemical compound or agent is determined to induce ubiquitination of the protein of interest. For example, a chemical compound, biochemical compound or agent may bind to the E3 ligase complex and the POI. As another example, a chemical compound or agent may alter the function of a target protein, e.g. by modifying posttranslational changes of a target protein. A posttranslational modification may include but is not limited to the phosphorylation status of a protein, e.g. a tyrosine kinase phosphorylating a protein. Thus, a chemical compound or agent may induce ubiquitination of a POI, e.g., by modifying a POI in that the POI becomes accessible for a E3 ligase, thereby a chemical compound or agent may not associate with a POI and/or a E3 ligase. In context of this invention, the term "biochemical compound" also comprises, but is not limited to chemical compounds which comprise biological/biochemical parts, like, *inter alia,* peptide linkers bound to said chemical compounds.

In one embodiment, the present invention relates to a method for identifying a chemical compound or agent for its capacity to induce ubiquitination of a protein of interest/target protein, wherein said
method comprises the following steps:
   (i) contacting a chemical compound or agent of interest with a eukaryotic cell expressing said protein of interest/target protein and manipulated to comprise enhanced cullin-RING ubiquitin ligase (CRL) activity;
   (ii) determining the level of at least one member of an E3 ligase complex present in the cell;
wherein said (chemical) compound or agent is identified to be able to induce ubiquitination of the protein of interest/target protein if the level of the at least one member of the E3 ligase complex is increased in said manipulated eukaryotic cell compared to the level of the at least one member of the E3 ligase complex in said manipulated cell determined in the absence of said chemical compound or agent.

In accordance with this invention, the assessment of the "increased level of the at least one member of the E3 ligase complex" in the manipulated eukaryotric cell may comprise the comparative measurement of
(i) (a) manipulated cell(s) which is/are contacted with the test compound; and of
(ii) the same cell(s)/same kind of manipulated cells which have not been contacted with said test compound.

By said "test compound" the "chemical compound or agent suspected to induce ubiquitination of a protein of interest or tested for its capacity to induce ubiquitination of a protein of interest or tested/suspected to recruit the POI/target protein to the E3 ligase" is meant in context of this invention. The definitions for "compound or agent" as provided herein above apply here *mutatis mutantis* and the term "test compound" also relates to (a) mixture(s) of compounds, like "test compounds" in a compound collection.

In context of this invention, the term "induce ubiquitination of a protein of interest" means that the test compound ((chemical) compound or agent) may act directly or indirectly. The term "inducing ubiquitination"" also comprises "facilitating ubiquitination" and/or "enabeling ubiquitination". For example, said test compound/chemical compound/agent may facilitate the recognition of a protein of interest/target protein/POI by the E3 ligase complex or may facilitate ubiquitination even without physically engaging the protein of interest/target protein/POI at the same time. The test compound/chemical compound/agent may also enable said recognition of a POI by the E3 ligase complex.

A further non-limiting option of the "induction of ubiquitination of a protein of interest" may comprise the conformational change of the protein of interest/target protein/POI that has been induced as a direct consequence of binding/interaction with said test compound/chemical compound/agent inducing the ubiquitination of the protein of interest/target protein/POI.

As discussed herein above, the term "(chemical) compound or agent of interest" also relates to a test compound that is to be assessed for its capability/ablity to induce ubiquitination of a protein of interest/target protein. Said "(chemical) compound or agent of interest" may be a compound that may induce the ubiquination of a protein of interest directly or indirectly (for example without physically binding to the E3 ligase and the protein of interest/target protein at the same time). Said "(chemical) compound or agent of interest" or said compound to be tested may also be assessed for its capacity to bring the E3 ligase in contact with the protein of interest, i.e. a protein to be degraded. The "chemical compound or agent to be identified/determined to induce ubiquitination of the POI" may be a test compound to be assessed for its capacity to augment the intracellular ubiquitination system, in particular for its capacity to augment the degradation of a protein of interest/target protein via E3 ubiquitin ligase. As mentioned above and as illustrated in the appended Examples, said "(chemical) compound or agent" may also be a compound that is capable of bringing the E3 ligase complex in close proximity to the protein of interest/target protein (i.e. the protein to be degraded via ubiquination), whereby, also in context of this invention, such compounds or agents may be designated as "molecular glues". The "chemical compounds agents to be tested and/or assessed" for their capacity to induce ubiquitination of a protein of interest may also comprise heterobifunctional degraders, like Proteolysis Targeting Chimeras (PROTAC^{®}) or heterobifunctional compounds composed of a target protein-binding ligand and an E3 ubiquitin ligase ligand. Accordingly, "chemical compounds or agents"to be tested with the herein disclosed inventive methods may comprise but are not limited to PROTAC^{®} compounds or molecular glues. The term chemical "compounds/agents" also comprises libraries and mixtures of such compounds/agents. Therefore with the means and methods of the present invention also mixtures of compounds may be tested.

The "protein of interest"/"target protein"/"POI" in context of this invention is a protein which is desired or is desirable to be degraded in an *in vivo* or *in vitro* situation, for example in a diseased cell, like a cancer cell. Particular proteins of interest (POIs) are, in one specific embodiment, proteins that are the cause, the driver and/or the maintaining entity of a malignancy, disease, or a diseased status. Such POIs may comprise proteins that are overexpressed and/or overactive in a diseased cell, like in a cancer cell. Accordingly, in one embodiment, the POI is involved in the cause, development and/or maintainance of the diseased status of a cell and/or a tissue. Potential POIs/target proteins are also discussed herein below and illustrative, non-limited examples are provided herein below. A "POI" illustrated in the non-limiting examples is BRD4 (a bromodomain protein often required for the expression of tumor driving oncogenes, like Myc).

In context of this invention the term "cullin-RING ubiquitin ligase" is also abbreviated by "CRL". These terms are used herein interchangeably. The term "manipulating [the eukaryotic] cell to comprise enhanced cullin-RING ubiquitin ligase (CRL) activity compared to the CRL activity in the absence of manipulating the cell to comprise enhanced CRL activity" means that a given cell, for example a cancer cell, is manipulated via recombinant or chemical methods to obtain in said cell an enhanced cullin-RING ubiquitin ligase (CRL) activity as compared to a corresponding cell that is not manipulated in the same way, i.e. a cell that does not have an enhanced cullin-RING ubiquitin ligase (CRL) activity. As documented herein below and in the appended examples, such an "enhanced cullin-RING ubiquitin ligase (CRL) activity" may be achieved recombinantly e.g. by overexpression of at least one member of the E3 ligase complex, such as the substrate receptor, for example CRBN. Another illustrative example for said "manipulation of an eukaryotic cell to comprise enhanced cullin-RING ubiquitin ligase (CRL) activity" comprises the inactivation of CAND1, CAND2 or CAND1 and CAND2 for example via recombinant technologies. Non-limiting example of such a technology is the transduction of the eukaryotic cell with inhibitory RNAs, like siRNS or sgRNA.

A chemical method to achieve "enhanced cullin-RING ubiquitin ligase (CRL) activity" comprises (but is not limited to) contacting the cell with a specific CSN inhibitor, like CSN5i-3 (a potent, selective and orally available inhibitor of CSN5, inhibiting CSN-catalysed Cul1 deneddylation as described in Schlierf (2016) NatCommun 7, 13166. doi: 10.1038/ncomms13166). Further examples how the eukaryotic cell may be manipulated in order to achieve enhanced cullin-RING ubiquitin ligase (CRL) activity are provided herein below and illustrated in the appended examples. Means and methods how the enhanced CRL activity may be determined are means and methods known in the art and comprise, *inter alia,* immunoassays (like Western blots, ELISA tests and the like) and/or reporter assay (like luciferase assays and the like). Accordingly, and as disclosed herein, the eukaryortic cell manipulated to comprise enhanced CRL activity is a cell manipulated to comprise constantly neddylated CRLs. This may be achieved by the methods described herein above and herein below and as illustrated in the appended examples. As disclosed herein and as illustrated in the appended examples, said manipulation of the cell to comprise enhanced CRL activity may be carried out *before* said cell is contacted with the chemical compound or agent suspected to be able to induce ubiquitination or even *after* said cell is contacted with the (chemical) compound or agent suspected to be able to induce ubiquitination.

As disclosed herein and as discussed herein above, a corresponding read-out whether the compound (chemical compound or agent) to be identified in the methods of this invention is able and/or capable to induce ubiquitination of POI/target protein may be the measurement of the level of the at least one member of the E3 ligase complex. When said "level" of at least one member of the E3 ligase complex is increased compared to the "level" determined in the absence of said test compound in the eukaryotic cell (manipulated to a have an increased CRL activity), said compound (chemical compound or agent) is able to increase the activity of the E3 ubiquitin ligase. In other words, the activity of the test compound is measured in cells which were manipulated in accordance with this invention, i.e. in eukaryoric cells that have an increased CRL activity compared to a corresponding "wild-type cell", i.e. a cell that was not manipulated to have an increased CRL activity.

The term "determining the level of at least one member of an E3 ligase complex" is also further defined herein below and relates to the measurement of the amount of at least one member of an/the E3 ligase complex in said cell expressing the protein of interest.

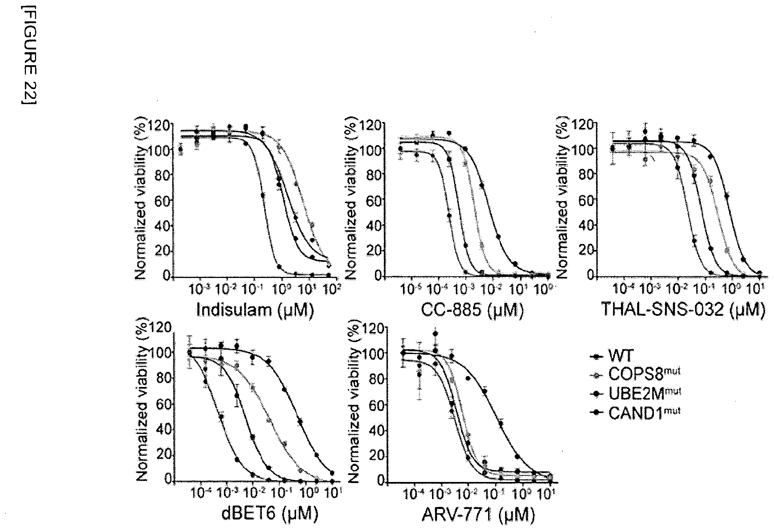

Another read-out whether a given "compound or agent" is able to induce ubiquitinationmay comprise the test of viability of a cell that has "enhanced CRL activity". As for example documented in Figure 22, compounds that function as molecular glues (such as CC-885) and PROTAC^{®}s (such as dBET6) that function via the CRBN E3 ligase lose their cytotoxic activity in cells where hyperneddylation has been induced due to modifying/inhibiting COPS8 or CAND1. Withouth being bound by theory, this is due to CRBN destabilization induced by constant E3 ligase activity and corresponding induced auto-ubiquitination and consequential auto-degradation (Figure 1B). In context of this invention, the term auto-ubiquitination refers to the transfer of at least one ubiquitin group(s) of the same E3 ligase complex i.e. in other words the ubiquitin ligase self-ubiquitinates and is thereby degraded. Accordingly, said auto-ubiquitination leads to a destabilization of the at least one member of the E3 ligase complex and/or of the complete ligase complex.

Due to a lack of CRBN in these cells, said chemical agents lose their activity to induce ubiquitination of a protein of interest/target protein/POI and hence their cellular activity and the modified/manipulated cell survives higher concentrations of said chemical agents.

The cullin-RING ubiquitin ligase, its activity and means and methods for the detection and/or measurement of this activity are described in detail herein below and are also illustrated in the appended examples. The same applies, *mutatis mutantis,* for the E3 ligase complex and its members.

In accordance with the above and in one aspect, the present invention relates to a method for identifying a (chemical) compound or agent able to induce ubiquitination of a protein of interest/target protein,
the method comprising:
   (i) contacting said chemical compound or agent with a eukaryotic cell and said cell being manipulated to comprise enhanced cullin-RING ubiquitin ligase (CRL) activity compared to the CRL activity in the absence of manipulating the cell to comprise enhanced CRL activity, wherein said eukaryotic cell expresses the protein of interest/target protein;
   (ii) determining the level of at least one member of an E3 ligase complex present in the cell;
wherein the (chemical) compound or agent is determined to induce ubiquitination of the protein of interest/target protein if the level of the at least one member of the E3 ligase complex is increased compared to the level determined in the absence of the chemical compound or agent. As described herein above and as exemplified herein, said eukaryotic cell manipulated to comprise enhanced cullin-RING ubiquitin ligase (CRL) activity is in particular a cell manipulated to comprise constantly neddylated cullin-RING ubiquitin ligases/CRLs and/or that is manipulated to comprise constant neddylation of cullin-RING ubiquitin ligases/CRLs.

As disclosed herein above and for all embodiments of the present invention, the determination of the level of the at least one member of the E3 ligase complex comprises in particular the determination of the amount of said at least one member of the E3 ligase complex in said eukaryotic cells to be employed in the methods of the present invention. The read-out of this "level" may also comprise a viability test as illustrated in the appended examples

Furthermore and as also discussed herein above, in the embodiments of the methods of the present invention as provided herein, said "manipulation of the [eukaryotric] cell to comprise enhanced cullin-RING ubiquitin ligase (CRL) activity" may be carried out *before* said cell is contacted with the chemical compound or agent suspected to be able to induce ubiquitination or after said cell is contacted with the chemical compound or agent suspected to be able to induce ubiquitination.

In a preferred aspect of the methods of the present invention, the eukaryotic cell to comprise enhanced cullin-RING ubiquitin ligase actvitiy (CRL activity) is manipulated by inactivation of CAND1, CAND2 or CAND1 and CAND2 (i.e. components/members of the E3 ligase complex) and/or or by inactivation of at least one member of the COP9 signalosome (CSN).

Accordingly, in the methods of the present invention, the eukaryotic cell manipulated to comprise enhanced cullin-RING ubiquitin ligase (CRL) activity and/or to comprise constantly neddylated cullin-RING ubiquitin ligases/CRLs may be manipulated by inactivation of a target protein like a Cullin-associated NEDD8-dissociated protein selected from the group consisting of CAND1, CAND2 or CAND1 and CAND2 or by inactivation of (a) target protein(s) being at least one member of the COP9 signalosome (CSN) and/or by inactivation of the COP9 signalosome (CSN). Said inactivation of the COP9 signalosome (CSN) may comprises the inactivation of the COPS5 subunit, the COPS8 subunit and/or of the COPS7B subunit. In context of this embodiment said target protein may be selected from the group consisting of CAND1, CAND2 or a protein member of the COP9 signalosome (CSN) Inactivation may comprise the inactivation by recombinant means and/or a hypomorphic mutation leading to a decrease of activity and/or abundance of the corresponding target protein or comprises the inactivation via a chemical compound/chemical inhibitor.

As also shown in the appended examples, the inactivation of a target protein may also comprises the step of contacting the eukaryotic cell (to be manipulated to comprise enhanced cullin-RING ubiquitin ligase (CRL) activity and/or to comprise constantly neddylated cullin-RING ubiquitin ligases/CRLs) with an inhibitor of the COP9 signalosome (CSN). Said inhibitor may be targeting the COPS5 subunit, preferably CSN5i-3.

As detailed herein, the eukaryotic cell manipulated to comprise enhanced cullin-RING ubiquitin ligase (CRL) activity and/or to comprise constantly neddylated cullin-RING ubiquitin ligases/CRLs may also be manipulated by overexpression of relevant proteins, i.e. by overexpressing at least one protein able to induce neddylation of the cullin-RING ubiquitin ligases/CRLs. Proteins to be overexpressed which do not form part of this invention may be proteins of the ligase complex, preferably of the E3 ligase complex such as a cullin/cullin backbone protein/culling homolog, (ii) an adaptor protein, a ligase substrate receptor, an E1 activating enzyme and an E2 conjugating enzyme. Accordingly, and as also illustrated below and in the appended examples, the protein to be overexpressed to induce neddylation of the cullin-RING ubiquitin ligases/CRLs may be at least one protein member of the E3 ligase complex and whereby said
(i) cullin/cullin backbone protein/cullin homolog may be selected from the group consisting ofCUL4A, CUL4B, CUL1, CUL2, CUL3, CUL5, CUL 6 [like Drosophila or C. elegans CUL6] and CUL7;preferably selected from the group consisting of human CUL4A, human CUL4B, human CUL1, human CUL2, human CUL3, human CUL5 and/or human CUL7, more preferably human CUL4A and/or human CUL4B ;
(ii) adaptor protein may be selected from the group consisting of DBB1, SKP1, preferably DDB1;
(iii) ligase substrate receptor may be selected from the group consisting of cereblon (CRBN), DCAF15, DCAF16, DCAF1, DCAF5, DCAF8, DET1, FBXO7, FBXO22, KDM2A, KDM2B, preferably cereblon (CRBN) or DCAF15;
(iv) E1 activating enzyme may be NAE1, UBA3 subunit or a heterodimer of NAE1 and UBA3 subunit (NAE1 or UBE2M ); and
(v) E2 conjugating enzyme may be EBc12 or UBE2M, preferably UBE2M.

Accordingly, means and methods for enhancing the CRL activity in a cell/cellular system are disclosed herein and comprise, *inter alia,* the overexpression of ligase substrate receptors, cullins/cullin backbone proteins/culling homologs, adaptor proteins, E1 activating enzymes and/or an E2 conjugating enzymes (as also illustrated in the appended examples). Details on these (E3 ligase) complex members/proteins are also provided herein below.

As detailed herein, the gist of the present method-invention is the provision of cellular test system wherein the cullin-RING ubiquitin ligase activity/CRL activity is enhanced and wherein said enhancement is achieved via neddylated cullin-RING ubiquitin ligase. Therefore, in a preferred aspect of the method of the present invention, the eukaryotic cell manipulated to comprise enhanced activity may be a cell manipulated to comprise constantly neddylated cullin-RING ubiquitin ligases/ constantly neddylated CRLs. Said constantly neddylated cullin-RING ubiquitin ligases/CRLs and/or said constant neddylation of cullin-RING ubiquitin ligases/CRLs leads to destabilization and/or auto-degradation of at least one member of the cullin-RING ubiquitin ligase/CRL, for example the constant neddylation may lead to destabilization and/or auto-degradation of a substrate receptor of the cullin-RING ubiquitin ligase/CRL. This is also documented in the appended examples. For example, the constantly neddylated CRLs/hyperneddylated CRLs may be achieved by a hypomorphic mutation or inactivation of at least one member of the COP9 signalosome (CSN). COP9 (Constitutive photomorphogenesis 9) signalosome (CSN) is a protein complex with isopeptidase activity. It catalyses the hydrolysis of NEDD8 protein from the cullin subunit of Cullin-RING ubiquitin ligases (CRL). Therefore, it is responsible for CRL deneddylation - at the same time, it is able to bind deneddylated cullin-RING complex and retain them in deactivated form. COP9 signalosome thus serves as a sole deactivator of CRLs. As illustrated in the appended examples, said at least one member of the CSN to be inactivated may be the COPS5, COPS8 or COPS7B subunit. This leads to the desired neddylation and, accordingly, to the desired enhanced/augmented CRL activity in context of the present invention. In another aspect of the method of the present invention, the cell manipulated to comprise constantly neddylated CRLs is manipulated by contacting the cell with a chemical, like a specific CSN inhibitor, for exmaple with CSN5i-3. Also this is illustrated herein and detailed in the appended examples. Also this chemical exposure of the eukaryotic cell to be employed in the methods of the present invention leads to the desired neddylation and, hence to the desired enhanced/augmented CRL activity. Further details are provided herein below

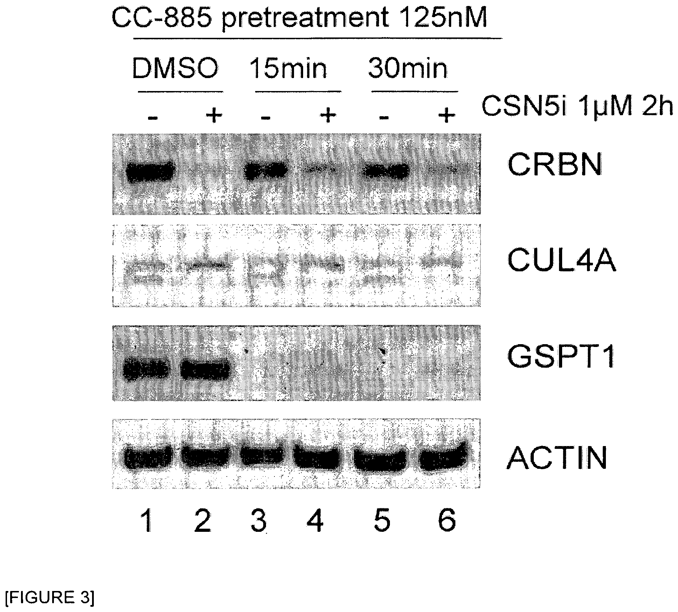

The following provides additional illustrations of the inventive methods. For example, the present invention provides for an method for identifying or testing compounds. In appended example 1, illustratively a molecular glue like CC-885 o or other test compounds like dBEt6 or SNs-THAL-O32 are evaluated for their capcity to induce ubiquitination of (a) desired protein(s). Desired proteins to be preferably destructed in a diseased cell may be proteins upregulated and/ or overexpressed in a diseased cell, like certain translation factors, for example GSPT1 As illustrated in the appended examples, methods for the identification whether a given test compound ("chemical or agent") is able to induce ubiquitination may comprise the following steps (i) and (ii):
(i) contacting said test compound with a eukaryotic cell, for example a leukemia cell, like KBM7 comprising and/or expressing said protein of interest, for example GSPT1, and manipulating said cell to comprise enhanced cullin-RING ubiquitin ligase (CRL) activity, for example by gene modification technologies including but not limited to CRIPSR (e.g. CRISPR modification of genes involved in neddylation, like COP9 signalosome subunits, in particular COPS8 or COPS7B, or like CAND1 and/or CAND2) or by chemical inhibition of proteins involved in neddylation, like inhibition of COP9 signalosome subunits, in particular COPS5, COPS8 or COPS7B, whereby this manipulation leads to constant neddylation/hyperneddylation of cullins/CRLs.
(ii) determining the level of at least one member of an E3 ligase complex for example a substrate receptor, like CRBN, present in the cell;
wherein said test compound is identified to induce ubiquitination of the protein of interest/target protein (in this case GSPT1) if the level of the at least one member of the E3 ligase complex, for example a substrate receptor, like cereblon (CRBN), is increased compared to the level determined in the absence of said test compound. A corresponding, non-limiting example is provided in the appended Figure 3 and Example 3. Accordingly, the "increased level" may be determined via quantitative means. One example of such a quantitative test and readout is an immunoblot technology, like quantitative Western blots (see first lane of Figure 3).

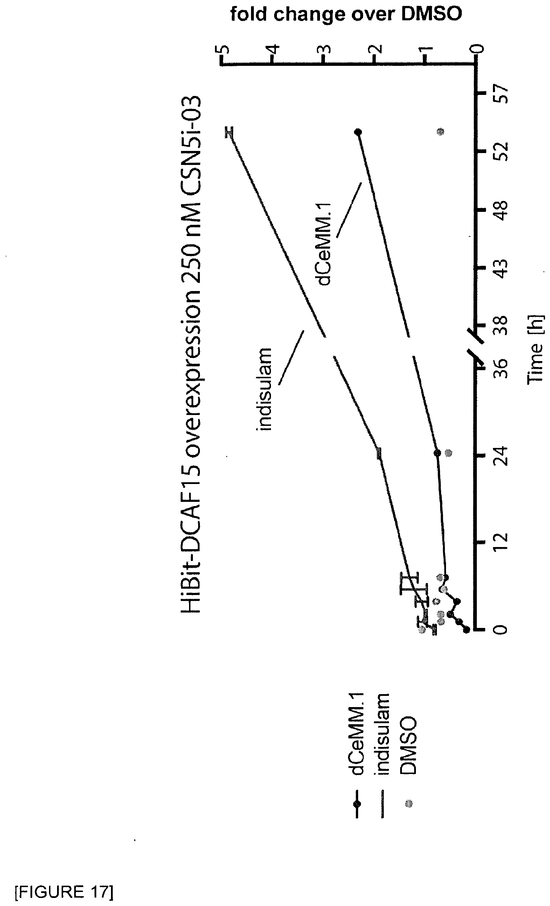

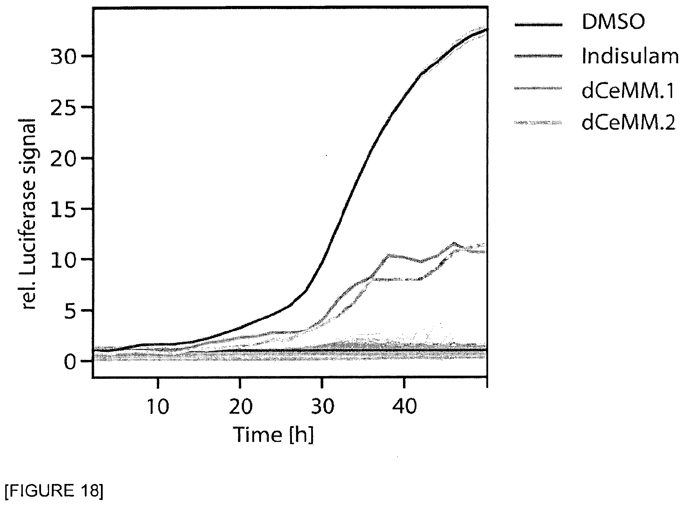

Another, non-limiting illsutration illustration of the inventive method is the provision of a method for a test compound, for example a molecular like indisulam (able to induce ubiquitination of a protein of interest), for example a protein preferably to be destructed in a diseased cell, like proteins upregulated and/ or overexpressed in a diseased cell, like certain gene control factors, for example RBM39). This illustrative method comprises in context of this invention the following steps:
(i) contacting said test compound (indisulam) with a eukaryotic cell, for example a human cell, like 293T comprising and/or expressing said protein of interest, here illustratively RBM39, and manipulating said cell to comprise enhanced cullin-RING ubiquitin ligase (CRL) activity, for example by ectopically overexpressing at least one component of a CRL, for example by stable lentiviral delivery. In a preferred instance, the at least one component of a CRL is a substrate receptor, for example DCAF15;
(ii) determining the level of at least one member of an E3 ligase complex for example a substrate receptor, here DCAF15, present in the cell;
wherein said test compound is identified to induce ubiquitination of the protein of interest/target protein (in this case RBM39) if the level of the at least one member of the E3 ligase complex, for example a substrate receptor, like DCAF15, is increased compared to the level determined in the absence of said chemical compound or agent of interest. A corresponding, non-limiting example is provided in the appended Figures 17 and 18. Accordingly, the "increased level" may be determined via quantitative means. One example of such a quantitative test and readout is a luciferase assay based on quantifying bioluminescence intensity. Accordingly, the at least one member of the E3 ligase complex, for example a substrate receptor, like DCAF15 needs to be expressed as a fusion to a luciferase, like a nanoluciferase, or as a fusion to a split luciferase approach, such as the HiBit^{®} (Promega) tag.

In one aspect of the method of the present disclosure, the protein of interest (POI)/target protein (to be ubiquitinated and, thereby, degraded) may be a protein encoded by an oncogene-addiction (OA) gene or a non-oncogene addiction (NOA) gene. A protein of interest/target protein/POI of the present invention may be selected from the group consisting of DNA-binding proteins including transcription factors, RNA binding proteins, scaffolding proteins, GTPases, solute carriers, kinases, phosphatases, bromodomain- and chromodomain containing proteins, and G-protein coupled receptors. As discussed above, the POI may be in particular a protein that is desired to be degraded in a cell via ubiquination, in particular via directed ubiquination. Such a desired ubiquination may be achieved with the chemical agents or agents tested and/or detected/indetified with the methods of the present invention. "Proteins of interest" are readily deducible by the person skilled in the art. Non-limiting, illustrative examples of such POIs, for example in cancer cells or in cells that are involved in metabolic or neurological disorders, are provided herein below and in the following. POIs involved in cancer may be, *inter alia,* DNA-binding proteins including transcription factors such as ER, AR, MYB, MYC; RNA binding proteins; scaffolding proteins; GTPases such as HRAS, NRAS, KRAS; solute carriers; kinases, such as CDK4, CDK6, CDK9, EGFR, SRC, PDGFR, ABL, HER2, HER3, BCR-ABL, MEK, ARAF, BRAF, CRAF, phosphatases, bromodomain- and chromodomain containing proteins such as BRD2, BRD3, BRD4, CBP, p300, ATAD2, SMARCA2, SMARCA4, PRBM1, G-protein coupled receptors; anti-apoptotic proteins such as SHP2, PTPN1, PTPN12; immune regulators such as CTLA-4, PD-1 or PD-L1 and combinations thereof. Other POIs in cancer may be selected from the group consisting of BRD2, BRD3, BRD4, CBP, p300, ATAD2, SMARCA2, SMARCA4, PRBM1, CDK4, CDK6, CDK9, CDK12 and/or CDK13, EWS-FLI, CDC6, CNPE, EGFR, SRC, PDGFR, ABL, HER2, HER3, BCR-ABL, MEK, ARAF, BRAF, CRAF, HRAS, NRAS, KRAS, BCL2, MCL2, SHP2, PTPN1, PTPN12, ER, AR, MYB, MYC,and combinations thereof. Non-limiting examples of such POIs in cells that are involved in metabolic disorders may be ARX, SUR and DPP4. Non-limiting examples of POIs in cells that are involved in neurologic disorders may be CCR5 and PLA2G16.

In one aspect of the methods of the present invention, the (eukaryotic) cullin-RING ubiquitin ligases/CRLs may comprise eukaryotic cullins, like CUL1, CUL2, CUL3, CUL4A, CUL4B, CUL5, CUL6 (like Drosophila or C. elegans CUL6] or CUL7. Said cullin-RING ubiquitin ligases/CRLs are, in context with the methods of the present invention to be enhanced in their activity. Preferably and in accordance with this invention, said enhancement is based on a neddylation of said cullin-RING ubiquitin ligases/CRLs in particular a neddylation of human cullins, like hCUL1, hCUL2, hCUL3, hCUL4A, hCUL4B, hCUL5, hCUL7 or complexes thereof. Preferably, said cullin-RING ubiquitin ligases/CRLs are constantly neddylated in the eukaryotic cells to be employed in the methods of the present invention. As detailed herein, such a constant neddylation of the said cullin-RING ubiquitin ligases/CRLs in particular of hCUL1, hCUL2, hCUL3, hCUL4A, hCUL4B, hCUL5 or hCUL7 may be achieve, *inter alia,* via the overexpression of the substrate receptor of the CRL, like DCAF15 and/or CRBN. This also illustrated in the appended examples, wherein e.g. DCAF15 is overexpressed.

As mentioned and illustrated above, in another aspect of the methods of the present disclosure, the at least one member of the E3 ligase complex (the level of which is to be assessed in context of the present invention) may comprise a modification allowing its direct or indirect detection (which does not form part of this invention), and, correspondingly the determination of its [expression] "level". Such a modification which does not form part of this invention may be the addition of a tag, like a fluorescent tag or a report molecule, like a reporter enzyme. Such tags comprise, but are not limited to split-fluorescent tags, (bio-luminescence molecules, like luciferase(s). Reporter enzymes may also comprise horseradish peroxidase orluminescence molecules, like luciferases, or a peptide binding such tags, (bio-)luminescence molecules or reporter enzyme such as a luciferase. Further useful reporter molecules and/or tags are disclosed herein below. Also corresponding (labeling) peptides are known in the art and comprise, HiBit peptides. Also the appended Examples illustrate this embodiment. For example, in the experimental part of this disclosure a substrate receptor of the E3 ligase complex, illustratively DCAF15, is employed as said "at least one member of the E3 ligase complex" and said DCAF15 is labeled with an luciferase molecule in order to determine its [expression] level after contacting the eukaryoric cell with the test compound (i.e. the chemical compound or agent to be identified as being able to induce ubiquitination of a POI in accordance with the methods of this invention).

An accordance with the above and in a further aspect of the methods of the present invention, the chemical compound or agent to be identified, evaluated and/or tested for its capacity to induce/facilitate/enable ubiquitination of a POI may be a peptide, a small molecule, or a molecule having a size between 0.5 and 2.0 kDa, preferably between 0.8 and 1.5 kDa (such as PROTAC^{®} compounds). In the appended examples, it is documented that the method as provided herein are powerful tools to identify and/or evaluate compounds that are capable, either directly or indirectly, of inducing, facilitating and/or enabeling ubiquination of desired target proteins (POIs). The method of the present invention is shown to be fully enabled. For example, the inventive method is verified in the appended Examples by employing test compounds ("chemical compounds or agents") which are known in the art as inducing ubiquitination of a target protein (i.e "positive controls" for the inventive methods). These positive test/control compounds comprise an "IMiD" (CC-885), an modulator of the E3 ligase substrat receptor DCAF15 (indisulam), a selective CDK9 degrader PROTAC^{®} (THAL-SNS-032) and a selective and cell-permeable degrader of BET based on PROTAC^{®} (dBET6). It is documented in the appended Examples that the inventive methods can readily be employed to verify these compounds as inducers and/or facilitators of the desired ubiquitination. Accordingly, in these illustrative examples, the PROTAC^{®} THAL-SNS-032 is the "compound to be tested" (as appositive control since is known that this PROTAC^{®} leads to ubiquitination. In this example, CDK9 would be the POI to be ubiquitinated/degraded.

In another aspect of the method of the present disclosure, the chemical compound or agent to be identified/tested may be part of a library of compounds or agents which does however not form part of this invention. The term "compound or agent" also relates to mixtures of compounds as discussed herein above. The ability to induce ubiquitination of the protein of interest is determined by the methods of the present disclosure for at least two compounds or agents comprised in the library. In other words, the present disclosure is also useful for screeing of mixtures of test compounds for their ability to induce, facilitate and/or enable ubiquitination of (a) protein of interests.

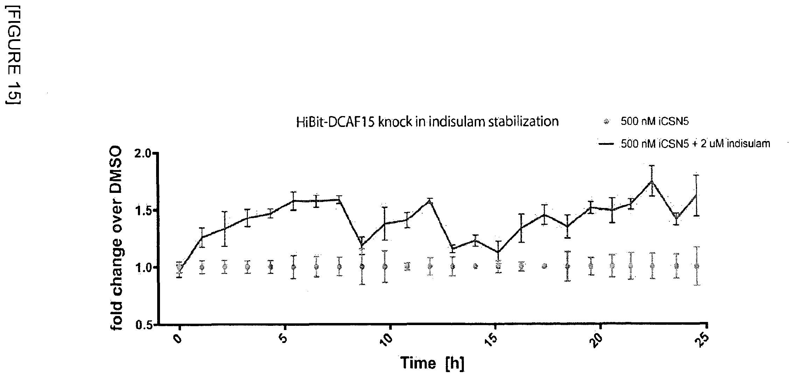

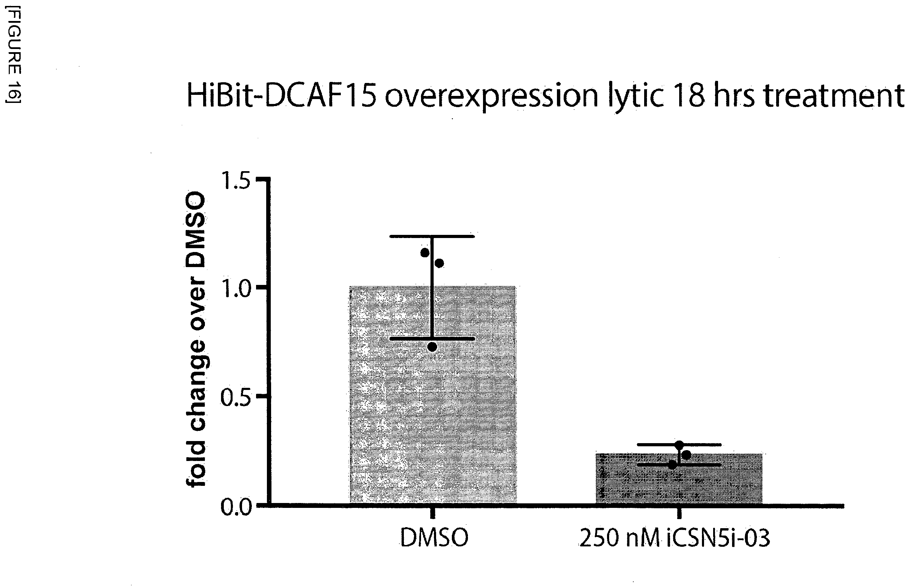

The methods of the present invention may also be employed in high-throughput screenings. In this context, in particular readouts with labels and tags are preferably employed. See, for example, appended Figures 15-18 where bioluminescence measurements are employed. In the corresponding illustrative, non-limiting examples, 384 multiwell plates are used for high-throuput screenings employing the method of the present invention. As illustrated herein below and in the appended examples, in some tests/assays described herein, about 8000 compounds could be assessed/testedin an arrayed format, wherein each compound was assessed individually (e.g. one compound per well) at a concentration of about 10 uM.

Also described herein but not part of the invention is a eukaryotic cell manipulated to comprise an enhanced cullin-RING ubiquitin ligase (CRL) activity which does not form part of this invention. Said cell of the present disclosure comprises enhanced CRL activity, achieved by neddylation of at least one member of the E3 ligase complex. neddylation is a constant neddylation and/or a hyperneddylation. As discussed herein and as illustrated in the appended examples, said constant neddylation and/or hyperneddylation may be achieved in the eukaryotic cell by inactivation of a target protein like a Cullin-associated NEDD8-dissociated protein selected from the group consisting of CAND1, CAND2 or CAND1 and CAND2, by inactivation of (a) target protein(s) being at least one member of the COP9 signalosome (CSN) and/or by inactivation of the COP9 signalosome (CSN). For example, the cell may be manipulated by inactivation of CAND1, CAND2 or CAND1 and CAND2 [also leading to the desired neddylation as illustrated in the appended example for CAND1]. As discussed herein above, this may, *inter alia,* be achieved in that the cell is manipulated by a hypomorphic mutation or inactivation of at least one member of the COP9 signalosome (CSN), for example by inactivation of the COPS8 or COPS7B subunit of the COP9 signalosome (CSN). In another preferred aspect, the cell manipulated to comprise constantly neddylated CRLs is manipulated by contacting the cell with a chemical capable of inducing said neddylation/constant neddylation. Such a chemical may be a specific CSN inhibitor, for example CSN5i-3 as illustrated in the appended examples.

Other options for achieving the desired constant neddylation and/or hyperneddylation in the eukaryotic cell may comprise the overexpression at least one protein able to induce neddylation of the cullin-RING ubiquitin ligases/CRLs, for example the overexpression of of (i) a cullin/cullin backbone protein/culling homolog, (ii) an adaptor protein, (iii) a ligase substrate receptor, (iv) an E1 activating enzyme and (v) an E2 conjugating enzyme. Therefore, said neddylation may comprise the neddylation of cullin-RING ubiquitin ligases/CRLs, preferably the neddylation of eukaryotic CUL1, CUL2, CUL3, CUL4A, CUL4B, CUL5, CUL6 (CUL6 is for example known in Drososphila or in C. elegans], CUL7, and/or complexes thereof like the CUL4A/B complex. Preferably, said neddylation comprises the neddylation of a human cullin. An illustrative example of such a cell comprising a neddylation of at least one member of the E3 ligase complex is a cell that comprises the neddylation of CUL4A (or the CUL4A/B complex). In this illustrative example, this is achieved overexpressing the substrate receptor DCAF15; see also the appended experimental part. Another illustrative option to achieve the desired [constant] neddylation is the overexpression of proteins able to induce neddylation of the CRLs in the cell, such as the overexpression of NAE1 or UBE2M. Further means and methods for the enhancement of CRL activity are provided herein below and illustrated in the appended examples (for example the overexpression of substrate receptor(s)).

In accordance with the above and in another preferred aspect of the present disclosure, the cell manipulated to comprise enhanced CRL activity is further a cell manipulated to comprise constantly neddylated CRLs. More preferably, the inventive cell and/or the cell to be employed in the methods of the present invention cell is manipulated to comprise constantly neddylated or hyper-neddylated CRLs.

In one aspect, the eukaryotic cell to be used in context of the present disclosure, i.e. the cell to be employed in the inventive methods the present invention, may be a cell that expresses an oncogene or may be a cancer cell such as a lung cancer cell, a gastric cancer cell, a melanoma cell, a sarcoma cell, a leukemia cancer cell, a colon cancer cell or a neuroblastoma cell which however does not form part of this invention. These cells are considered in context of this invention as "wild-type cells". However, in accordance with this invention, the cells are to be manipulated in comprise an enhanced cullin-RING ubiquitin ligase (CRL) activity. Means and methods to obtaining said desired enhanced cullin-RING ubiquitin ligase activity/CRL activity are provided herein. These manipulated cells and (as contol cells the "wild-type" or "originator cells") are to be employed in the methods of the present invention. Yet, as disclosed herein, the present disclosure also relates to the herein abtained and first described cells which are manipulated to comprise enhanced cullin-RING ubiquitin ligase (CRL) activity. Preferably, these inventive cells/cellular systems/test cell systems are characterized by constantly neddylated CRLs/hyperneddylated CRLs. Accordingly, in one aspect, the present disclosure also provides for novel and inventive cells/cellular systems/test cell systems which can also be employed in context of the present invention, i.e these cells may be used in the method of the present invention.

Therefore, here described but not part of the invention is an eukaryotic cell/cellular system/test cell system manipulated to comprise an enhanced cullin-RING ubiquitin ligase (CRL) activity. Preferably, said cell and/or cellular systems/test cell system which is manipulated to comprise enhanced cullin-RING ubiquitin ligase (CRL) activity is a cell manipulated to comprise constantly neddylated cullin-RING ubiquitin ligases/CRLs and/or that is manipulated to comprise constant neddylation of cullin-RING ubiquitin ligases/CRLs. As discussed herein and as exemplified in the appended Examples, such a constant neddylation may be achieved in some embodiments of the inventive cell/cellular systems/test cell systems by inactivation of a Cullin-associated NEDD8-dissociated protein selected form the group consisting of CAND1, CAND2 or CAND1 and CAND2 or by inactivation of at least one member of the COP9 signalosome (CSN). Said inactivation of the COP9 signalosome (CSN) may comprise, in one embodiment, the inactivation of the COPS5 subunit, the COPS8 subunit and/or of the COPS7B subunit. The inactivation in the cell/cellular system/test cell system may also comprises an inactivation of the coding genes of the relevant target proteins by recombinant means and/or a hypomorphic mutations leading to a decrease of activity and/or decrease of abundance of the corresponding target protein. The inactivation may also comprise inactivation of the corresponding target proteins via a chemical compound/chemical inhibitor. These target proteins may, in accordance with the above, comprise CAND1, CAND2 or a protein member of the COP9 signalosome (CSN). It is understood that in context of the present disclosure, also the corresponding coding genes and/or the corresponding RNA may be inactivated. However, also chemical means may be employed to inactivate relevant molecules. For example, the (eukaryotic) cells/cellular system/test cell system may be characterized in that the cell/cellular system/test cell system comprising constantly neddylated cullin-RING ubiquitin ligases/CRLs is treated/contacted with an inhibitor of the COP9 signalosome (CSN), for example an inhibitor COPS5 subunit, preferably CSN5i-3.

As is evident from the current disclosure, the (eukaryotic) cell/cellular system/test cell system comprising enhanced CRL activity may also be obtained by overexpressing at least one protein able to induce neddylation of the CRLs. Said at least one protein to be overexpressed in said cell/cellular system/test cell system may be selected from the group consisting of (i) a cullin/cullin backbone protein/culling homolog, (ii) an adaptor protein, (iii) a ligase substrate receptor, (iv) an E1 activating enzyme (like NAE; a heterodimer of NAE1 and UBA3 subunit), and (v) an E2 conjugating enzyme (like Ubc12, UBE2M). In one aspect of the eukaryotic) cell/cellular system/test cell system said overexpression of at least one protein may be the overexpresion of at least one member of the E3 ligase complex and whereby said member of the E3 ligase complex may be selected from the group consisting of
(i) a cullin/cullin backbone protein/cullin homolog which may be selected from the group consisting of an eukaryotic cullin, like CUL4A, CUL4B, CUL1, CUL2, CUL3, CUL5, CUL6 (e.g. CUL6 from Drsosphila or from C. elegans) and CUL7; a cullin/cullin backbone protein/cullin homolog preferably selected from the group consisting of human CUL4A, human CUL4B, human CUL1, human CUL2, human CUL3, human CUL5 and/or human CUL7, more preferably human CUL4A and/or human CUL4B;
(ii) an adaptor protein that may be selected from the group consisting of DBB1, SKP1, preferably DDB1;
(iii) a ligase substrate receptor which may be selected from the group consisting of cereblon (CRBN), DCAF15, DCAF16, DCAF1, DCAF5, DCAF8, DET1, FBXO7, FBXO22, KDM2A, KDM2B, preferably cereblon (CRBN) or DCAF15;
(iv) an E1 activating enzyme that may be NAE1, UBA3 subunit or a heterodimer of NAE1 and UBA3 subunit; and
(v) an E2 conjugating enzyme that may be EBc12 or UBE2M, preferably UBE2M.

In a preferred aspect of the cell/cellular system/test cell system and/or the c cell/cellular system/test cell system to be employed in the inventive method, the eukaryotic cell manipulated to comprise enhanced cullin-RING ubiquitin ligase (CRL) activity may express an oncogene or may be, in its origin, a cancer cell or an established cancer cell line, such as a lung cancer cell, a gastric cancer cell, a melanoma cell, a sarcoma cell, a leukemia cancer cell, a colon cancer cell or a neuroblastoma cell.

In accordance with the above, in one embodiment of the present disclosure for ample means and methods are provided for the generation of cellular systems/test cells that can be employed in the inventive methods, i.e. in assays, also screening assays for compounds/ agents able to induce ubiquitination of (a) protein of interests as defined herein.

The present disclosure also relates to a (chemical) compound or agent identified/ tested/obtained by the method of the present invention which does however not form part of the present invention. Preferably, said (chemical) compound or agent is for use in medicine, more preferably for use in the treatment of cancer, a metabolic disease/disorder, an infectious disease/disorder or a neurological disease/disorder, most preferably in the treatment of cancer. Accordingly, here described but not part of the invention is a method for treating cancer, a metabolic disease/disorder, an infectious disease/disorder or a neurological disease/disorder, most preferably treating cancer, said method of treating comprising administering the (chemical) compound or agent as obtained with the methods of present invention to a patient having aor suspected to suffer from said cancer, metabolic disease/disorder, infectious disease/disorder or said neurological disease/disorder.

Hence, in terms of clinical applications, the methods provided herein may not only be usaed as a research tool, but also in clinical developments and/or in the testing/evaluation of medicaments. The compounds/agents to be screened/identified in accordance with this embodiment are compounds/agents that are able to induce ubiquitination of a protein of interest/target protein/POI. Accordingly, the present invention is not only of relevance as research tools but also in clinical applications, for example in the identification of compounds or agents able to induce molecular proximity to a cullin-RING substrate receptor and, in consequence, ubiquitination of a protein of interest/target protein/POI.

In accordance with the above, the present invention also relates to an in *vivo* (i.e. from the human and/or animal body isolated living cell systems/ cells/cell lines) assay for identifying/testing/obtaining a compound or agent able to induce ubiquitination of a protein of interest. The method of the invention may comprise an *in vivo* method for identifying/testing/obtaining a (chemical) compound or agent able to induce ubiquitination of a protein of interest/target protein/POI,
the method comprising:
   (i) contacting said (chemical) compound or agent with a eukaryotic cell and manipulating the cell to comprise enhanced cullin-RING ubiquitin ligase (CRL) activity compared to the CRL activity in the absence of manipulating the cell to comprise enhanced CRL activity, wherein the cell expresses the protein of interest/target protein/POI;
   (ii) determining the level of at least one member of an E3 ligase complex present in the cell;
wherein the chemical compound or agent is determined to induce ubiquitination of the protein of interest if the level of the at least one member of the E3 ligase complex is increased compared to the level determined in the absence of the chemical compound or agent.

The present invention provides also an *in vivo* assay (as defined herein, i.e *in vivo* assays based on isolated cellular systems and/or laboratory animals. like transgenics and/or xenograft models) for identifying a chemical compound or agent able to induce ubiquitination of a protein of interest/target protein/POI. As is evident from the appended examples and as already detailed herein above, the method for identifying a compound or agent able to induce ubiquitination of a protein of interest/target protein/POI as provided herein comprises the determination of the level of an at least one member of the E3 ligase complex in a eukaryotic cell manipulated to comprise enhanced CRL activity in the absence and presence of the chemical compound or agent. The enhanced CRL activity in the cell is induced by genetical or chemical manipulation of the cell as described below. In the absence of the chemical compound or agent, the enhanced CRL activity of the cell leads to ubiquitination and degradation of the at least one member of the E3 ligase complex by the CRL, a process denoted as autodegradation and exemplified in the appended examples. A (chemical) compound or agent contacted with the cell may be able to induce molecular proximity between a component of a CRL E3 ligase complex and a protein of interest/target protein/POI which may be bound to the chemical compound or agent or which may be part of a ternary complex.

As shown in the examples, it has been surprisingly found that a (chemical) compound or agent bound to the protein of interest/target protein/POI may be able to impair the autodegradation of the at least one member of the E3 ligase complex by hijacking the at least one member of the E3 ligase complex, e.g. by proximity or binding of the chemical compound or agent contacted with the protein of interest/target protein/POI to the at least one member of the E3 ligase complex. Thus, as shown in the examples and as discussed herein, the autodegradation of the at least one member of the E3 ligase complex may be impaired in that the protein of interest/target protein/POI may be ubiquitinated by the CRL rather than the at least one member of the E3 ligase complex.

Thus, it has been surprisingly found in context of this invention that determining and correlating the level of the at least one member of the E3 ligase complex in the absence and presence of the (test) (chemical) compound or agent can be used to identify/verify/test whether said compound or agent is able to induce ubiquitination of the protein of interest. In particular, if the level of the at least one member of the E3 ligase complex is increased compared to the level determined in the absence of the chemical compound or agent, it is indicative that the autodegradation of the at least one member of the E3 ligase complex is impaired in the presence of the chemical compound or agent. In this case, the chemical compound or agent is determined to induce ubiquitination of the protein of interest. For example, a (chemical) compound, or agent may bind to the E3 ligase complex and the POI. As another example, a chemical compound or agent may alter the function of a target protein, e.g. by modifying posttranslational changes of a target protein. A posttranslational modification may include but is not limited to the phosphorylation status of a protein, e.g. a tyrosine kinase phosphorylating a protein. Thus, a compound or agent may induce ubiquitination of a POI, e.g., by modifying a POI in that the POI becomes accessible for a E3 ligase, thereby a chemical compound or agent may not associate with a POI and/or a E3 ligase. As discussed in the embodiments above, the term "compounds or agents able to induce ubiquitination of a protein of interest" also comprises "biochemical compounds". Such biochemical compounds may be, but are not limited to chemical compounds which comprise biological/biochemical parts, like, *inter alia,* peptide linkers bound to said chemical compounds.

Therefore, the present invention provides an improved methods for identification of agents/compounds capable of ubiquitination or of inducing ubiquitination of a protein of interest as compared to corresponding methods in the art.

It is an advantage of the present invention that the methods of the invention can be performed with any chemical compound or agent as long as the protein of interest/target protein/POI to be targeted by the chemical compound or agent is present in the cell to be employed in the inventive methods, i.e. a cell that is manipulated to comprise enhanced cullin-RING ubiquitin ligase activity/ CRL activity.

Therefore, the method of the present invention offers a "one-fits it all" setup, i.e. a method for identifying compounds and agents able to induce ubiquitination of all conceivable proteins of interest/POIs by recruiting the POI to the CRL. In particular, the methods of the present invention only require modification of the at least one member of the E3 ligase complex, e.g a ligase substrate receptor of the CRL, a cullin/cullin backbone protein/culling homolog, an (ligase) adaptor protein, an E1 activating enzyme or an E2 conjugating enzyme. By this means, modification of the at least one member of the CRL allows for the identification of (chemical) compounds or agents able to induce ubiquitination of all conceivable POIs without the need of changing the set up of the assay depending on the POI to be analysed. In contrast to the method of the present invention (i.e. the use of cells/cellular systems which are modified to comprise enhanced cullin-RING ubiquitin ligase (CRL) activity and which are therefore modified to comprise constantly neddylated cullin-RING ubiquitin ligases/CRLs), a modification of POIs would require a specific modification of each POI so that assays for identifying compounds able to induce ubiquitination by detection of different POIs would require an individual assay development for each POI. Thus, since the POI in the method in the present invention is not modified, it is a further advantage of the method of the present invention that there is no need of individual assay development. Specifically, it is not required to transiently transfect the modified POI by methods known in the art. The above described methods and (screening) assay of the present invention may require only the determination of the at least one member of the E3 ligase complex. Thus, it is a particular advantage of the method of the present invention that a wide array of chemical compounds or agents can be tested in the same set up of the method, i.e. without the need of adapting the method, e.g. to each POI to be analysed.

Further, since modification of the POI is not required by the method of the present invention, binding of cognate cellular binding partners and effectors of the POI, and post-translational modifications of the POI are not affected as it may occur by the modification of a POI. Therefore, false negative results that may occur due to an artificial modification of the POI that may cause an impaired binding of the modified POI to the chemical compound or agent can be avoided. In addition, since the method of the present invention is performed in a cell, the method is informative for cellular target degradation by the CRL by considering physiological processes such as cellular uptake, compartmentalization, and compound stability. In this context, the method(s) of the present invention is/are an *in vivo* method performed in and on (a) isolated cell and thus reports on a chemical compound or agent able to induce ubiquitination of a protein of interest under physiological conditions. The term "in vivo" is defined herein as not comprising humans and or animals and is to be restricted to isolated cells/isolated cellular systems and/or isolated tissues. In one very specific embodiment, the methods of the present invention may also comprise the testing of transgenic animals that are genetically modified to comprise cells/tissues wherein said cells/tissues comprise an enhanced cullin-RING ubiquitin ligase (CRL) activity (as compared to animals/trasngenics that are not modified to comprise enhanced cullin-RING ubiquitin ligase (CRL) activity. Means and methods to obtain "enhanced cullin-RING ubiquitin ligase (CRL) activity" are amply provided herein for isolated cells/cellular systems. The same embodiments apply, *mutatis mutantis,* for the herein incorpotated transgenic test animals. Similarly, the present invention may also comprise the use of xenograft and/or allograft (allotransplantation) models, for example the use of genetically modified tissues/cells that are manipulated to comprise enhanced cullin-RING ubiquitin ligase (CRL) activity as defined above and that are transferred to living test animals. Such models are also comprised under the term "in vivo" as employed in context of the present invention.

It is a further advantage of the method of the present invention that stabilization of the at least one member of the E3 ligase complex upon treatment with a chemical compound or agent is observed over many different hours and thus it is less time-sensitive as compared to measuring ternary complex formation via tagged subunits as applied in existing methods for determining the activity of a E3 ubiquitin ligases.

Furthermore, autodegradation of the at least one member of the E3 ligase complex is only induced if a E3 ligase complex has been formed. Consequently, an increased level of the at least one member of the E3 ligase complex in the presence of the chemical compound or agent compared to the level determined in the absence of the chemical compound or agent specifically reports on the CRL activity of the E3 ligase complex. Thus, no major artifacts are expected, e.g. from measuring dimerization of the compound or agent contacted to the POI with the monomeric at least one member or a dimeric complex comprising the at least one member and e.g. an adaptor protein of the at least one member. Therefore, the method of the present invention is specific for identifying a chemical compound or agent able to induce ubiquitination of a POI by the CRL in a E3 ligase complex. In other words, the method of the present invention reports on the cellular degradation activity of the CRL, i.e. the CRL activity to ubiquitinate and degrade a POI, in the absence and presence of a chemical compound or agent.

As discussed herein and as illustrated in the appended examples, the present invention provides for means and methods wherein in the inventive methods , *inter alia,* cells are employed in which the *de*-neddylation of the CRL complexes is weakend and/or hampred. A corresponding non-limiting example is the inactivation of COPS5, COPS8 and/or COPS7B impairing the functionality of the COP9 signalosome (CSN). Thereby, the deneddylation of cullin RING ligase (CRL) complexes is abrogated, weakened and/or hampered. This leads to constantly neddylated CRL complexes that are locked in a constitutive active state of enhanced/uninterrupted activity and as a consequence to auto-degradation. Similarly, modification/inactivation of CAND1 and/or CAND2 as discussed herein impairs functionality of CRL decommissioning/ impairing the dynamic exchange of substrate receptors of CRLs. This also leads to constantly neddylated CRL complexes that are locked in a constitutive active state of enhanced/uninterrupted activity and as a consequence to autodegradation. Such autodegradations (for example also of cereblon/CRBN) and corresponding constant neddylations/hyperneddylation is also illustrated in appended Example 1 and in the appended Figures, see, e.g. Figure 1 B and G to I. As explained herein, the term "constantly neddylated" means/is equivalent to the term "hyperneddylation". In context of this invention, the term "constantly neddylated", "constant neddylation", and "hyperneddylation" are considered to be equivalent. These terms relate to intracellular levels of neddylated cullins, as detailed also herein below.

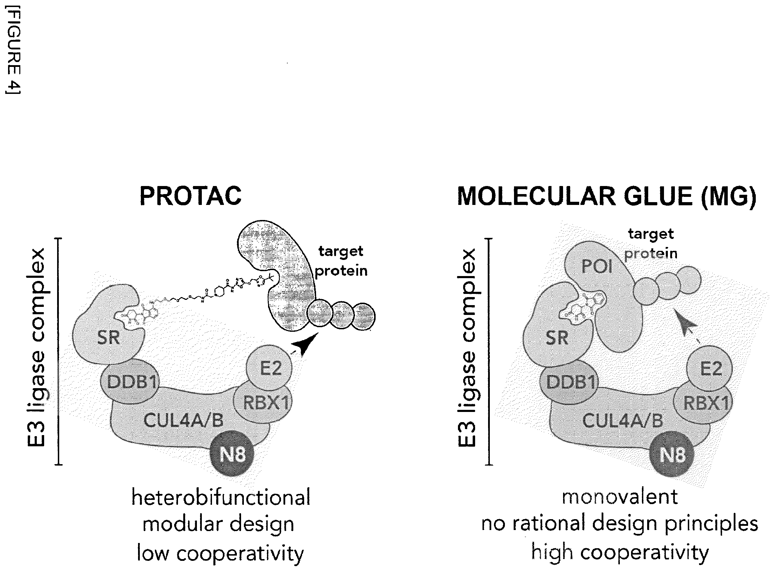

Without being bound by theory, a molecular glue (MG) can be used to bring the protein of interest in the direct physical interaction with E3 ligases. Accordingly, the herein claimed method may comprise the use of (small) molecules inducing direct physical interaction between a protein of interest and at least one member of the E3 ligase, such as substrate receptors or adaptor molecules of the E3 ligases. Such a "molecule" may be a "molecular glue" in context of this invention. As evident from the description herein as well as from the experimental data, such "molecular glues" may, in context of this invention, also be the chemical compounds or agents that are able to induce the desired ubiquitination of the target protein/protein of interest. These MG may be found or identified to degrade target proteins by inducing cooperative binding to E3 ligases. MG may include but are not limited to thalidomide and related immunomodulatory drugs (IMiDs), such as lenalidomide, pomalidomide and CC-885. In general, MG may induce cooperative binding with target proteins (neo-substrates) that are naturally not bound by a particular substrate receptor of a CRL (Figure 4). Thus, when studied in isolation, it would not be possible to develop ligands to target for instance the zinc-finger transcription factors IKZF1 and IKZF3. In this context, molecular glues may engage both proteins, POI and the E3 ligase, in a highly cooperative manner.The ternary complex formation comprising the E3 ligase, the MG and the POI may be enabled and stabilized by a range of protein-protein interactions (PPIs). These PPIs may contribute the extra stabilization energy required for molecular recognition. In this context, cooperativity by a MG may contribute to potency and selectivity of induced proteolysis and thus highlights the potential of MG.

As illustrated herein above, the present invention relates in its basis to methods for identifying/testing/screening compounds or agents which are able to induce ubiquitination of (a) protein(s) of interest said method(s) comprising the steps as characterized herein above and in the appended claims and wherein cells/cellular systems are employed which are manipulated to comprise an enhanced cullin-RING ubiquitin ligase (CRL) activity.

The compound or agent (to be identified/tested/obtained/screened) is determined to induce ubiquitination of the protein(s) of interest if the level of the at least one member of the E3 ligase complex is increased compared to the level determined in the absence of the chemical compound.

As used herein, "ubiquitin" refers to a polypeptide which is ligated to another polypeptide by ubiquitin ligase enzymes. The ubiquitin can be from any species of organism, preferably a eukaryotic species. Preferably, the ubiquitin is mammalian. More preferably, the ubiquitin is human ubiquitin. In a preferred embodiment, when ubiquitin is ligated to a target protein of interest, that protein is targeted for degradation by the 26S proteasome. Also encompassed by "ubiquitin" are naturally occurring alleles.

The ubiquitination of these proteins is mediated by a cascade of enzymatic activity. Ubiquitin is first activated in an ATP-dependent manner by an ubiquitin activating enzyme (E1). The C-terminus of an ubiquitin forms a high energy thiolester bond with E1. The ubiquitin is then passed to an ubiquitin conjugating enzyme (E2; also called ubiquitin carrier protein), also linked to this second enzyme via a thiolester bond. The ubiquitin is finally linked to its target protein to form a terminal isopeptide bond under the guidance of an ubiquitin ligase (E3). In this process, chains of ubiquitin are formed on the target protein, each covalently ligated to the next through the activity of E3. Thus, as used herein, the term "ubiquitination" refers to the covalent attachment of ubiquitin to a protein through the activity of ubiquitination enzymes.

E3 enzymes contain two separate activities: an ubiquitin ligase activity to conjugate ubiquitin to target proteins and form ubiquitin chains via isopeptide bonds, and a targeting activity to physically bring the ligase and target protein together. The specificity of the process is controlled by the E3 enzyme, which recognizes and interacts with the target protein to be degraded. Thus, as used herein, the term "ubiquitin ligase", "ubiquitin E3 ligase" or "E3 ligase" are used interchangeably and refer to an ubiquitination enzyme capable of catalyzing the covalent binding of an ubiquitin to another protein.

As used in accordance with the methods of the present invention, it is to be understood that ubiquitination of a protein of interest or ubiquitination of the at least one member of the E3 ligase complex may be induced if the protein of interest or the at least one member of the E3 ligase complex is in molecular proximity to a CRL. The term "molecular proximity" refers to the physical distance between two molecules that results in a biological event if the molecules are in close proximity to each other. It often but not always involves some chemical bonding, for example non-covalent bonds or covalent bonds.

Further, it is to be understood that ubiquitination of a protein of interest or the at least one member of the E3 ligase complex leads to degradation of the protein of interest or the at least one member of the E3 ligase complex by the ubiquitin proteasome system.

Thus, the method of the present invention equally relates to a method for identifying a (chemical) compound able to induce molecular proximity between a cullin-RING ubiquitin ligase (CRL) and a protein of interest,
the method comprising:
   (i) contacting a chemical compound or agent with a eukaryotic cell and manipulating the cell to comprise enhanced cullin-RING ubiquitin ligase (CRL) activity compared to the CRL activity in the absence of manipulating the cell to comprise enhanced CRL activity, wherein the cell expresses the protein of interest;
   (ii) determining the level of at least one member of an E3 ligase complex present in the cell;
wherein the chemical compound or agent is determined to induce molecular proximity between a cullin-RING ubiquitin ligase (CRL) and a protein of interest if the level of the at least one member of the E3 ligase complex is increased compared to the level determined in the absence of the chemical compound.

Equally, the method of the present invention relates to a method for identifying a chemical compound or agent able to induce degradation of a protein of interest,
the method comprising:
   (i) contacting a chemical compound or agent with a eukaryotic cell and manipulating the cell to comprise enhanced cullin-RING ubiquitin ligase (CRL) activity compared to the CRL activity in the absence of manipulating the cell to comprise enhanced CRL activity, wherein the cell expresses the protein of interest;
   (ii) determining the level of at least one member of an E3 ligase complex present in the cell;
wherein the chemical compound or agent is determined to induce degradation of a protein of interest if the level of the at least one member of the E3 ligase complex is increased compared to the level determined in the absence of the chemical compound.

The E3 ligases are classified into four families: HECT, RING-domain, U-box, and PHD-domain. According to the methods of the present invention, the E3 ligase is a cullin RING ubiquitin E3 ligase (CRL). As used herein, the term "cullin RING ubiquitin E3 ligase" or "CRL" are used interchangeably and refer to an ubiquitin ligase in a complex in which the catalytic core consists of a member of the cullin family and a RING domain protein; the core is associated with one or more additional proteins that confer substrate specificity. The RING domain proteins of the CRL mediate the transfer of ubiquitin from the E2 to the E3-bound substrate. In particular, the cullin RING ubiquitin E3 ligase (CRL) are modular multi-subunit complexes that all contain a common core comprising a cullin subunit and a zinc-binding RING domain subunit.

In particular, the cullin subunit folds into an extended structure that forms the backbone of CRLs. The C-terminal region of the cullin subunit forms a globular domain that wraps itself around the RING protein, which in turn recruits the E2 conjugating enzyme to form the enzymatic core. The N-terminal region of the cullin subunit, which resides at the opposite end of the elongated cullin structure, recruits substrate receptors via adapter proteins.

Cullin-based E3 ligases comprise a large family of ubiquitin ligases and are composed of several subunits, consisting of one of seven eukaryotic and/or mammalian, also human, cullin homologs (like eukaryotic CUL1, CUL2, CUL3, CUL4A/B, CUL5, CUL6 [for example CUL6 in C.elegans or in Drosophila] or CUL7, or CLU4A and/or CUL4B) that bind to the RING domain protein. The cullin N terminus mediates binding of cullin homolog-specific substrate recognition subunits. Binding of the substrate recognition subunits often but not always requires specific adaptor proteins that bridge the interaction with the cullin homologs. For instance, CUL1 is known to bind substrate recognition subunits containing a conserved F-box via the adaptor protein Skp1, thus forming SCF (Skp1-Cul1-F-box) E3 ligases, whereas CUL2 and CUL5 recruit substrate recognition subunits with a VHL or SOCS box, respectively, via the adaptor proteins Elongin B and C. In contrast, CUL3 is known to bind directly to substrate recognition subunits via their BTB domain (also known as POZ domain). CUL4A acts as an assembly factor that provides a scaffold for assembly of a RING-box domain protein (RBX1) and the adaptor protein Damaged DNA Binding Protein 1 (DDB1) (Angers et al., Nature, 2006. 443(7111):590-3). RBX1 is the docking site for the activated E2 protein, and DDB1 recruits substrate specificity receptors or DCAFs (DDB1-cullin4-associated-factors) to form the substrate-presenting side of the CUL4 complex (Angers et al., Nature, 2006. 443(7111):590-3; He et al., Genes Dev, 2006. 20(21):2949-54; Higa et al. Nat Cell Biol, 2006. 8(11): p. 1277-83). Cereblon (CRBN) interacts with damaged DNA binding protein 1 and forms an E3 ubiquitin ligase complex with CUL4 where it functions as a substrate receptor in which the proteins recognized by CRBN might be ubiquitinated and degraded by proteasomes. In one aspect of the method of the present invention, the CRLs comprise CUL1, CUL2, CUL3, CUL4A, CUL4B, CUL5, CUL6 or CUL7 [see also herein above]. In a preferred aspect of the method of the present invention, the CRL comprises CRLs which are able to induce degradation of the at least one member of the E3 ligase complex in the absence of a chemical compound or agent. The ability of a CRL to induce degradation of the at least one member of the E3 ligase complex in the absence of a chemical compound or agent can be determined by methods as described below in more detail and is further exemplified by the appended examples.

As provided herein, the at least one member of the E3 ligase complex refers to any protein that may be associated, directly or indirectly, with the E3 ligase complex. As used herein, the "at least one member of the E3 ligase complex" refers to a polypeptide comprising an amino acid of which the skilled person in the art is aware of. Preferably, the at least one member of the E3 ligase complex as used in accordance with the method of the present invention is at least one member which is in molecular proximity of the CRL, is able to be ubiquitinated by the CRL and is degradable by the CRL. For example, the at least one member of the E3 ligase complex may be a substrate receptor or an adaptor protein of the of the E3 ligase complex. In one aspect of the method of the present invention, the at least one member of the E3 ligase complex may be a substrate receptor or an adaptor protein of the of the E3 ligase complex. Preferably, the at least one member of the E3 ligase complex is a substrate receptor of the E3 ligase complex. For example, the substrate receptor may be cereblon (CRBN). The term "cereblon" or "CRBN" are used interchangeably and refer to the polypeptides ("polypeptides," "peptides" and "proteins" are used interchangeably herein) comprising the amino acid sequence any CRBN, such as a human CRBN protein (e.g., human CRBN isoform 1, GenBank Accession No. NP_057386; or human CRBN isoforms 2, GenBank Accession No. NP_001166953), and related polypeptides, including SNP variants thereof. Related CRBN polypeptides include allelic variants (e.g., SNP variants); splice variants; fragments; derivatives; substitution, deletion, and insertion variants; fusion polypeptides; and interspecies homologs, which, in certain aspects, retain CRBN activity and/or are sufficient to generate an anti-CRBN immune response.

As detailed in the embodiments above other examples of the relevant ligase substrate receptors DCAF15, DCAF16, DCAF1, DCAF5, DCAF8, DET1, FBXO7, FBXO22, KDM2A, orKDM2B. In context of the present invention, preferably cereblon (CRBN) or DCAF15 is used and is preferably overexpressed in order to obtain the desired cell/cellular system with enhanced cullin-RING ubiquitin ligase (CRL) activity and/or with constantly neddylated cullin-RING ubiquitin ligases/CRLs.

As used herein, the term "level of the at least one member of the E3 ligase complex" refers to the quantity of the molecular entity of the respective least one member of the E3 ligase complex in the cell. In other words, the concentration of the at least one member of the E3 ligase complex is determined. Suitable methods to determine the level of the at least one member of the E3 ligase complex are described below in more detail. In one aspect of the method of the present invention, the method further comprises determining the level of the at least one member of the E3 ligase complex in the absence of the chemical compound or agent. Typically, the level of the at least one member of the E3 ligase complex in the presence of the chemical compound or agent is compared to the level of the at least one member of the E3 ligase complex in the absence of the chemical compound or agent, and a chemical compound or agent inducing ubiquitination of a protein of interest is identified by comparing the level of the at least one member of the E3 ligase complex in the presence and absence of the chemical compound or agent. As used herein, the chemical compound or agent is determined to induce ubiquitination of the protein of interest if the level of the at least one member of the E3 ligase complex is increased compared to the level determined in the absence of the chemical compound or agent.

The skilled person is aware of method of how to determine whether the at least one member of the E3 ligase complex is increased compared to the level determined in the absence of the chemical compound. For example, a ratio (c1/c2) of the level of the at least one member of the E3 ligase complex determined in the presence of the chemical compound or agent (c1) to the level determined in the absence of the chemical compound or agent (c2) may be calculated to determine if the chemical compound or agent is able to induce ubiquitination of the protein of interest. For example, a ratio (c1/c2) of the level of the at least one member of the E3 ligase complex determined in the presence of the chemical compound or agent (c1) to the level determined in the absence of the chemical compound or agent (c2) of ≥ 1.5, preferably of 2, more preferably of 3 is determined to induce ubiquitination of the protein of interest. In particular, the ratio is determined in cells that are manipulated to comprise enhanced CRL activity. Such an enhanced CRL activity may also be obtained via (ectopic) overexpression(s), as discussed herein below and as illustrated for the substrate receptor in the appended examples. As discussed herein, the c1/c2 ratio may be determined via methods known in the art. Furthermore and also in accordance with this invention, the c1/c2 ratio may be determined in cells that are manipulated to comprise constantly neddylated CRLs. Such cells are described herein and are illustrated in the appended Examples. Without being limited to the cells of the examples, such cells (i.e. "wildtype cells" that are to be manipulated in order to obtain "constantly neddylated cells") comprise cancer cells, such as lung cancer cells, gastric cancer cells, melanoma cells, sarcoma cells, leukemia cancer cells, colon cancer cells or neuroblastoma cells.

Neddylation of CRLs, in particular constant neddylation of CRLs, is described herein and illustrated in the appended Examples. Further, methods of how to determine constantly neddylated CRLs in a cell are described below and are exemplified in the appended Examples. Furthermore, the skilled person in the art knows how to determine the levels and ratio by methods described below in detail. For example, the ratio may be determined by the level of fluorescence intensity of a fluorescence tag that is contacted with the at least one member of the E3 ligase complex in the presence of the chemical compound or agent to the level in the absence of the chemical compound or agent. In other words, the skilled person is aware of method of how to determine the neddylation status in a given cell. The gist of the present invention is a provision of cellular systems wherein via recombinant and/or chemical modification the neddylation status of the cullins (CUL1, CUL2, CUL3, CUL4A/B, CUL5, CUL6 or CUL7 constituting the various [E3] cullin RING ligases (CRL)) in a cell is modified/manipulated so that the CRL is constantly neddylated/hyperneddylated. A skilled person considers a CRL to be constantly neddylated/hyperneddylated if neddylation in said modified/manipulated cell is increased over a corresponding control cell. A control cell may be the same cell/cell line that has been modified/manipulated, but which did not undergo said modification/manipulation. The skilled person is readily in a position to measure, if desired, the neddylation status of a given CRL in a given cell. One example of such a measurement or determination is to perform an immunoblot analysis of said cullin in both the modified/modulated and the control cell and to quantify the ratio of neddylated versus non-neddylated cullin. For example, commercially available cullin antibodies might be employed for such an immunoblot analysis. Commercial antibodies to cullins are readily available to the skilled person and/or can be obtained and generated by the skilled person via routine methods. Examples of cullin antibodies that are commercially available comprise, *inter alia,* CUL4A Cell Signaling Technology #2699S; CUL2 Sigma-Aldrich SAB2501565; CUL4B Proteintech #12916-1-AP).

Cullins without a neddylation are of a lower molecular weight than corresponding neddylated cullins and can thus be resolved (see appended Figure 3). As documented in appended Example 3 and Figure 3, the neddylation status of cullins constituting CRLs may readily be assessed. As evident from figure 3, cells comprising a normal neddylation status or a stready-state neddylation status show two bands corresponding to a non-neddylated and a neddylated cullin, respectively. Average increase of neddylated to non-neddylated approximately 8 kDa. Constantly neddylated cells show preferentially/to a vast majority the culling band of the higher molecular weight (corresponding to the constant neddylation (i.e. hyperneddylation)).

The prediction whether a chemical/biochemical compound or agent is able to induce ubiquitination of a protein of interest can be assessed by the determination of a threshold or cut-off ratio of the level of the at least one member of the E3 ligase complex in the presence of the chemical compound or agent to the level in the absence of the chemical compound or agent. Hereby, the respective cut-off value for identifying a chemical compound or agent able to induce ubiquitination of a protein of interest refers to measurements of the level of the at least one member of the E3 ligase complex in the presence of the chemical compound or agent and the level in the absence of the chemical compound or agent. All values disclosed herein relating to the ratio between the level of the at least one member of the E3 ligase complex in the presence of the chemical compound to agent to the level in the absence of the chemical compound or agent are to be understood as "equal or above" a certain cut-off. For example, an aspect relating to the ratio of the level of the at least one member of the E3 ligase complex in the presence of the chemical compound or agent to the level in the absence of the chemical compound or agent of at least 1.5 is to be understood as relating to the ratio of the level of the at least one member of the E3 ligase complex in the presence of the chemical compound or agent to the level in the absence of the chemical compound or agent equal or above the cut-off value of 1.5. Cut-off values of the ratio between the level of the at least one member of the E3 ligase complex in the presence of the chemical compound to agent to the level in the absence of the chemical compound or agent may be determined by previously described methods known in the art. For example, methods are known to a skilled person for using the "Coefficient of Variation" in assessing variability of quantitative assays in order to establish cut-off values (Reed et al., Clin Diagn Lab Immunol.2002; 9(6):1235-1239).

As provided herein, the method of the present invention refers to an *in vivo* method. As used herein the term *"in vivo*" refers to the natural environment, e.g., an animal or a cell, and to processes or reactions that occur within a natural environment. In one aspect of the method of the present invention, the method is a cellular method, which encompasses any assay of cellular protein activity and content, including but not limited to whole or fixed-cell assays. Particulary, the cell used in the context of the method of the present invention is a eukaryotic cell. The term "eukaryotic cell" is used herein to mean any nucleated cell, i.e., a cell that possesses a nucleus surrounded by a nuclear membrane, as well as any cell that is derived by terminal differentiation from a nucleated cell, even though the derived cell is not nucleated. Examples of the latter are terminally differentiated human red blood cells. Mammalian, and particularly human, eukaryotes are preferred.

In certain aspect, the eukaryotic cell as used in the method of the present invention may be manipulated to comprise enhanced activity of the CRL compared to the CRL activity in the absence of manipulating the cell to comprise enhanced CRL activity. Preferably, the eukaryotic cell as used in the method of the present invention may be manipulated to comprise constantly nedddylated CRLs compared to the CRL activity in the absence of manipulating the cell to comprise constantly neddylated CRLs.

As used herein, the at least one member of the E3 ligase complex may be expressed in a eukaryotic host cell. As used herein, the term "host cell" refers to any cell that contains a heterologous nucleic acid. The heterologous nucleic acid can be a vector, such as a shuttle vector or an expression vector. As used herein, the terms "heterologous" or "exogenous" as applied to polynucleotides or polypeptides refers to molecules that have been rearranged or artificially supplied to a biological system and are not in a native configuration (e.g., with respect to sequence, genomic position or arrangement of parts) or are not native to that particular biological system. These terms indicate that the relevant material originated from a source other than the naturally occurring source, or refers to molecules having a non-natural configuration, genetic location or arrangement of parts. The terms "exogenous" and "heterologous" are sometimes used interchangeably with "recombinant."

Methods (i.e., means) for delivering vectors/constructs or other nucleic acids (such as in vitro transcribed RNA) into host cells such as mammalian cells are well known to one of ordinary skill in the art, and are not provided in detail herein. Any method for nucleic acid delivery into a host cell finds use with the invention. Methods for delivering vectors or other nucleic acid (such as RNA) into mammalian cells in culture (termed transfection) are routine, and a number of transfection methods find use with the invention. These include but are not limited to calcium phosphate precipitation, electroporation, lipid-based methods (liposomes or lipoplexes) such as Transfectamine^{®} (Life Technologies^{™}) and TransFectin^{™} (Bio-Rad Laboratories), cationic polymer transfections, for example using DEAE-dextran, direct nucleic acid injection, biolistic particle injection, and viral transduction using engineered viral carriers (termed transduction, using e.g., engineered herpes simplex virus, adenovirus, adeno-associated virus, vaccinia virus, Sindbis virus), and sonoporation. Any of these methods find use with the invention.

Suitable host cells for expression of the at least one member of the E3 ligase complex include eukaryotic cells and are well known in the art. Appropriate cloning and expression vectors for use with eukaryotic, in particular mammalian cellular hosts are well known in the art, e.g., Pouwels et al. Cloning Vectors: A Laboratory Manual, Elsevier, New York (1985). The vector may be a plasmid vector, a single or double-stranded phage vector, or a single or double-stranded RNA or DNA viral vector. Such vectors may be introduced into cells as polynucleotides, preferably DNA, by well known techniques for introducing DNA and RNA into cells. The vectors, in the case of phage and viral vectors also may be and preferably are introduced into cells as packaged or encapsulated virus by well known techniques for infection and transduction. Viral vectors may be replication competent or replication defective. In the latter case viral propagation generally will occur only in complementing host cells. Cell-free translation systems could also be employed to produce the protein using RNAs derived from the present DNA constructs.

Transcriptional and translational control sequences for mammalian host cell expression vectors may be excised from viral genomes. Commonly used promoter sequences and enhancer sequences are derived from Polyoma virus, Adenovirus 2, Simian Virus 40 (SV40), and human cytomegalovirus. DNA sequences derived from the SV40 viral genome may be used to provide other genetic elements for expression of a structural gene sequence in a mammalian host cell, e.g., SV40 origin, early and late promoter, enhancer, splice, and polyadenylation sites. Viral early and late promoters are particularly useful because both are easily obtained from a viral genome as a fragment which may also contain a viral origin of replication.

In one aspect of the method of the present invention, the host cell has been manipulated to comprise enhanced CRL activity. The person skilled in the art will readily understand that the term "enhanced activity" does not only refer to a complete activation of CRLs in a cell, but rather to an increase of their frequency of CRL activation over a threshold level. In particular, the skilled person will appreciate that when manipulating the cell to comprise an enhanced CRL activity as used in the methods of the present invention, enhanced CRL activity refers to an increased average number of CRLs that are activated compared to a wild type cell or a cell that has not been manipulated to comprise enhanced CRL activity. The skilled person is aware of the fact that the CRL activity of a cell may depend on the type of cell used. The term "wild-type" in the context of the present invention means a strain of a eukaryotic cell as it may occur in nature or may be isolated from the environment, which does not carry any genetically engineered mutations. The enhanced CRL activity may be induced by methods known in the art, e.g. by genetic or chemical manipulation of the cell as described below in detail. Thus, the activity of a CRL in a cell induced by genetic or chemical manipulation as used in the method of the present invention and described below in more detail is enhanced compared to the cell without genetic or chemical manipulation to enhance the CRL activity. Herein, a ratio (c1/c2) of the level of the at least one member of the E3 ligase complex in a cell that has been manipulated to comprise enhanced CRL activity (c1) to the level in a wild type cell or a cell that has not been manipulated to comprise enhanced CRL activity (c2) has been calculated to determine the enhanced CRL activity in a cell that has been manipulated to comprise enhanced CRL activity. For example, a ratio (c1/c2) of the level of the at least one member of the E3 ligase complex in a cell that has been manipulated to comprise enhanced CRL activity (c1) to the level in a wild type cell or a cell that has not been manipulated to comprise enhanced CRL activity (c2) of ≥ 0.75, preferably of 0.60, more preferably of 0.50 is determined to comprise enhanced CRL activity. In particular, the level of the at least one member of the E3 ligase complex in a cell that has been manipulated to comprise enhanced CRL activity is a cell that has been manipulated to comprise constantly neddylated CRLs. Neddylation of CRLs, in particular constant neddylation of CRLs, are described herein. Further, methods of how to determine constantly neddylated CRLs in a cell are described below and are exemplified in the appended Examples. Furthermore, the skilled person in the art knows how to determine the enhanced CRL activity based on the ratio of the level of the at least one member of the E3 ligase complex by methods described herein. For example, the enhanced CRL activity may be determined by the ratio of fluorescence intensity of a fluorescence tag that is contacted with the at least one member of the E3 ligase complex in a cell that has been manipulated to comprise enhanced CRL activity and in a wild type cell or a cell that has not been manipulated to comprise enhanced CRL activity.

In context of this invention, a cell without genetic or chemical manipulation refers to a cell which has not been manipulated to comprise enhanced CRL activity, for example a wild type cell without genetic or chemical manipulation or a cell which has been manipulated to comprise an at least one member of the E3 ligase complex. Typically, the level of the at least one member of the E3 ligase complex in a cell without genetic or chemical manipulation to comprise enhanced CRL activity is used as a control level. For example, the level of the at least one member of the E3 ligase complex in a wild type cell may be used as a control level. Hence, the control level is used for the determination of the CRL activity in a cell without genetic or chemical manipulation to comprise enhanced CRL activity, for example in a wild type cell. In another example, the control level may be determined in a cell without genetic or chemical manipulation to comprise enhanced CRL activity and which has been manipulated to comprise a modified at least one member of the E3 ligase complex. Methods of modifying the cell to comprise an at least one member of the E3 ligase complex are described below. Further, the skilled person is aware of the fact that the CRL activity of a cell may depend on the type of cell used.CRLs may be activated when dissociated from Cullin-associated NEDD8-dissociated protein 1 (CAND1) and/or Cullin-associated NEDD8-dissociated protein 2 (CAND2). The CAND1 gene encodes an essential regulator of Cullin-RING ubiquitin ligases, which are in involved in ubiquitinylation of proteins degraded by the ubiquitin proteasome system. The encoded CAND1 binds to unneddylated cullin-RING box protein complexes and acts as an inhibitor of cullin neddylation and of Skp1, cullin, and F box ubiquitin ligase complex assembly and activity (Liu et al., (2018) Molecular Cell 69, 773-786). In mammalian cell culture, this protein predominantly localizes to the cytoplasm. Alternative splicing results in multiple transcript variants. In one aspect of the method of the present invention, the cell manipulated to comprise enhanced CRL activity is manipulated by inactivation of CAND1, CAND2 or CAND1 and CAND2.

Cullins may be found covalently conjugated with an ubiquitin-like molecule, NEDD8 (neural-precursor-cell-expressed developmentally down-regulated 8). As used herein, the term "NEDD8" refer to a protein that in humans is encoded by the *NEDD8* gene. Nucleotide and amino acid sequences of NEDD8 proteins are known in the art. Non-limiting examples of NEDD8 sequences include *Homo sapiens* NEDD8, the nucleotide and amino acid sequences of which are set forth in GenBank Ace. Nos. NM_006156 and NP_006147, respectively; *Mus musculus* NEDD8, the nucleotide and amino acid sequences of which are set forth in GenBank Acc. Nos. NM_008683 and NP_032709, respectively (Kamitani et al. (1997) J Biol Chem 272:28557-28562; Kumar et al. (1992) Biochem Biophys Res Comm 185:1155-1161); and *Saccharomyces cerevisiae* Rub1, the nucleotide and amino acid sequences of which are set forth in GenBank Acc. Nos. Y16890 and CAA76516, respectively.

CRLs may be activated when CRLs are present in a neddylated state, i.e. upon neddylation. As used herein, the term "neddylation" refers to a type of protein modification process by which the ubiquitin-like protein NEDD8 is conjugated to the CRL through E1 activating enzyme (NAE; a heterodimer of NAE1 and UBA3 subunit), E2 conjugating enzyme (Ubc12, UBE2M) and E3 ligase (Gong et al. J. Biol. Chem. 2013; 274: 1203612042). This modification, termed neddylation, activates the E3 ligase activity of CRLs by promoting substrate ubiquitination.

In one aspect of the method of the present invention, the cell has been manipulated to comprise enhanced CRL activity by overexpressing proteins able to induce neddylation of the CRLs in the cells. Such proteins able to induce neddylation may comprise NAE, preferably NAE1 or UBE2M. As further examples, substrate receptors or core components of CRL ligases including adaptors (such as DDB1 or SKP1) or cullin backbones (such as human CUL1, CUL2, CUL3, CUL4A, CUL4B, CUL5, and CUL7 or complexes therof, like CULAA/B) may be overexpressed in the cells and/or cells to be employed in the methods of the present invention Accordingly, in a preferred aspect of the method of the present invention or in a preferred cell, the eukaryotic cell has been manipulated to comprise enhanced CRL activity by overexpressing NAE, preferably NAE1 or UBE2M, DCN1, NEDD8, RNF7, UBA3, UBE2F. This overexpression induces neddylation of the CRLs. In particular, CRL neddylation catalyzed by the NEDD8-activating enzyme (NAE1) induces a conformational rearrangement that enables the transfer of ubiquitin from the E2 enzyme to the ubiquitin-receiving substrate, which is recruited by the substrate receptor of the respective CRL. This modification may induce conformational changes, which promotes flexibility in the RING domain of cullin proteins and enhanced ubiquitin ligase activity. The neddylation system is similar to UPS (ubiquitin-proteasome system) in which ubiquitin activating enzyme E1, ubiquitin conjugating enzyme E2 (UBC) and ubiquitin-protein isopeptide ligase E3 are involved (Hershko, A. Cell Death Differ. 2005; 12: 1191-1197). Thus, as used herein, the terms "NAE" or "NEDD8 activating enzyme," refer to a protein capable of catalyzing the transfer of NEDDS's C terminus to the catalytic cysteine of NEDD8 E2, forming a thiolester-linked E2-NEDD8 intermediate (Gong and Yeh (1999) J Biol Chem 274:12036-12042; and Liakopoulos et al. (1998) EMBO J 17:2208-2214; Osaka et al. (1998) Genes Dev 12:2263-2268). NEDD8 E1 enzymes described in the art include a heterodimer of NAE1 (also referred to as APPBP1; amyloid beta precursor protein binding protein 1; and NEDD8-activating enzyme E1 regulatory subunit). Nucleotide and amino acid sequences of NAE1 proteins are known in the art. Non-limiting examples of NAE1 sequences include *Homo sapiens* NAE1, the nucleotide and amino acid sequences of which are set forth in GenBank Ace. Nos. NM_001018159 and NP_001018169, respectively; and *Mus musculus* NAE1, the nucleotide and amino acid sequences of which are set forth in GenBank Ace, Nos. NM_144931 and NP_659180, respectively. NEDD8 E2 enzymes play central roles in the E1-E2-E3 NEDD8 conjugation cascade. As used herein, the terms "NEDD8 conjugating enzyme," and "NEDD8 E2 enzyme" refer to a protein capable of transiently binding a NEDD8 E1 enzyme for generation and interacting with a NEDD8 E3 ligase. The two known NEDD8 conjugating enzymes are UBC12, which is also known as UBE2M, and UBE2F. In a preferred aspect of the method of the present invention, the cell has been altered to show enhanced CRL activity by overexpressing UBE2M able to induce neddylation of the CRLs. Nucleotide and amino acid sequences of UBE2M proteins are known in the art. Non-limiting examples of UBE2M sequences include *Homo sapiens* UBE2M, the nucleotide and amino acid sequences of which are set forth in GenBank Acc. Nos. NM_003969 and NP_003960, respectively; *Mus musculus* UBC12, the nucleotide and amino acid sequences of which are set forth in GenBank Ace. Nos. NM_145578 and NP_663553, respectively; and *Saccharomyces cerevisiae* UBC12, the nucleotide and amino acid sequences of which are set forth in GenBank Acc. Nos. NM_001182194 and NP_013409, respectively.

In one aspect of the method of the present invention, the cell manipulated to comprise enhanced CRL activity is manipulated to comprise constantly neddylated CRLs. The person skilled in the art will readily understand that the term "constantly neddylated" does not only refer to a complete neddylation of CRLs in a cell, but rather to an increase of their neddylation frequency over a threshold level. The term "constantly" is intended to mean that the neddylation of the CRL is substantially uninterrupted. In other words, the term "constantly" refers to the neddylated state of the CRL, which is substantially not reversed to a deneddylated state. In particular, the skilled person will appreciate that when manipulating the cell to comprise constantly neddylated CRLs as used in the methods of the present invention, constantly neddylated CRLs refer to an increased average number of CRLs that are constantly neddylated compared to a wild type cell or a cell that has not been manipulated to comprise constantly neddylated CRLs. The skilled person is aware of the fact that the level of neddylated CRLs of a cell may depend on the type of cell used. Methods for manipulating a cell to comprise constantly neddylated CRLs are described below in detail. Thus, the number of constantly neddylated CRLs in a cell induced by manipulation as used in the method of the present invention is enhanced compared to the cell without manipulation. Herein, a ratio (c1/c2) of the level of the at least one member of the E3 ligase complex in a cell that has been manipulated to comprise constantly neddylated CRLs (c1) to the level in a wild type cell or a cell that has not been manipulated to comprise constantly neddylated CRLs (c2) is calculated to determine the constantly neddylated CRLs in a cell manipulated to comprise constantly neddylated CRLs. For example, a ratio (c1/c2) of the level of the at least one member of the E3 ligase complex in a cell that is manipulated to comprise constantly neddylated CRLs (c1) to the level in a wild type cell or a cell not manipulated to comprise constantly neddylated CRLs (c2) of ≥ 0.75, preferably of 1.00, more preferably of 1.5 is determined to comprise constantly neddylated CRLs. Vice versa, a ratio of <0.5 may be determined not to comprise constantly neddylated CRLs. In a particular aspect, the ratio is determined in cells that are manipulated to comprise constantly neddylated CRLs to cells that are not manipulated to comprise constantly neddylated CRLs. Neddylation of CRLs, in particular constant neddylation of CRLs, is described herein. Further, methods of how to determine constantly neddylated CRLs in a cell are described herein. As shown in the appended examples, the ratio of the level of the at least one member of the E3 ligase complex in a cell manipulated to comprise constantly neddylated CRLs (c1) to the level in a wild type cell or a cell not manipulated to comprise constantly neddylated CRLs (c2) can be determined by Western Blot analysis as shown for the CRL comprising CUL4A. In the Figures 1 C to F, Figures 2 B to D and Figure of the Examples, the CRL reveals two very clear and distinct bands, wherein the lower corresponds to the non-neddylated fraction and the higher to the neddylated fraction of the CRL comprising CUL4A. Thus, the ratio as used in the context of the method of the present invention may refer to the ratio of the higher band to the lower band in order to determine the constantly neddylated CRLs in a cell manipulated to comprise constantly neddylated CRLs. The skilled person in the art knows how to determine the constantly neddylated CRLs in a cell based on the ratio of the level of the at least one member of the E3 ligase complex and how to determine the levels by methods described herein. The cell without manipulation or in the absence of a manipulation refers to a cell which is not manipulated to comprise enhanced CRL activity, preferably constantly neddylated CRLs, for example a wild type cell without genetic or chemical manipulation or a cell which is manipulated to comprise an at least one member of the E3 ligase complex.

Neddylation may be reversed by the COP9 signalosome (CSN), which enzymatically removes NEDD8 from a cullin molecule. Thus, the CSN is a central component of the activation and remodeling cycle of cullin-RING E3 ubiquitin ligases (Schlierf et al., Nat.Commun. 7, 13166 (2016)). The human CSN consists of nine protein subunits (COPS1-7A, 7B,8), of which COPS5 contains a metalloprotease motif that provides the catalytic centre to the complex. COPS5 exhibits proper deneddylating activity only in the context of the holocomplex and only the fully assembled CSN is competent to specifically remove NEDD8 from CRLs. Genetic or pharmacologic perturbation, e.g. by CSN interference such as by an COPS5 inhibitor, may be employed to interfere with the ubiquitin proteasome system in a unique manner by enforcing the auto-inactivation of only a subset of CRLs.

As used herein, the eukaryotic cell comprising constantly neddylated CRLs may be manipulated genetically or chemically to comprise constantly neddylated CRLs. In one aspect of the method of the present invention, the eukaryotic cell comprises constantly neddylated CRLs without genetically manipulating the cell to comprise constantly neddylated CRLs. Thus, the eukaryotic cell as used in the context of the present invention may comprise a wild type eukaryotic cell. As used herein, the term "wild type" refers to a cell whose genome is in a state such as arose naturally by evolution. The term is used both for the whole cell and for individual genes. The term "wild type" therefore in particular does not include such cells or such genes whose gene sequences have been altered at least partially by man by recombinant methods. In another aspect of the present invention, the control or "wild type" cell may comprise a cell which has been manipulated to comprise an at least one member of the E3 ligase complex but has not been (genetically) manipulated to comprise enhanced CRL activity or constantly neddylated CRLs.

In a preferred aspect of the method of the present invention, the eukaryotic cell manipulated to comprise constantly neddylated CRLs is manipulated by a specific inhibitor of the CSN or a subunit thereof. Preferably, the eukaryotic cell manipulated to comprise constantly neddylated CRLs is manipulated by contacting the cell with an inhibitor of the COP9 signalosome (CSN), like CSN5i-3.

As shown in the Examples of the present invention, the induced neddylation of the CRL, e.g. by genetic or pharmacologic perturbation such as by COPS5 inhibitors interfering with CRLs, resulted in ubiquitination of the at least one member of the E3 ligase complex, the substrate receptor (SR) by the CRL in which it is assembled, a process denoted as autodegradation. Moreover, treatment with a COPS5 inhibitor resulted in autodegradation of the SR by the CRL in which it is assembled. A less prominent ubiquitination of the SR by the CRL was shown in CAND1 deficient CRLs. VHL levels, the SR of the CRL2^{VHL} E3 ligase, were not autodegraded. In sum, it was shown that upon genetic or chemical manipulation of the CSN or CAND1, the SR of the CRL in which they are assembled were destabilized via continuous degradation. Mechanistically, this is due to a failure to inactivate CRLs that have already accomplished the ubiquitination of their substrate. As a result, the associated E2 starts to ubiquitinate the SR (i.e. CRBN), leading to its proteasomal degradation.

In one aspect of the method of the present invention, the cell manipulated to comprise constantly neddylated or hyper-neddylated CRLs may manipulated by use of a hypomorphic mutation or by inactivation of at least one member of the COP9 signalosome (CSN). In a more preferred aspect of the method of the present invention, the at least one member one member of the COP9 signalosome (CSN) may be the COPS8 or COPS7B subunit. Further examples and means and methods to obtain an eukaryotic cell to be employed in context of the inventive methods are provided and illustrated herein.

As used herein, the term "hypomorphic mutation" refers to a genetic variant that results in a reduction-in-function of a particular gene as generally understood by those skilled in the art. The hypomorphic state can be caused by a mutation in the endogenous copy of a gene, or can be caused by epi-allelic factors such as pharmaceutical inhibitors or dominant genetic inhibitors, like inhibitory RNA molecules e.g., shRNA molecules, siRNA molecules, sgRNAmolecules and the like. A "hypomorphic mutation" may refer to a version of a gene having a lower expression level and/or level of activity of its gene product relative to wild-type levels. A hypomorphic mutation is to be distinguished from inactivation mutations. An inactivation mutation refers to a complete or substantially complete loss of function of such gene. As used herein, the term "inactivation" refers to a genetic variant that results in a loss-of-function of a particular gene as generally understood by those skilled in the art. The inactivation may be caused by a mutation in the endogenous copy of a gene or can be caused by various synthetic or natural agents or materials that can inhibit the at least one member of the CSN at the transcriptional and/or translational level. Such synthetic or natural agents or materials may include but are not limited to small molecules, proteins including antibodies and polypeptides, and nucleic acid molecules including such as RNA and DNA for example antisense oligonucleotides, siRNA, shRNA or miRNA, or any combinations thereof. An inactivation by a mutation in the endogenous copy of a gene may be caused by a knock out. Preferably, the inactivation may be performed by Cas9/CRISPR (Clustered Regularly interspaced Short Palindromic Repeats). The Cas9/CRISPR system exploits RNA-guided DNA-binding and sequence-specific cleavage of target DNA. A guide RNA (gRNA) contains nucleotides that are complementary to a target genomic DNA sequence upstream of a genomic PAM (protospacer adjacent motifs) site and a constant RNA scaffold region. The Cas (CRISPR-associated) 9 protein binds to the gRNA and the target DNA to which the gRNA binds and introduces a double-strand break in a defined location upstream of the PAM site. Cas9 harbors two independent nuclease domains homologous to HNH and RuvC endonucleases, and by mutating either of the two domains, the Cas9 protein can be converted to a nickase that introduces single-strand breaks (Cong, L. et al. Science 339, 819-823 (2013)).

As used herein, the term "Cas protein" refers to an essential protein component in the CRISPR/Cas system, forms an active endonuclease or nickase when complexed with two RNAs termed CRISPR RNA (cNA) and transactivating cNA (tracNA). The information on the gene and protein of Cas are available from GenBank of National Center for Biotechnology Information (NCBI), without limitation. The CRISPR-associated (cas) genes encoding Cas proteins are often associated with CRISPR repeatspacer arrays. More than forty different Cas protein families have been described. Of these protein families, Casl appears to be ubiquitous among different CRISPR/Cas systems. There are three types of CRISPR/Cas system. Among them, Type II CRISPR/Cas system involving Cas9 protein and cNA and tracNA is representative and is well known.

As used herein, the term "guide RNA" refers to a RNA which is specific for the target DNA and can form a complex with Cas protein and bring Cas protein to the target DNA. In the present invention, the guide RNA may consist of two RNA, i.e., CRISPR RNA(cNA) and transactivating cNA(tracNA) or be a singlechain RNA(nNA) produced by fusion of an essential portion of cNA and tracNA.

In one aspect of the method present disclosure, the cell has been manipulated to comprise enhanced CRL activity prior to contacting a chemical compound or agent with the cell. In another aspect of the method of the present disclosure, the cell has been manipulated to comprise enhanced CRL activity after contacting a chemical compound or agent with the cell. As shown in Figures 2 B to D of Example 1, the cells were first treated with the inhibitor CSN5i-3 and afterwards with the small molecules SNS-THAL-032, CC-885 and dBET6 at the indicated exposure times. In Figure 3 of Example 3, cells were first treated with the small molecule CC-885 and afterwards with the inhibitor CSN5i-3. In both Examples, it was shown that downregulation of CRBN could be rescued in the presence of the small molecules recruiting the neo-substrates (CDK9 for SNS-THAL-032, GSPT1 for CC-885, and BRD2, BRD3, and BRD4 for dBET6). Hence, it could be demonstrated that the cell manipulated, preferably chemically manilulated, to comprise enhanced CRL activity, in particular constantly neddylated CRLs, can be manipulated prior or after contacting the chemical compound or agent to the cell in order to determine a chemical compound or agent to induce ubiquitination of a POI. In one aspect of the method of the present disclosure, the cell has been manipulated prior to contacting the cell with the chemical compound or agent in order to determine a chemical compound or agent to induce ubiquitination of a POI. For example, the cells have been manipulated for 1h to 2h prior to contacting the cells to the chemical compound or agent. In another aspect of the method of the present disclosure, the cell has been manipulated after contacting the cell to the chemical compound or agent in order to determine a chemical compound or agent to induce ubiquitination of a POI. For example, the cells have been contacted 15 to 30 min with a chemical compound or agent prior to manipulating the cells. In one aspect of the method of the present invention, chemical manipulation of the cell is induced by contacting the cell with a specific CRL inhibitor. Preferably, the inhibitor is CSN5i-3. For example, the concentration of CSN5i-3 contacted with the cell may be in the range of 25 nM-1 uM

As used herein, the term "target", "target protein", "POI" or "target protein of interest" are used interchangeably and refer to a protein other than ubiquitin to which ubiquitin may be ligated by ubiquitination enzymes. For example, a target protein may be modified, e.g. by an associated enzyme such as a tyrosine kinase, so that it becomes accessible to a E3 ligase and ligated by ubiquitin. A target protein may be modified upon a chemical compound or agent associating with an enzyme such as a tyrosine kinase, which, in turn, enables the enzyme to associate with a target protein, thereby modifiying the target protein. In another embodiment, target proteins refer to any protein that is able to bind the chemical compound or agent as used in the present invention. Thus, the target protein as used herein refers to any protein having a structure that is suitable for binding to the chemical compound or agent. In one aspect, the target protein of the present invention is suitable for binding to the chemical compound or agent in its free state. For example, the target protein of the present invention may bind to a heterobifunctional compound as described herein in its free state. In another aspect, the target protein of the present invention is suitable for binding to the chemical compound or agent upon binding to the CRL. For example, the target protein of the present invention may bind to a molecular glue as described herein upon binding to the CRL. Preferably, the target as used in accordance with the method of the present disclosure is endogenously expressed by the cell. The term "endogenous" as used herein with reference to genes or polypeptides expressed by genes, refers to genes or polypeptides that are native to the cell and are not derived from another organism.

In one aspect of the method of the present disclosure, the protein of interest is encoded by an oncogene-addiction (OA) gene such as a kinase or a non-oncogene addiction (NOA) gene such as a transcription factor. "Oncogene-addiction genes" refer to the dependence of the gene for a cancer cell to proliferate. As used herein, the "dependence (depending)" concerning the proliferation of the cell refers to the state of the oncogene addiction or the addiction, where the cell proliferates depending on the particular oncogene. Whether or not the cell proliferates depending on the particular oncogene can be confirmed by treating the cell with an inhibitor of the particular oncogene addiction gene and then evaluating a proliferation ability of the treated cell. The proliferation ability can be evaluated by, for example, an MTT assay or an MTS assay. It is known that cell death due to apoptosis can be induced, when the cell in the oncogene addiction for the particular oncogene is treated with the inhibitor of such an oncogene. Therefore, the oncogene addiction in the cell for the particular oncogene may be confirmed by evaluating whether or not the apoptosis can be induced by inhibition of the oncogene. The induction of the apoptosis can be evaluated by, for example, a TUNEL assay, detection of active caspase, or detection of annexin V. The term "non-oncogene addiction gene" refers to a gene that is required for maintenance of the tumorigenic state, wherein non-oncogene addiction genes do not undergo oncogenic mutations or functionally significant genomic alterations in tumors. In other words "non-oncogene addiction genes" are genes where cancer cells come to be highly dependent for growth and survival on the functions of genes that are themselves not oncogenes (N. L. Solimini, J. Luo, S. J. Elledge, Cell 130, 986 (2007); Luo, et al., Principles of cancer therapy: oncogene and nononcogene addiction. Cell 2009;136:823-37). Specificity protein transcription factors (SpTFs) are a prototypical example of "non-oncogene addiction" by cancer cells. In particular, Sp1, Sp3, and Sp4 are highly expressed in cancer cells and tumors compared to non-tumor tissues.

In a particularly preferred aspect of the method of the present invention, the target protein of interest is selected from the group consisting DNA-binding proteins including transcription factors, RNA binding proteins, scaffolding proteins, GTPases, solute carriers, kinases, phosphatases, bromodomain- and chromodomain containing proteins, and G-protein coupled receptors.

As used herein, DNA-binding proteins refer to proteins that have DNA-binding domains and thus have a specific or general affinity for single- or double-stranded DNA. DNA-binding proteins include transcription factors which modulate the process of transcription, various polymerases, nucleases which cleave DNA molecules, and histones which are involved in chromosome packaging and transcription in the cell nucleus. As used herein, the term "transcription factor" refers to any polypeptide that may act by itself or in combination with at least one other polypeptide to regulate gene expression levels and the term is not limited to polypeptides that directly bind DNA sequences. The transcription factor typically increases expression levels. However, in some cases it may be desirable to suppress expression of a particular pathway. The transcription factor may be a transcription factor identified by sequence analysis.

As used herein, RNA-binding proteins refer to proteins that bind to RNA molecules, are generally found in the cytoplasm and nucleus, and are important in formatin ribonucleoproteins (RNP). RNA-binding proteins contain various structural motifs, such as RNA recognition motif (RRM), dsRNA binding domain, zinc finger and others.

As used herein, scaffolding proteins refer to proteins that regulate signaling pathways. Scaffolding proteins interact and/or bind with multiple members of a signaling pathway, tethering them to complexes. In such pathways, they regulate signal transduction and help localize pathway components that may be organized in complexes to specific areas of the cell such as the plasma membrane, the cytoplasm, the nucleus, the Golgi, endosomes, and the mitochondria. An example is the Ste5 scaffold in the mitogenactivated protein kinase (MAPK) pathway.

As used herein, GTPases refer to hydrolase enzymes that bind and hydrolyze GTP. GTPases are key elements in the signal transduction at the intracellular domain of transmembrane receptors, protein biosynthesis at the ribosome, control and differentiation during cell division, translocation of proteins through membranes and in the transport of vehicles with the cell. Examples of GTPases include but are not limited to RAS family proteins selected from the group of KRAS, NRAS and HRAS.

As used herein, solute carriers function to regulate the uptake and efflux of compounds such as sugars, amino acids, nucleotides, inorganic ions and drugs into and out of cells and organelles. Carriers can be divided into active and passive transporters. Passive transporters, also known as facilitated transporters, allow the passage of solutes across membranes down an electrochemical gradient. Active transporters consume energy and create an ion/solute gradient that actively moves compounds across membranes. This latter form of carrier is further characterised as a primary or secondary carrier in accordance with the directness of coupling to ATP hydrolysis. For example, primary ATP-dependent transporters include the ABC transporter family and ion pumps such as ATPases.

The term "kinase" refers to any enzyme that catalyzes the addition of phosphate groups to a protein residue; for example, serine and threonine kinases catalyze the addition of phosphate groups to serine and threonine residues.

As used herein, the term "phosphatase" includes a protein or polypeptide, e.g., an enzyme, or another non-proteinaceous molecule which is capable of facilitating, e.g., catalyzing, the removal of a phosphate group from, for example, a protein or polypeptide which is phosphorylated. Phosphatases can have a specificity for (i.e. a specificity to dephosphorylate) serine/threonine residues, tyrosine residues, or both serine/threonine and tyrosine residues.

Kinases and Phosphatases play a role in signaling pathways associated with cellular growth. For example, protein kinases and protein phosphatases are involved in the regulation of signal transmission from cellular receptors, e.g., growth-factor receptors; entry of cells into mitosis; and the regulation of cytoskeleton function, e.g., actin bundling.

Bromodomains refer to conserved protein structural folds which bind to N-acetylated lysine residues that are found in some proteins. The BET family of bromodomain containing proteins is comprised of four members (BRD2, BRD3, BRD4 and BRDT). Each member of the BET family employs two bromodomains to recognize N-acetylated lysine residues found primarily, but not exclusively, on the amino-terminal tails of histone proteins. These interactions modulate gene expression by recruiting transcription factors to specific genome locations within chromatin. For example, histone-bound BRD4 recruits the transcription factor P-TEFb to promoters, resulting in the expression of a subset of genes involved in cell cycle progression (Yang et al, Mol. Cell. Biol. 28: 967-976 (2008)). BRD2 and BRD3 also function as transcriptional regulators of growth promoting genes (LeRoy et al., Mol. Cell 30: 51-60 (2008)). BET family members were recently established as being important for the maintenance of several cancer types (Zuber et al., Nature 478: 524-528 (2011); Mertz et al; Proc. Nat'l. Acad. Sci. 108: 16669-16674 (2011); Delmore et al, Cell 146: 1-14, (2011); Dawson et al., Nature 478: 529-533 (2011)).

Chromodomains refer to structural domains found in proteins related to remodeling and manipulation of chromatin. In mammals, chromodomain-containing proteins are responsible for aspects of gene regulation related to chromatin remodeling and formation of heterochromatin regions. Examples of Chromo-domain-containing proteins include transcriptional repressors HP1 and Polycomb (Pc), and the human retinoblastoma binding protein (RBP-1).

G protein coupled receptors (GPCR) refer to a guanine nucleotide-binding proteincoupled receptor. In particular, GPCRs are seventransmembrane receptors that are located in the cell membranes of a wide range of organisms, including mammals, plants, microorganisms, and invertebrates. Some examples of GPCRs include betaadrenergic receptors, which bind epinephrine; prostaglandin E₂ receptors, which bind inflammatory substances called prostaglandins; and rhodopsin, which contains a photoreactive chemical called retinal that responds to light signals received by rod cells in the eye.

For example, the target protein as used in accordance with the present invention may be selected from the group consisting of CDK9, GSPT1 and BRD2/3/4, but not limited thereto.

As used herein, the term "BRD4" or "Brd4"relates to Bromodomain-containing protein 4 and is a protein in humans encoded by the BRD4 gene. BDR4 is a member of the BET (bromodomain and extra terminal domain) family, along with BRD2, BRD3, and BRDT. BRD4, similar to its BET family members, contains two bromodomains that recognize acetylated lysine residues. BRD4 is an exemplary, non-enzymatic protein target. BRD4 is a transcriptional co- activator involved in dynamic transcriptional activation and elongation. BRD4 binds to enhancer and promoter regions adjacent to target genes, via recognition of side-chain acetylated lysine on histone proteins and transcription factors (TFs) by twin acetyl-lysine binding modules or bromodomains. Recently, a first direct-acting inhibitor of BET bromodomains (JQ1) was developed (P. Filippakopoulos et al, Nature 468, 1067-1073 (2010)), that displaces BRD4 from chromatin leading to impaired signal transduction from master regulatory TFs to RNA Polymerase II ( B. Chapuy et al.., Cancer Cell 24, 777-790 (2013); J. E. Delmore et al., Cell 146, 904-917 (201 1); J. Loven et al, Cell 153, 320-334 (2013).). Molecular recognition of the BRD4 bromodomains by JQI is stereo-specific, and only the (+)-JQI enantiomer (JQI S; from here forward JQI ) is active; the (-)-JQI enantiomer (JQ1 R) is inactive. Silencing of BRD4 expression by RNA interference in murine and human models of acute myeloid leukemia (AML) elicited rapid transcriptional downregulation of the MYC oncogene and a potent anti-proliferative response ( J. E. Delmore et al., Cell 146, 904-917 (201 1); J. Zuber et al., Nature 478, 524-528 (201 1 )). These and other studies in cancer, inflammation ( E. Nicodeme et al., Nature 468, 1 1 19-1 123 (2010)) and heart disease (P. Anand et al., Cell 154, 569-582 (2013); J. D. Brown et al., Mol. Cell 56, 219-23 1 (2014)), establish a desirable mechanistic and translational purpose to target BRD4 for selective degradation.

As used herein, the term "GSPT1" refers to G1 to S phase transition protein 1 homolog and is involved in translation termination in response to the termination codons UAA, UAG, and UGA, and is also involved in regulation of mammalian cell growth. GSPT1 stimulates the activity of eRF1 and is a component of the transient SURF complex, which recruits UPF1 to stalled ribosomes in the context of nonsense-mediated decay (NMD) of mRNAs.

As used herein, CDK9 refers to a member of the cyclin-dependent protein kinase (CDK) family and is involved in the regulation of transcription. It can phosphorylate various substrates involved in the control of transcription and cell-cycle progression in response to different stimuli. CDK9 has a key role in the control of transcription by RNA polymerase II. CDK9 responds specifically to several cytokines, including tumor necrosis factor and interleukin-6, indicating that it might have special roles in the regulation of a variety of physiological processes, especially immune responses, inflammation, cell activation, and differentiation.

In one aspect of the method of the present invention, the level of the at least one member of the E3 ligase complex is determined by addition of a binding agent specific for said at least one member of the E3 ligase complex.

For example, the level of the at least one member of the E3 ligase complex by addition of a binding agent specific for said at least one member of the E3 ligase complex can be determined by an immunoassay. Preferred detection methods comprise immunoassays in various formats such as for instance radioimmunoassays, chemiluminescence- and fluorescence- immunoassays, Enzyme-linked immunoassays (ELISA), Luminex-based bead arrays, protein microarray assays, assays suitable for point-of-care testing and rapid test formats such as for instance immune-chromatographic strip tests. Such an assay may be based on the binding of an analyte to be detected to one or more binding agents specific for said at least one member of the E3 ligase complex with a certain affinity. As used herein, an immunoassay is a biochemical test that measures the presence or concentration of a macromolecule/polypeptide in a solution through the use of an antibody or immunoglobulin as a binding agent specific for said at least one member of the E3 ligase complex. According to the invention, the antibodies may be monoclonal as well as polyclonal antibodies. Thus, at least one antibody is a monoclonal or polyclonal antibody. In certain aspects, the level of the marker is determined by high performance liquid chromatography (HPLC). In certain aspects, the HPLC can be coupled to an immunoassay. For example, in a sandwich immunoassay, two antibodies are applied for, e.g., one marker such as the at least one member of the E3 ligase complex.

The method according to the present invention can furthermore be embodied as a method, wherein the sandwich complexes formed by the antibody/antibodies and the marker, e.g., the at least one member of the E3 ligase complex, which is to be detected uses two antibodies, wherein both antibodies are labeled with parts of a detection system, which leads to generation of a signal or triggering of a signal if both antibodies are integrated into a single sandwich. It also envisaged herein that the level of the at least one member of the E3 ligase complex can be, for example, determined by mass spectrometric methods or by a high performance liquid chromatography (HPLC) method, which can be coupled to an immunoassay, or a mass-spectrometric based approach. The skilled person understands that any available assay can be used as long as the level of the at least one member of the E3 ligase complex can be reliably determined.

In one aspect, the method is an immunoassay comprising the steps of:
a) contacting the sample with
   (i) a first antibody or an antigen-binding fragment or derivative thereof specific for a first epitope of the at least one member of the E3 ligase complex, and
   (ii) a second antibody or an antigen-binding fragment or derivative thereof specific for a second epitope of the at least one member of the E3 ligase complex; and
b) detecting the binding of the first and second antibodies or antigen-binding fragments or derivates thereof to the at least one member of the E3 ligase complex.

Typically, in this context one of the antibodies is labeled and the other antibody is bound to a solid phase or can be bound selectively to a solid phase. For example, the first antibody and the second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to specific for the at least one member of the E3 ligase complex, a measurable signal which permits detection of the resulting sandwich complexes in the measuring solution is generated.

In principle, all labeling techniques which can be applied in assays of said type can be used, such as labeling with radioisotopes, enzymes, fluorescence-, chemoluminescence- or bioluminescence labels and directly optically detectable color labels, such as gold atoms and dye particles.

In one aspect of the method of the present invention, the level of the at least one member of the E3 ligase complex is determined by addition of a fluorescence binding agent specific for said at least one member of the E3 ligase complex. Thus, the level of the at least one member of the E3 ligase complex is determined by fluorescence.

As used herein, the term "fluorescence" is used herein as known in the art. Preferably, the term "fluorescence" refers to the emission of light of a longer wavelength by a molecule or a group of molecules that have been excited by light of a shorter wavelength. Alternatively, the term fluorescence may also include two-photon effects, thus the emission of one photon by a molecule excited with two photons of a longer wavelength. Furthermore, other photophysical effects such as effects dependent on triplet states such as phosphorescence may be included. As described above, the term "determined by fluorescence" preferably refers to the detection of the alterations of the fluorescent signal over time may be measured by (i) detecting alterations of the fluorescence intensity in a fluorescence spectrophotometer or in a microscopic device, (ii) detecting alterations of the fluorescence spectrum in a fluorescence spectrophotometer or in a microscopic device, (iii) detecting alterations of the mobility of the fluorescent molecule in a suitable device such as via a microscopic device, via fluorescence correlation spectroscopy (FCS), via fluorescence cross- correlation spectroscopy (FCCS) or via a fluorescence depolarization assay, via fluorescence resonance energy transfer (FRET)-based methods or via an amplified luminescence proximity homogeneous assay (ALPHA). These detection techniques may be known to a person skilled in the art. Preferably, the detection of the alterations of the fluorescent signal over time may be measured by detecting alterations of the fluorescence intensity. The level of the at least one member of the E3 ligase complex may be defined by the change in fluorescence with time, i.e., the slope of the line in the fluorescence versus time.

In one aspect of the method of the present disclosure, the at least one member of the E3 ligase complex comprises a modification allowing its direct or indirect detection. In particular, the at least one member of the E3 ligase complex may be modified to express a tagged at least one member of the E3 ligase complex. As used herein, the term "tag" as used in protein tags refers generally to peptide sequences that are genetically fused to other protein open reading frames, thereby producing recombinant fusion proteins. Ideally, the fused tag does not interfere with the native biological activity or function of the larger protein to which it is fused.

In one aspect of the method of the present invention, the at least one member of the E3 ligase complex is tagged and the amount of tag is determined. In one preferred aspect of the method of the present invention, the signal of the tag varies with the extent of degradation of the at least one member of the E3 ligase complex by the CRL. The tagged at least one member of the E3 ligase complex can be produced by culturing a recombinant cell transformed with a tag nucleic acid sequence that encodes a peptide or polypeptide including the tag attached to either the carboxyl and/or amino terminal end of the at least one member of the E3 ligase complex.

In one aspect of the method of the present invention, the at least one member of the E3 ligase complex is tagged with a molecule to which an interaction member specifically binds. In a preferred aspect of the method of the present disclosure, the modification of the at least one member of the E3 ligase complex is the addition of a fluorescent tag including split fluorescent tag, a luciferase or a peptide binding a reporter enzyme such as a luciferase.

Fluorscent tags may include but are not limited to GFP and GFP-like proteins. GFP-like proteins are an expanding family of homologous, 25-30 kDa polypeptides sharing a conserved 11 beta-strand "barrel" structure. The GFP-like protein family currently comprises some 100 members, cloned from various *Anthozoa* and *Hydrozoa* species, and includes red, yellow and green fluorescent proteins and a variety of nonfluorescent chromoproteins (Verkhusha et al., supra). A wide variety of fluorescent protein labeling assays and kits are commercially available, encompassing a broad spectrum of GFP spectral variants and GFP-like fluorescent proteins, including cyan fluorescent protein, blue fluorescent protein, yellow fluorescent protein, etc., DsRed and other red fluorescent proteins (Zimmer, 2002, Chem. Rev. 102: 759-781; Zhang et al., 2002, Nature Reviews 3: 906-918; Clontech, Palo Alto, Calif.; Amersham, Piscataway, N.J.). Method of modifying a protein by the addition of a fluoresecent tag are well known in the art.

Split fluorescent tags refer to tags in which two fragments can associate to form a fully functional fluorescence tag. Complementing split fluorescence proteins have been widely used for protein labeling, visualization of subcellular protein localization and detection of cell-cell contact. In one aspect of the present disclosure, the split fluorescent tag may be a split fluorescent protein. For example, GFP may be used as a self complementing split fluorescent protein. Methods of modifying a protein by the addition of a split fluorescent tag are well known in the art.

Luciferases refers to one or more oxygenases that catalyze a light emitting reaction. Thus, luciferase refers to an enzyme or photoprotein that catalyzes a reaction that produces bioluminescence. Luciferases of the disclosure can be recombinant or naturally occurring, or a variant or mutant thereof, such as a variant produced by mutagenesis that has one or more properties, such as thermal stability, that differ from the naturally-occurring protein. Non-limiting examples of naturally occurring luciferases include, luciferases found among marine arthropods, firefly luciferase, click beetle luciferase, and railroad worm luciferase. A non-limiting example of a luciferase photoprotein is the aequorin photoprotein. Luciferase detetion methods are well known in the art. See, for instance, U.S. application Ser. No. 10/665,314, filed Sep. 19, 2003 (P450 activity); U.S. patent application Ser. No. 09/813,279, filed Mar. 19, 2001 (kinase activity); and U.S. provisional application no. 60,353,158, filed Feb. 1, 2002 (protease activity), commonly owned by Promega Corporation.

In another aspect of the disclosure, tags comprising fluorescence tags and splitfluorescent tags refer to epitope tags consisting of short amino acid sequences. For example, epitope tags are recognized by well-characterized antibodies or by luciferase. Suitable tags for antibodies and fragments thereof include but are not limited to Myc tag, Flag-peptide tag, His Tag, Strep-Tag, GST-Tag, MBP- Tag, SNAP-Tag, Halo-Tag, HiBit-Tag, Tap-Tag, INPACT-CN, pk or V5 tag, HA-tag and NE-tag. The cognate interaction members for each of these tags are known and can be used for detection of the tagged at least one member of the E3 ligase complex.

In one aspect of the method of the present disclosure, the at least one member of the E3 ligase complex may be determined in an immunoassay. Suitable immunoassays may be selected from the group of immunoprecipitation, enzyme immunoassay (EIA)), enzyme-linked immunosorbenassays (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or Luminex ^{®} Assays.

In one aspect of the present invention, the at least one member of the E3 ligase complex is detected in a Western Blot. Western blotting involves application of a protein sample (lysate) onto a polyacrylamide gel, subsequent separation of said complex mixture by electrophoresis, and transferal or "electro-blotting" of separated proteins onto a second matrix, generally a nitrocellulose or polyvinylidene fluoride (PVDF) membrane. Following the transfer, the membrane is "blocked" to prevent nonspecific binding of antibodies to the membrane surface. Many antibody labeling or tagging strategies are known to those skilled in the art. In the simplest protocols, the transferred proteins are incubated or complexed with a primary enzyme-labeled antibody that serves as a probe. After blocking non-specific binding sites a suitable substrate is added to complex with the enzyme, and together they react to form chromogenic, chemiluminescent, or fluorogenic detectable products that allow for visual, chemiluminescence, or fluorescence detection, respectively. This procedure is described by Gordon et al., U.S. Patent 4,452,901 issued June 15, 1984.

In a preferred aspect of the method of the present invention, the level of at least one member of the E3 ligase complex may be detected in a FRET (Förster Resonance Energy Transfer) analysis. The theory of FRET (Förster Resonance Energy Transfer) defines a distance dependent, non-radiative transfer of energy from an excited donor (D) to an acceptor molecule (A). The relationship between easily accessible spectroscopic data and theoretical equations was the achievement of Theodor Förster, thereby enabling the possibility of many FRET applications in all kinds of natural sciences. FRET has been used in biochemical applications within the 1 to 10 nm scale (K. E. Sapsford et al., Angew. Chem. Int. Ed., 45, 4562, 2006) (e.g. protein-protein binding, protein folding, molecular interactions at and in cell membranes, DNA hybridization and sequencing, immunoreactions of antigens and antibodies). Details of the theory of FRET are well known.

In one aspect of the present invention, the level of at least one member of the E3 ligase complex may be detected in a protein complementation assay (PCA). Protein complementation assays (PCA) provide a means to detect the interaction of two biomolecules, e.g., polypeptides. PCA utilizes two fragments of the same protein, e.g., enzyme, that when brought into close proximity with each other can reconstitute into a functional, active protein. In some aspects, the NANOBIT^{®} technology (Promega Corporation) may be used to detect molecular proximity by virtue of the reconstitution of a luminescent enzyme via the binding interaction of enzyme components or subunits. By design, the NanoBiT subunits (i.e., 1.3 kDa peptide, 18 kDa polypeptide) weakly associate so that their assembly into a luminescent complex is dictated by the interaction characteristics of the target proteins, such as the at least one member of the E3 ligase complex used herein, onto which they are appended. Details are described, *inter alia,* in Dixon et al., "NanoLuc Complementation Reporter Optimized for Accurate Measurement of Protein Interactions in Cells," ACS Chem. Biol., Publication Date (Web): November 16, 2015. In some aspects, the Nano-Glo^{®} HiBiT Detection System (Promega Corporation) may be used to quantify HiBiT-tagged proteins in cell lysates using a add-mix-read assay protocol. For example, HiBiT-tagged proteins, such as ligase substrate receptors, e.g. DCAF15, may be ectopically expressed. HiBit-DCAF15 fusion protein may be ectopically expressed via a viral vector. As another example, a HiBit tag may be knocked in at the endogenous DCAF15 locus. HiBiT is an 11-amino-acid peptide tag that is fused to the N or C terminus of the protein of interest or inserted into an accessible location within the protein structure. The amount of a HiBiT-tagged protein expressed in a cell may be determined by adding a lytic detection reagent containing the substrate furimazine and Large BiT (LgBiT), the large subunit used in NanoLuc^{®} Binary Technology (NanoBiT^{®}; 1). Alternatively, when the LgBit may be ectopically introduced, such as by but not limited to lentiviral expression, the HiBit level may be measured in living cells by adding luciferase substrate(s).

Unlike Small BiT (SmBiT, 11 a.a.), which binds to LgBiT with low affinity (KD = 190µM), HiBiT binds tightly to LgBiT (KD = 0.7nM), promoting complex formation in the cell lysate to generate a bright, luminescent enzyme. Details are described, *inter alia,* in Sasaki et al., "Development of a rapid and quantitative method for the analysis of viral entry and release using a NanoLuc luciferase complementation assay" Virus Res. Publication Date (Web): October 23, 2017.

As used herein, the at least one member of the E3 ligase complex produced by the recombinant expression systems in the manipulated eukaryotic cell as described above may be recovered for the determination of its level, for example the expression level. An illustrated example of such an member of the E3 ligase complex is an E3 ligase substrat receptor, like CRBN or DCAF-15 Thus, the inventive method may also comprises culturing the eukarotic cell (i.e. a host cell) which is transformed with an expression vector comprising a DNA sequence that encodes the at least one member of the ligase complex under conditions sufficient to promote expression of the protein. Such a cell may also be used as an eukaryotic cell that comprises enhanced CRL activity in accordance with this invention. Furthermore, if desired, for example to elucidate the level of at least one member of an E3 ligase complex, said at least one member of the ligase complex may be recoverd (for example from) cell extracts, depending upon the expression system employed. As is known to the skilled person in the art, procedures for purifying a recombinant protein will vary according to such factors as the type of host cells employed and whether or not the recombinant protein is secreted into the culture medium. When expression systems that secrete the recombinant at least one member of the ligase complex are employed, the culture medium first may be concentrated. Following the concentration step, the concentrate can be applied to a purification matrix such as a gel filtration medium. Alternatively, an anion exchange resin can be employed, e.g., a matrix or substrate having pendant diethylaminoethyl (DEAE) groups. The matrices can be acrylamide, agarose, dextran, cellulose, or other types commonly employed in protein purification. Also, a cation exchange step can be employed. Suitable cation exchangers include various insoluble matrices comprising sulfopropyl or carboxymethyl groups. Further, one or more reversed-phase high performance liquid chromatography (RP-HPLC) steps employing hydrophobic RP-HPLC media (e.g., silica gel having pendant methyl or other aliphatic groups), ion exchange-HPLC (e.g., silica gel having pendant DEAE or sulfopropyl (SP) groups), or hydrophobic interaction-HPLC (e.g., silica gel having pendant phenyl, butyl, or other hydrophobic groups) can be employed to further purify the protein. Some or all of the foregoing purification steps, in various combinations, are well known in the art and can be employed to provide an isolated and purified recombinant protein. Recombinant protein produced in bacterial culture is usually isolated by initial disruption of the host cells, centrifugation, extraction from cell pellets if an insoluble polypeptide, or from the supernatant fluid if a soluble polypeptide, followed by one or more concentration, salting- out, ion exchange, affinity purification, or size exclusion chromatography steps. Finally, RP- HPLC can be employed for final purification steps. Microbial cells can be disrupted by any convenient method, including freeze-thaw cycling, sonication, mechanical disruption, or use of cell lysing agents.

As used herein, the term "nucleic acid" may refer to either DNA or RNA, or molecules which contain both deoxy- and ribonucleotides. The nucleic acids include genomic DNA, cDNA and oligonucleotides including sense and anti-sense nucleic acids. Such nucleic acids may also contain modifications in the ribose-phosphate backbone to increase stability and half life of such molecules in physiological environments. The nucleic acid may be double stranded, single stranded, or contain portions of both double stranded or single stranded sequence. As will be appreciated by those in the art, the depiction of a single strand ("Watson") also defines the sequence of the other strand ("Crick"); thus the sequences depicted in figures also include the complement of the sequence. By the term "recombinant nucleic acid" herein is meant nucleic acid, originally formed in vitro, in general, by the manipulation of nucleic acid by endonucleases, in a form not normally found in nature. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e. using the in vivo cellular machinery of the host cell rather than in vitro manipulations; however, such nucleic acids, once produced recombinantly, although subsequently replicated non-recombinantly, are still considered recombinant for the purposes of the invention.

As used herein the terms "polypeptide" and "protein" as used interchangeably herein, refer to a polymer of amino acids of three or more amino acids in a serial array, linked through peptide bonds. The term "polypeptide" includes proteins, protein fragments, protein analogues, oligopeptides and the like. The term polypeptide as used herein can also refer to a peptide. The amino acids making up the polypeptide may be naturally derived, or may be synthetic. The polypeptide can be purified from a biological sample.

The term "method for identifying a chemical compound or agent" as used in accordance with the present invention also refers to a procedure in which a property of a chemical compound or agent is determined by determining the level of the at least one member of the E3 ligase complex. Specifically, "identifying a chemical compound or agent" may be understood as interchangeable with "detection of a compound" or "finding a compound". The term "identifying" herein may be understood as a relative term as evidently to a person skilled in the art. Therefore, a certain threshold may be applied at which the level of the at least one member of the E3 ligase complex is increased in the presence of the chemical compound or agent compared to the level determined in the absence of the chemical compound in order to consider a chemical compound or agent as "identified". It will be understood that the threshold may considerably depend on the experimental setup and may be adjusted depending on the purpose of the method or the intended use of the identified chemical compound or agent.

The chemical compound and agent identified/tested with the inventive method may comprise but is not limited to small molecules, metabolites, molecular glues, mixture of compounds such as natural extracts or cell or tissue culture products. Also biologicals and/or biological material such as peptides, proteins, fusion proteins antibodies, monobodies, nanobodies , DNA, RNA, antisense oligonucleotides, RNAi, aptamer, RNAzymes and DNAzymes, or glucose and lipids are envisaged to be tested/assessed/identified with the methods of the present invention. As illustrated in the appended examples, chemical compound and agent identified/tested and in particular verified as being able to to induce ubiquitination of a protein of interest in context of the methods of the present invention comprise SNS-THAL-032, CC-885 or dBEt6. "SNS-THAL-032" or "THAL-SNS-032" refers to a cereblon-dependent degrader of CDK9 comprising a CDK-binding SNS-032 ligand linked to a thalidomide derivative that binds the E3 ubiquitin ligase Cereblon (CRBN). "CC-885" refers to a modulator of the E3 ligase protein cereblon with antiproliferative effects on human myeloid leukemia cell lines. It forms a complex with cereblon and the cell cycle regulator and translation termination factor GSPT1. The antitumor activity of CC-885 relies on cereblon-dependent ubiquitination and degradation of the GSPT1. Furthermore, "dBET6" refers to a cereblon-dependent degrader of BET bromodomain proteins, in particular of BRD4; see also Winter (2017), Mol Cell 67, 5-18.

As used herein, the term "small molecule" (as a chemical compound or agent to be identified and or tested in accordance with the present invention) refers to a nonpeptidic, non-oligomeric organic compound either synthesized in the laboratory or found in nature. The term "molecular weight", as used herein, shall refer to number average molecular weight expressed in Dalton, unless otherwise specified. Small molecules, as used herein, can refer to compounds that are "natural product-like", however, the term "small molecule" is not limited to "natural product-like" compounds. Rather, a small molecule is typically characterized in that it possesses one or more of the following characteristics including having several carbon-carbon bonds, having multiple stereocenters, having multiple functional groups, having at least two different types of functional groups, and having a molecular weight of less than 1500, although this characterization is not intended to be limiting for the purposes of the present invention. Small molecules to be assessed/tested with the inventive methods may also comprise, e.g., molecules that change posttranslational modifications of a target protein, such as phosphorylation status of a target protein, thereby making a target protein accessible to an E3 ligase. For example, a small molecule may induce degradation of a target protein by inhibition of an enzyme implicated in the posttranslational modification of a target protein, such as a tyrosine kinase phosphorylating a target protein. Alternatively, a small molecule may change posttranslational modification of a protein, thereby inducing degradation of a posttranslationally modified protein without associating with a target protein and/or E3 ligase.

As used herein, the term "metabolite" refers to a substance which can be converted invivo into a biologically active agent by such reactions as hydrolysis, esterification, desterification, activation, salt formation and the like.

As discussed herein, the "chemical compound or agent" to be tested in accordance with the inventive methods may also comprise biological and/or biological molecules, like peptides, proteins, including fusion proteins and/or complex biological molecules, like antibodies and antibody derivatives, like monobodies, scFvs, nanobodies that are renders cell-permeable. The term "fusion protein" has its generally accepted meaning in the art. In accordance with the present invention, it particularly refers to a protein having at least two linked heterologous polypeptide portions. The term "heterologous" used in the context of the present invention indicates that said polypeptide portions are not found in the same relationship to each other in nature. The fusion protein is typically recombinantly produced, i.e. coding regions from different sources are ligated and the ligation product is expressed as the fusion protein. Methods for producing fusion proteins are generally known in the art.

The term "molecular glue" is generally known in the art and refers to a chemical compound or agent that can bind at least two different molecules at a time by cooperative binding but has no binding affinity to one of the at least two different molecules separately. For example, the term "molecular glue" may refer to a multimolecular structure comprising at least two specific recognition elements capable of specifically attaching two surfaces. Preferably, a molecular glue refers to a chemical compound or agent that binds to a protein of interest if the chemical compound or agent simultaneously binds to the protein of interest and a second protein. As used in context of the method of the present invention, , a molecular glue refers to a chemical compound or agent that binds to a protein of interest if the chemical compound or agent simultaneously binds to the protein of interest and the member of the E3 ligase complex. The term "binding affinity" refers to the strength of the interaction of a binding molecule with its respective substrate. Examples for molecular glues include but are not limited to non-chimeric small molecules, lenalidomide, pomalidomide, CC-885 and related immunomodulatory drugs (IMiDs). In particular, even without a specific targeting moiety, MG may induce cooperative binding of E3 ligases CRBN to previous undruggable proteins like IKZF1 /3, thereby inducing their degradation. Hence, MG may induce highly cooperative associations with target proteins (POIs) that are naturally not bound by CRBN (Figure 4). MG, such as lenalidomide may be used in cancer therapy. Such MGs are also capable of such an association of target proteins/POIs and the substrate receptor such as CRBN.

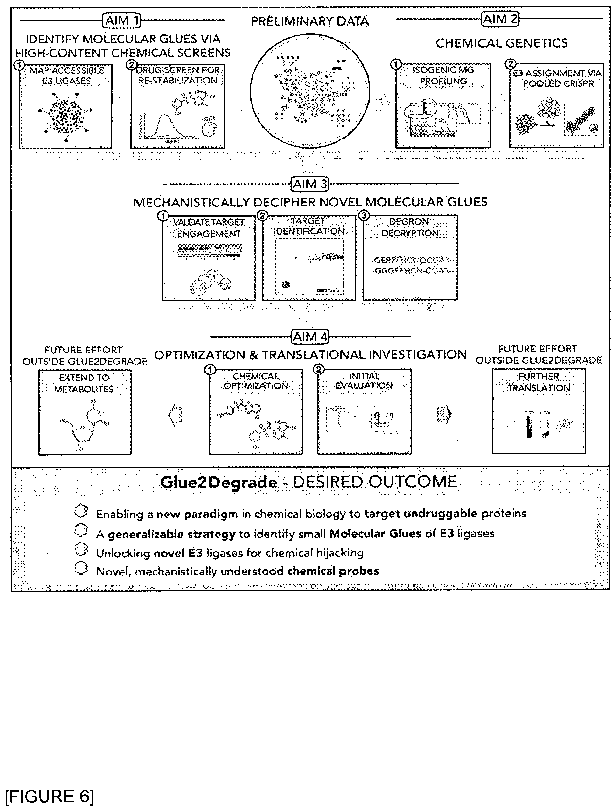

Identification of molecular glues and CRLs that can be hijacked by the molecular glue are outlined in Figure 6 and employ two parallel strategies. Strategy 1 explores the concept of chemical compound or agent-induced stabilization of the at least one member if the E3 ligase complex via screening of compound libraries against CRLs. The orthogonal strategy 2 is independent of chemical compound or agent-induced stabilization of the at least one member of the E3 ligase complex and investigates differential compound efficacy in neddylation-impaired cellular models coupled to a functional-genomics based target deconvolution strategy.

The term "cooperative binding" refers to the binding of at least two different molecules to different binding sites of a chemical compound or agent, wherein the chemical compound or agent only binds the at least two different molecules of binding of the at least two different molecules to the chemical compound or agent occurs simultaneously. In a preferred aspect of the present invention, one of the at least two different molecules is the protein of interest and one of the two different binding sites of the chemical compound or agent is a binding site for the protein of interest.

In one aspect of the present invention, the compounds may comprise derivatives comprising (aryl) sulphonamides and immunomodulatory drugs IMiDs. In another aspect of the present invention, the chemical compound or agent comprises a peptide, a small molecule, or a molecule having a size between 0.8 and 1.5 kDa. In a preferred aspect of the present invention, the chemical compound or agent is a peptide, a small molecule, or a molecule having a size between 0.8 and 1.5 kDa.

As used herein, the term "peptide" refers to two or more amino acids joined to each other by peptide bonds or modified peptide bonds. The term "amino acid" refers to the basic chemical structural unit of a protein or polypeptide.

In one aspect of the method of the present invention, the small molecule or molecule having a size between 0.8 and 1.5 kDa comprises a first moiety binding the protein of interest and a second binding moiety, wherein the first and the second binding moiety are optionally connected by a linker. The term "linker" refers to any chemically and biologically compatible covalent grouping of atoms which can serve to link together the first and second reagents of this invention. Generally, preferred linkers have from 20 to 40 bonds from end to end, preferably 25 to 30 bonds, and may be branched or straight chain or contain rings. The bonds may be carbon-carbon or carbon-heteroatom or heteroatom-heteroatom bonds. The linkage can be designed to be hydrophobic or hydrophilic. The linking group can contain single and/or double bonds, 0-10 heteroatoms (O, S preferred), and saturated or aromatic rings. The linking group may contain groupings such as ester, ether, sulfide, disulfide and the like. For example, such a small molecule or molecule having a size between 0.5 and 2 kDa, preferably between 0.8 and 1.5 kDa include Proteolysis Targeting Chimeras (PROTAC^{®}s) or heterobifunctional compounds composed of a target protein-binding ligand and an E3 ubiquitin ligase ligand. The term "binding moiety" as used herein refers to any molecule capable of forming a binding complex with another molecule. PROTAC^{®}s or heterobifunctional compounds as used herein refer to compounds that induce proteasome-mediated degradation of selected proteins via their recruitment to E3 ubiquitin ligase and subsequent ubiquitination (Crews C, Chemistry & Biology, 2010, 17(6):551 -555; Schnnekloth JS Jr., Chembiochem, 2005, 6(I):40-46). Thus, the term "PROTAC^{®}" or "heterobifunctional compound" are used interchangeably and refer to proteolysis-targeting chimera molecules having generally three components, an E3 ubiquitin ligase binding group, optionally a linker, and a protein binding group of a target. There is no particular limitation on the linkers that link binding moieties. In one aspect of the present invention, the linker may be a peptide linker. The amino acid sequence of the peptide linker can be suitably selected by those skilled in the art according to the objective. For example, the binding moieties are linked by a peptide bridge or by disulfide bonds. The binding moieties of the chemical compound or agent connected by a linker such as a peptide bridge can be produced by PCR methods known in the art. To link the binding moieties of the chemical compound or agent using the PCR method, a DNA encoding the entire or desired partial amino acid sequence of the DNAs comprising DNA sequence encoding the binding moieties of the chemical compound or agent is used as template. *Each DNA encoding the* binding moieties of the chemical compound or *agent* is amplified by the PCR method using pairs of primers with sequences corresponding to the sequences at both ends of the DNA to be amplified. Next, a DNA encoding the peptide linker portion is prepared. The DNA encoding the peptide linker can also be synthesized by PCR. Nucleotide sequences that can link the amplification products of each separately synthesized binding moieties of the chemical compound or agent region are added to the 5'side of the primers used. Next, a PCR reaction is carried out using the DNA of the binding moieties of the chemical compound or agent and the DNA of the linker together with the primers for the assembly PCR. The primers for the assembly PCR consist of a combination of a primer that anneals to the 5"side of the DNA of one binding moiety of the chemical compound or agent and a primer that anneals to the 3'side of the other binding moiety of the chemical compound or agent. Therefore, the primers for the assembly PCR consist of a primer set that can amplify the DNA encoding the entire sequence of the chemical compound or agent to be synthesized. As a result, these DNAs are linked together and the full-length chemical compound or agent is finally produced as an amplification product of the primers used for the assembly PCR.

In one aspect of the present invention, the chemical compound or agent is contacted with the eukaryotic cell manipulated to comprise enhanced cullin-RING ubiquitin ligase (CRL) activity.

The term "contacting" may be understood in the widest sense as any means that allow an interaction of the chemical compound or agent with the cell. Preferably, this is achieved by mixing the chemical compound or agent with the cell.

In certain aspects, the method of the present invention is particularly useful for screening for a chemical compound or agent able to induce ubiquitination of a protein of interest. Screening refers to a method in which a standardized molecular assay or a composition of several molecular assays is applied to a plurality of compounds to determine their properties of interest such as the binding to molecular targets and, as used herein, is a repetitive, or iterative, process, in which a chemical compound or agent is tested for the ability to induce ubiquitination of a protein of interest.

In particularly preferred aspects, the invention can be used for high throughput screenings of chemical compounds or agents for the ability to induce ubiquitination of a protein of interest. The term "high-throughput" relates to an assay system for the rapid testing of a plurality of compounds within in a short time, thus, the assaying time per tested compound is minimized. The assay is preferably carried out in multiwell plates, more preferably in 6- well plates, 12-well plates, 24-well plates, 96-well plates or 384-well plates, or 1536 plates, even more preferably in 96-well plates, 384-well or 1536 plates plates. Thus, the term "high-throughput" encompasses screening activity in which human intervention is minimized, and automation is maximized. For example, high-throughput screening involves automated pipetting, mixing, and heating. Alternatively, a high-throughput method is one in which for example hundreds of compounds can be screened per 24 hour period by a single individual operating a single suitable apparatus. "Performed automatically" refers to a method that is fully or partly controlled and/or carried out by one or more technical devices, preferably pipetting robots. Methods for conducting a variety of different HTS assays, in particular HTS screening assays are known in the art. The skilled person knows how to conduct HTS methods by the methods used in the context of the present invention.

In certain aspects of the method of the present disclosure, the chemical compound or agent is part of a library of compounds or agents. The term "library" as used herein refers to a collection of chemical compounds and agents. The library of the present invention comprises at least two compounds or agents. Thus, in one aspect of the method of the present disclosure, the ability to induce ubiquitination of the protein of interest is determined for at least two compounds or agents comprised in the library.

As discussed above, in a preferred aspect of the methods of the present disclosure, the method may be used to screen a library of compounds. In this context, the library of compounds may be composed of a plurality of chemical compounds and agents that may be assembled of multiple sources. A library may include chemical compounds and agents comprising chemically synthesized substances, products of biotechnological processes, naturally occurring substances or products resulting from combinatorial chemistry techniques or combinations thereof. In the context of the present disclosure, the library preferably comprises a peptide, a small molecule, or a molecule having a size between molecule having a size between 0.5 and 2 kDa, preferably between 0.8 and 1.5 kDa, more preferably chemically synthesized peptide, small molecule, or molecule having a size between 0.5 and 2 kDa, preferably between 0.8 and 1.5 kDa. Such moleculs may also comprise PROTAC^{®}s In one aspect of the present disclosure, the compounds or agents of the library comprise a first moiety binding to the protein of interest, a second binding moiety and a linker connecting the first and the second binding moiety; and wherein at least one of the compounds or agents comprises the second binding moiety of a second compound or agent of the library and/or a linker from the second or a third compound or agent of the library. For example, the compound may comprise a first binding moiety binding to the protein of interest and the second binding moiety has been exchanged with the second binding moiety of a second compound having a higher binding affinity and/or higher binding specificity to the at least one member of the E3 ligase complex. Alternatively, the compound may comprise a first binding moiety binding to the protein of interest, a second binding moiety and a linker, wherein the linker has been exchanged with the linker of a second compound and wherein the linker of the second compound has been shown to induce a higher binding affinity and/or higher binding specificity of the first binding moiety to the protein of interest and/or a higher binding affinity and/or higher binding specificity of the second binding moiety to the at least one member of the E3 ligase complex. Alternatively, the compound may comprise a first binding moiety binding to the protein of interest, a second binding moiety that has been exchanged with the second binding moiety of a second compound having a higher binding affinity and/or higher binding specificity to the at least one member of the E3 ligase complex and a linker that has been exchanged with the linker of a third compound, wherein the linker of the third compound has been shown to induce a higher binding affinity and/or higher binding specificity of the first binding moiety to the protein of interest and/or a higher binding affinity and/or higher binding specificity of the second binding moiety to the at least one member of the E3 ligase complex.

In one aspect, described herein but not part of the invention is a compound or agent identified by the method of the present invention. As used herein, the chemical compound or agent comprises any chemical compound or agent as described above and may be identified by means and methods of the present invention

In one aspect, described herein but not part of the invention is a compound or agent for use in medicine and/or as a pharmaceutical. As discussed herein, the disease to be treated with the compound(s) identified and/or obtained by the inventive methods comprise disorders which are characterized by an overactivity and/or overexpression of a POI. Said POI can be degraded with the compound(s) identified by the inventive methods since these compounds are able to induce ubiquitination, either directly or indirectly. Preferred diseases to be treated with the compounds identified with the methods of the present invention are cancer, neurological disorders/disease and or metabolic diseases. The term "medicine" as used herein is intended to be a generic term inclusive of prescription and non-prescription medications. The compound or agent for use in medicine should be understood as being useful in maintaining health or promoting recovery from a disease, preferably cancer. Further, the term "medicine" includes medicine in any form, including, without limitation, e.g., pills, salves, creams, powders, ointments, capsules, injectable medications, drops, vitamins and suppositories. In one aspect not part of the present invention, the chemical compound or agent is for use in the treatment of cancer.

As used herein, and unless otherwise specified, the terms "treat," "treating" and "treatment" refer to an action that occurs while a patient is suffering from the specified disease, disorder or condition. As used herein, the terms "treat," "treatment" and "treating" refer to the reduction or amelioration of the progression, severity, and/or duration of a disease, disorder or condition resulting from the administration of one or more therapies. As discussed above, the preferred diseases to be treated in accordance with this disclosure, are cancer, metabolic diseases, an infectious disease and/or neurological disorders. Non-limiting, illustrative examples of corresponding POIs (for example in cancer cells or in cells that are involved in metabolic or neurological disorders or in infectious dieases) to be targeted by the compounds identified with the methods of the present invention are also provide herein and have been discussed herein above.

The term "cancer" as used herein refers to any malignant tumor in the aforementioned tissue and cell type. Examples of the cancer may include a cancer which can be caused by an abnormal adherent cell, or a cancer which can be caused by an abnormal blood cell (e.g., leukemia, lymphoma, multiple myeloma). Specifically, examples of the cancer which can be caused by the abnormal adherent cell may include a lung cancer (e.g. squamous cell carcinoma, non-small cell carcinoma such as adenocarcinoma and large cell carcinoma, and small cell carcinoma), a gastrointestinal cancer (e.g., stomach cancer, small intestine cancer, large intestine cancer, rectal cancer), a pancreatic cancer, a renal cancer, a hepatic cancer, a thymic cancer, a spleen cancer, a thyroid cancer, an adrenal cancer, a prostate cancer, an urinary bladder cancer, an ovarian cancer, an uterus cancer (e.g., endometrial carcinoma, cervical cancer), a bone cancer, a skin cancer, a brain tumor, a sarcoma, a melanoma, a blastoma (e.g., neuroblastoma), an adenocarcinoma, a planocellular cancer, a solid cancer, an epithelial cancer, and a mesothelioma. The "cancer" in cancer-related terms such as terms "cancer cell" and "cancer gene (oncogene)" can also mean the same meaning. The cancer cell can be derived from any mammalian species. Such a mammalian species may include, for example, humans, monkeys, cattle, swines, mice, rats, guinea pigs, hamsters, and rabbits. The mammalian species is preferably the human in terms of clinical application. Therefore, the cancer cell may be a cancer cell isolated from a patient with cancer or a cancer cell derived therefrom. The cancer cell may be a cell not infected with virus or a cell infected with virus. Examples of a carcinogenic virus capable of infecting the cell may include Epstein Barr virus, hepatitis virus, human papilloma virus, human T cell leukemia virus, and Kaposi sarcoma-associated herpes virus. The cancer cell may also be a cancer cell derived from an embryonic stem cell, a somatic stem cell, or an artificial stem cell (e.g., iPS cell) produced from a normal cell. The cancer cell from which the artificial cell of the present invention is derived can express an inherent oncogene. As used herein, the term "inherent oncogene" means an oncogene responsible for proliferation of the cancer cell, which is expressed by the cancer cell that can be used as a material in the establishment of the artificial cell of the present invention. The oncogene can be a gene that is overexpressed in the cancer cell (e.g., overexpression due to increase of copy number of the gene) and transmits a signal for proliferation excessively, or a gene that a mutation occurs which continuously transmit a proliferation signal in the cancer cell. Examples of the mutation may include point mutation (e.g., substitution), deletion, addition, insertion, and mutation causing a fusion (e.g., inversion, translocation). As used herein, the term "gene" may intend to be a mutated gene. Examples of the inherent oncogene may include genes for kinase such as tyrosine kinase (receptor type, and non-receptor type) and serine/threonine kinase, small G-proteins, and transcription factors. Examples of the tyrosine kinase which can play a role in proliferation of the cancer cell may include molecules belonging to an epidermal growth factor receptor (EGFR) family (e.g., EGFR, HER2, HER3, HER4), molecules belonging to platelet derived growth factor receptor (PDGFR) family (e.g., PDGFRα, PDGFRβ), an anaplastic lymphoma kinase (ALK), a hepatocyte growth factor receptor (c-MET), and a stem cell factor receptor (c-KIT).

Accordingly, in one aspect, described herein but not part of the invention is a method treating a disorder comprising administering the chemical compound or agent as identified by the methods of the present invention to a patient in need of such treatment. The disorder to be treated may be selected from the group consisting of cancer, metabolic disorders/disases, infectious diseases and neurological disorders/diseases.

A "patient" or "individual" or "subject" is a mammal. Mammals include, but are not limited to, domesticated animals (*e.g*., cows, sheep, cats, dogs, and horses), primates (*e.g*., humans and non-human primates such as monkeys), rabbits, and rodents (*e.g*., mice and rats). In certain aspects, the patient, individual, or subject is a human. In some aspects, the patient may be a "cancer patient," *i.e*. one who is suffering or at risk for suffering from one or more symptoms of the disease, like cancer metabolic disorders/disases and neurological disorders/diseases.

It will be understood that the specific dose level for any particular patient will depend upon a variety of factors including the activity of the specific compound employed, the age, body weight, general health, sex, diet, time of administration, route of administration, rate of excretion, drug combination and the causative mechanism and severity of the particular disease undergoing therapy.

As detailed herein, in a further aspect but not part of the invention the present disclosure also relates to a eukaryotic cell manipulated to comprise an enhanced CRL activity. The cell comprises cells as described above and the cell is obtained by methods as described above. In one aspect the eukaryotic cell has been manipulated to comprise enhanced CRL activity, for example by inactivation of CAND1, CAND2 or CAND1 and CAND2. In another aspect not part of the present invention, the eukaryotic cell has been manipulated to comprise enhanced CRL activity by overexpressing proteins able to induce neddylation of the CRLs in the cell such as NAE1 or UBE2M. As further examples, substrate receptors or core components of CRL ligases including adaptors (such as DDB1 or SKP1) or cullin backbones (such as mammalian CUL1, CUL2, CUL3, CUL4A, CUL4B, CUL5 and CUL7) may be overexpressed in the eukaryortic cells. In one aspect of the present disclosure, the eukaryotic cell has been manipulated to comprise constantly neddylated and/or hyper-neddylated CRLs. Preferably, the cell has been further manipulated to comprise constantly neddylated CRLs by a hypomorphic mutation or inactivation of at least one member of the COP9 signalosome (CSN). More preferably, the at least one member is the COPS8 or COPS7B subunit. In another aspect not part of the present invention, the eukaryotic cell manipulated to comprise constantly neddylated CRLs is manipulated by contacting the cell with CSN5i-3.

In one aspect of the present inventive method, the eukaryotic cell manipulated to comprise an enhanced CRL activity and/or to be employed in the inventive method may expresses an oncogene or may be a cancer cell such as a lung cancer cell, a gastric cancer cell, a melanoma cell, a sarcoma cell, a leukemia cancer cell, a colon cancer cell or a neuroblastoma cell.

The term "cancer cell" as used herein means a tumor cell having an ability to proliferate depending on a particular oncogene expressed in the cancer cell. The cancer cell may include a primary cultured cell, a cell line, or a cancer stem cell. As used herein, the "dependence (depending)" concerning the proliferation of the cell refers to the state of the oncogene addiction or the addiction, where the cell proliferates depending on the particular oncogene. Whether or not the cell proliferates depending on the particular oncogene can be confirmed by treating the cell with an inhibitor of the particular oncogene and then evaluating a proliferation ability of the treated cell.The proliferation ability can be evaluated by, for example, an MTT assay or an MTS assay. It is known that cell death due to apoptosis can be induced, when the cell in the oncogene addiction for the particular oncogene is treated with the inhibitor of such an oncogene. Therefore, the oncogene addiction in the cell for the particular oncogene may be confirmed by evaluating whether or not the apoptosis can be induced by inhibition of the oncogene. The induction of the apoptosis can be evaluated by, for example, a TUNEL assay, detection of active caspase, or detection of annexin V. The cancer cell can be derived from any tissues. Examples of such a tissue may include respiratory tissues (e.g., lung, trachea, bronchi, pharynx, nasal cavity, paranasal cavity), gastrointestinal tissues (e.g., stomach, small intestine, large intestine, rectum), pancreas, kidney, liver, thymus, spleen, heart, thyroid, adrenal, prostate, ovary, uterus, brain, skin, and a blood tissue (e.g., bone marrow, peripheral blood). In another viewpoint, the cancer cell can be an adherent cell or a non-adherent cell (i.e., a blood cell). In still another viewpoint, the cancer cell can be a cell present in the above tissues or tissues other than the above tissues. Examples of such a cell may include a gland cell (e.g., gland cell (adenocyte) in lung, mammary gland cell), an epithelial cell, an endothelial cell, an epidermal cell, an interstitial cell, a fibroblast, an adipocyte, a pancreatic P cell, a nerve cell, a glia cell, and a blood cell.

In accordance with this invention, also other cells may be manipulated in accordance with the present invention, i.e. by enhanced cullin-RING ubiquitin ligase (CRL) activity. Such cells to be employed in the methods of the invention or as provided as inventive cells are characterized by neddylation of members of the cullin-RING ubiquitin ligase complex. Accordingly, and preferably, the inventive eukaryotic cells are manipulated to comprise constantly neddylated and/or hyper-neddylated cullin-RING ubiquitin ligase/ constantly neddylated and/or hyper-neddylated CRLs. Corresponding means and methods how to obtain such inventive eukaryotic cells are disclosed herein above and are illustrated in the appended Examples.

It is to be understood in accordance with the present invention that both the foregoing general description and the following description are exemplary and explanatory only and are not intended to limit the scope of the current teachings. The singular form "a", "an" and "the" include plural referents unless the context clearly dictates otherwise. Thus, for example, reference to "a polypeptide" includes a plurality of polypeptides. The terms "comprising," "having," "including," and "containing" of the present invention are to be construed as open-ended terms (i.e., meaning "including, but not limited to") unless otherwise noted. Recitation of ranges of values herein are merely intended to serve as a shorthand method of referring individually to each separate value falling within the range, and includes the endpoint boundaries defining the range, unless otherwise indicated herein, and each separate value is incorporated into the specification as if it were individually recited herein.

In general, the terms and phrases used in accordance with the present invention are to be construed with ordinary and typical meaning to those of ordinary skill in the art, and can be found by reference to standard texts, journal references and contexts known to those skilled in the art. The definitions provided herein above are provided to clarify their specific use in the context of the present invention.

### EXAMPLES

The following Examples further illustrate the present invention but are not construed to limit the scope thereof.

### EXAMPLE 1

### Autodegradation of substrate receptor CRBN by the CRL

It was explored why CSN (COP9 signalosome, the deneddylation complex)- and CAND1 ( Cullin-associated NEDD8-dissociated protein 1; a substrate receptor exchange factor)-mutant backgrounds were resistant specifically to degraders based on CRL4^{CRBN} (=cullin RING ligase 4 using the substrate receptor cereblon) and CRL4^{DCAF15} (=cullin RING ligase 4 using the substrate receptor DDB1- and CUL4-associated factor 15) recruitment, but not to ARV-771, that binds CRL2^{VHL}. It was hypothesized that autodegradation (ubiquitination of the SR by the CRL in which it is assembled) may explain, at least in part, the resistance showed by the CSN mutants, since it would imply the inactivation of only part of the CRLs (Fig. 1A). Regarding CAND1, it was speculated that the exchange of SRs was impaired, and, therefore, the implicit longer time of the SR assembled into the CRL complex could also ended up on its autodegradation.

As a first approach, the levels of cereblon (CRBN), the SR of the CRL4^{CRBN} E3 ligase machinery, were checked by western blot (WB) in the mutant backgrounds. A strong decrease in CRBN levels in the COPS8 (COP9 signalosome complex subunit 8)-mutant cells was observed, and a less prominent downregulation in the CAND1-deficient cells (Fig. 1B). VHL levels, the SR of the CRL2^{VHL} E3 ligase, were not affected, as expected due to the sensitivity to ARV-771 of these mutant cells. Regrettably, DCAF15 (DDB1- and CUL4-associated factor 15) by WB could not properly be detected, but the resistance to indisulam in both genetic backgrounds showed that this SR is likely also undergoing autodegradation. To validate that autodegradation was causing the low levels of CRBN, WT and mutant cells were transduced with sgRNA targeting *CUL4A (Cullin 4A),* what greatly rescued the downregulation of CRBN (Fig. 1C). The CSN-impaired condition was used as a focus as this condition was sought to chemically mimic the genetic mutants. To this end, treatment with a COPS5 (COP9 signalosome complex subunit 5) inhibitor (CSN5i-3) was found to recapitulate CRBN downregulation (Fig. 1D). CRBN destabilization following CSN5i-3 treatment were rescued in CUL4A-deficient cells (Fig. 1E) or in cells lacking UBE2G1 (ubiquitin-conjugating enzyme E2 G1), a E2 ubiquitin-conjugating enzyme previously related to target degradation via the CRL4^{CRBN} ligase (Fig. 1F).

In order to survey in an unbiased manner the subset of SRs undergoing autodegradation, global expression proteomics was applied to the different genetic backgrounds: WT, COPS8-mutant and CAND1-deficient KBM7 cells. In addition, expression proteomics following cellular treatment with CSN5i-3 was employed (1µM, 8 h) (Fig. 1G-I). Downregulation of CRBN in the three conditions could be recapitulated and the subgroup of SRs destabilized could be depicted. In agreement with previous reports, CSN5i-3 only affected a subset of the SRs associated to each of the cullins.

In summary, it was shown that, upon genetic or chemical manipulation of the CSN, SRs were strongly destabilized via continuous autodegradation. Mechanistically, this was due to a failure to inactivate CRLs that have already accomplished the ubiquitination of their substrate. As a result, the associated E2 starts to ubiquitinate the SR (i.e. CRBN), leading to its proteasomal degradation. Moreover, the absence of CAND1 caused a failure in the dynamic exchange of SRs, what finally results in the autoubiquitination of the SR when there is not more substrate bound.

### Stabilization of the substrate receptor CRBN (CRBN) in CRL4 by dBET6, CC-885 and SNS-THAL-032

In order to mechanistically delineate the autodegradation phenomenon, the dynamics of SR re-stabilization upon substrate presentation were explored. It was postulated that upon substrate availability (i.e. via treatment with small-molecule degraders), autoubiquitination would be substantially abrogated as bound ligands would protect the SR from the E2-mediated ubiquitination (Fig. 2A).

To explore the re-stabilization of SR upon ligand treatment, WT cells were pretreated with different doses of CSN5i-3 for 2h to firstly induce SR autodegradation. As a proof of principle, the SR CRBN was used, and the autodegradation by WB was checked (Fig. 2B-D). Then, the cells were treated with three different small-molecule degraders that hijack CRL4^{CRBN} (cullin RING ligase 4 using the substrate receptor cereblon): dBET6, CC-885 and SNS-THAL-032 that induced the ternary complex formation with the target proteins BRD2/3/4 (bromodomain containing protein 2, bromodomain containing protein 3, bromodomain containing protein 4,, GSPT1 (Eukaryotic peptide chain release factor GTP-binding subunit ERF3A) and CDK9 (cyclin dependent kinase 9), respectively. In all the cases, a time-dependent stabilization of CRBN upon ligand treatment was observed (Fig. 2B-D).

### EXAMPLE 2

### Autodegradation and Compound-dependent stabilization of substrate receptor CRBN by the CRL using CRBN HiBit and a modified nano luciferase ("LgBit")

In a cell line of interest (like 293T), the SR of interest (CRBN/cereblon or DCAF15 DDB1- and CUL4-associated factor 15) is tagged with the HiBit peptide (Promega) that is 11 AA long and has picomolar affinity to a modified nano luciferase ("LgBit") that depends on HiBit complementation for its luciferase activity. Knock-in is validated. Afterwards, CRISPR/Cas9 is used to knock-out the CSN (COP9 signalosome) subunit COPS8 (COP9 signalosome complex subunit 8). Ensuing loss of CRBN HiBit is assayed via immunoblot and loss of bioluminescence. Cellular treatment with CRBN-binding PROTAC^{®}s (e.g dBET1 and dBET6) leads to a quick stabilization of CRBN HiBit levels via BET-protein recruitment to CRL4^{CRBN} HiBit complexes. After 4 hours of compound treatment, CRBNHiBit levels are assayed via bioluminescence by adding the detection reagent, which consists of a lysis buffer and recombinant LgBit protein. Within 10 minutes after addition of the detection reagent, luminescence is read with a standard plate reader, and is a proxy for drug-induced changes in CRBN levels.

### EXAMPLE 3

### Stabilization of CRBN in CRL4 by the molecular glue CC-885

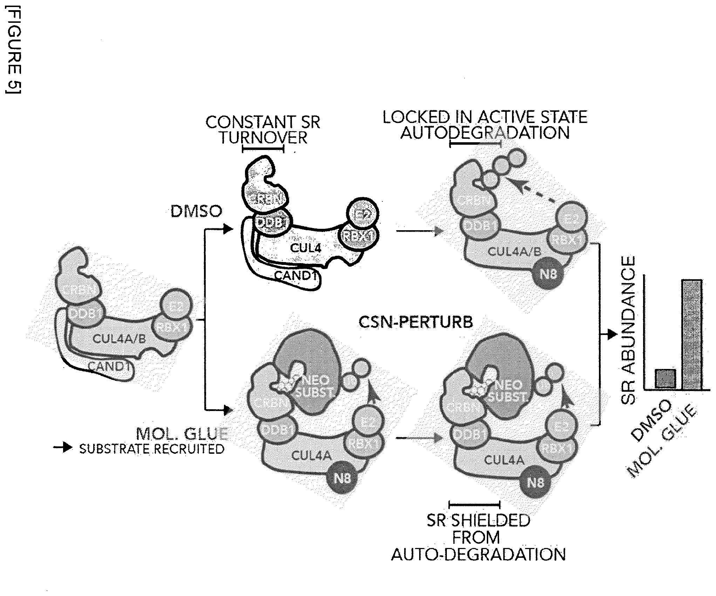

Cells were first pretreated with the small-molecule (CC-885) known to recruit a new substrate (GSPT1=Eukaryotic peptide chain release factor GTP-binding subunit ERF3A) to the substrate receptor CRBN of the CRL4^{CRBN} ligase. Pre-treatment was conducted for either 15 or 30 minutes. Afterwards, cells were treated with the CSN (COP9 signalosome) inhibitor (CNS5i-3) for 2 hours. It was observed that substrate recruitment protects from CRBN auto-degradation (or in general terms "substrate receptor auto-degradation"). This was shown in Figure 3 by comparing lane 2 of the CRBN blot (pre-treatment with DMSO/vehicle control) to lanes 4 (15 min pre-treatment) and lane 6 (30 min pre-treatment) of the same western blot. The same blot also documents that the present invention provides for means and methods how to determine the neddylation status of cullins constituting CRLs. As evident from figure 3, cells comprising a normal neddylation status or a stready-state neddylation status show two bands corresponding to a non-neddylated and a neddylated cullin, respectively. Average increase of neddylated to non-neddylated approximately 9 kDa. Constantly neddylated cells show preferentially/to a vast majority the culling band of the higher molecular weight (corresponding to the constant neddylation (i.e. hyperneddylation)) In sum, it was shown that substrate receptor CRBN was degraded when not bound to a chemical compound or agent such as CC-885, but was stabilized upon contacting the cell with the chemical compound or agent bound to the POI (GSPT1) as represented by Figure 5.

### EXAMPLE 4

### Phenotypic molecular glue identification in cellular models of impaired neddylation dynamics

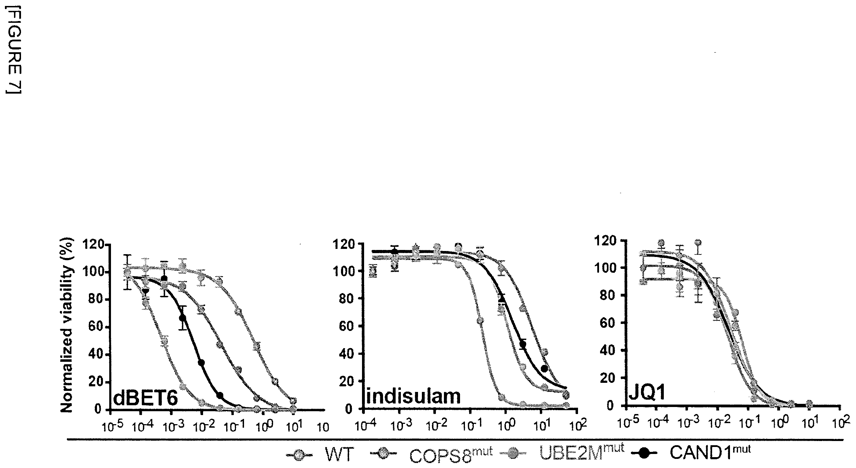

KBM7 cells mutated for five core genes were isolated and the underlying CRISPR-induced mutations were truncations leading to full knockouts (CAND1) or hypomorphic alleles (UBE2M, COPS8, COPS7B, GPS1). These mutations did not impact cellular fitness. However, these mutations caused resistance to tested molecular glues (indisulam; reprogramming the substrate receptor DCAF15 to degrade RBM39) or PROTAC^{®}s (dBET6, reprogramming the substrate receptor cereblon/CRBN to degrade bromodomain containing protein 4/BRD4 ), but not to traditional (non-MG) antagonists (JQ1, a competitive inhibitor of bromodomain containing protein 4/BRD4 ) of the respective POI (Figure 7). The observable shift in efficacy was at least 10-fold and up to 1000-fold, thus providing excellent signal to noise ratios. In sum, neddylation-impaired (NEDD^{mut}) cell line can be applied to find novel, yet uncharacterized MGs. This is further exemplified when said cells are treated with the compound CC-885 ( a molecular glue that reprograms the substrate receptor cereblon/CRBN to degrade GSPT1), or the PROTAC^{®} compound THAL-SNS-032 (known to reprogram the substrate receptor cereblon/CRBN to degrade the cyclin dependent kinase 9, CDK9) (both see appended Figure 22). Again, these data show that modulating the activity of the COP9 signalosome subunit 8 (COPS8) or CAND1 (Cullin-associated NEDD8-dissociated protein 1; a substrate receptor exchange factor) leads to a destabilization of the reprogrammed substrate receptors (CRBN for CC-885, THAL-SNS-032 and dBET6; DCAF15 for indisulam) and, as a consequence, to reduced activity of compounds that act by re-directing the activity if said ligases.

However, not for every cullin RING ligase (CRL), modifications of the COP9 signalosome subunit 8 (COPS8) or CAND1 (Cullin-associated NEDD8-dissociated protein 1; a substrate receptor exchange factor) lead to an ensuing destabilization via auto-degradation. An example for this is the substrate receptor VHL(von-Hippel-Lindau), which is part of the CRL2^{VHL} ligase complex. As shown in the appended Figure 1B, increasing cellular neddylation levels either by modification of COPS8, or by modification of CAND1 does not lead to a measurable (auto-) ubiquitination and ensuing destabilization of VHL in KBM7 leukemia cells compared to non-modified control cells. Consequently, said COPS8 and CAND1-modified cells do not display an altered sensitivity to the PROTAC^{®} compound ARV-771 (reprogramming the substrate receptor VHL to degrade the bromodomain containing protein 4/BRD4; see appended Figure 22). Therefore, measuring differential sensitivity of cells manipulated to contain altered neddylation states to a molecular glue/PROTACO compound is a feasible surrogate to measure whether the substrate receptor that is reprogrammed by said molecular glue/PROTAC^{®} undergoes destabilization/auto-degradation upon altering the neddylation states.

### EXAMPLE 5

### Mapping the inventory of accessible CRL substrate receptors

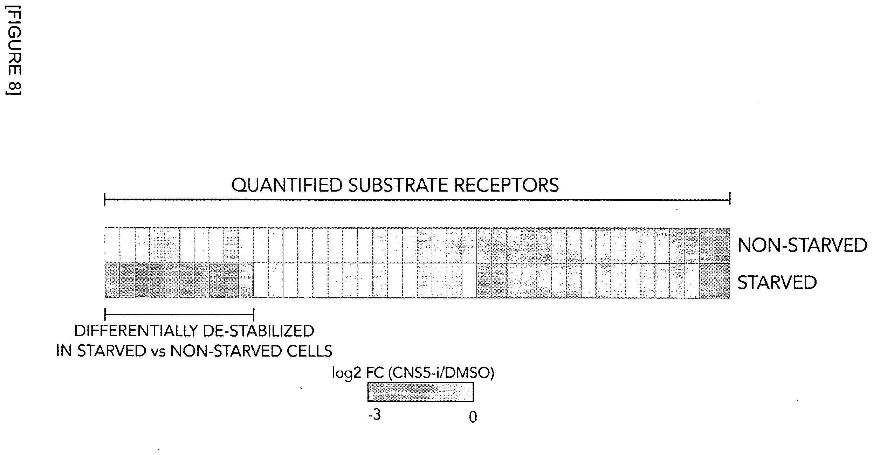

To identify all substrate receptors that were destabilized upon CSN (COP9 signalosome, the deneddylation complex) perturbation, unbiased, quantitative proteomics was employed (see also Example 1, in particular Figures 1G to 1I). Here, we additionally set out to show that the process of auto-degradation is dynamically controlled. Profiling was conducted in starved and stimulated 293T cells to further increase diversity of SR expression and activity (Figure 8). Experimentally, cells were treated with a pharmacologic inhibitor of the catalytic COP9-signalosome subunit CSN5 for 8 hours, followed by cellular lysis and global and unbiased quantification of differential protein abundance between treated and untreated samples using isobaric tagging/Tandem Mass Tag (TMT) labeling. This led to a complete inventory of CRLs that autodegrade their SRs in the absence of substrate as they were locked in a constitutively active state that did not allow the CRL complex to be decommissioned.

### EXAMPLE 6

### High-content chemical screens to discover MGs via substrate receptor stabilization

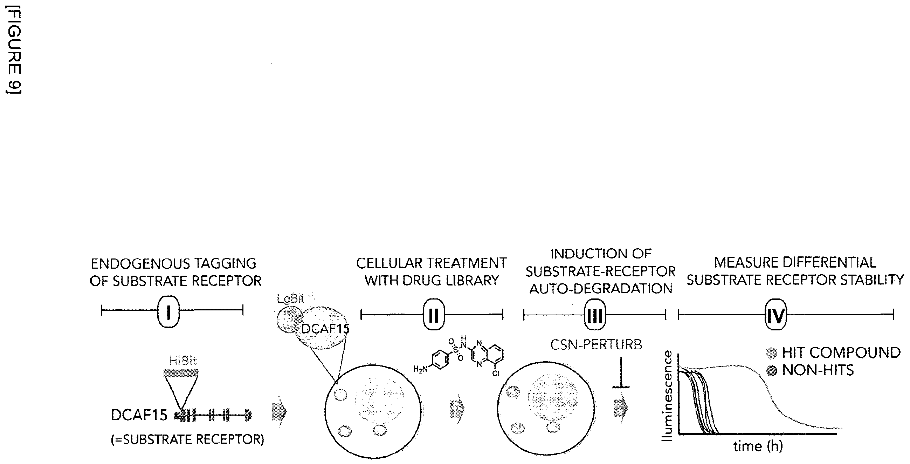

To devise a scalable approach that allows parallel tracing of thousands of drugs on abundance of substrate receptors, the recently developed HiBit technology is employed (see schematics in Figure 9 for the SR DCAF15). SR genes are tagged endogenously with the 11 AA HiBit peptide (Promega^{®}), which has picomolar affinity to a modified nanoluciferase ("LgBit") that depends on HiBit complementation for activity. Alternatively, HiBit-DCAF15 fusions was ectopically overexpressed via a lentiviral vector. LgBit is either stably expressed in tagged cells (continuous measurements over time), or added recombinantly after cell lysis (endpoint measurements). Tagging is conducted in 293T cells by transiently expressing Cas9 along with single-stranded DNA oligos encoding a HiBit sequence.

### EXAMPLE 7

### Autodegradation and Compound-dependent stabilization of substrate receptor DCAF15 using DCAF15 HiBit and a modified nano luciferase ("LgBit").

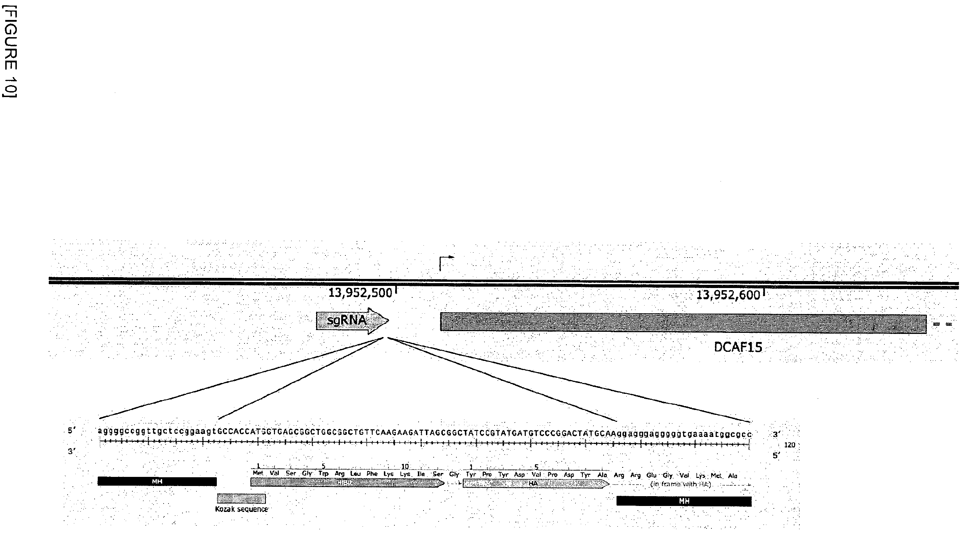

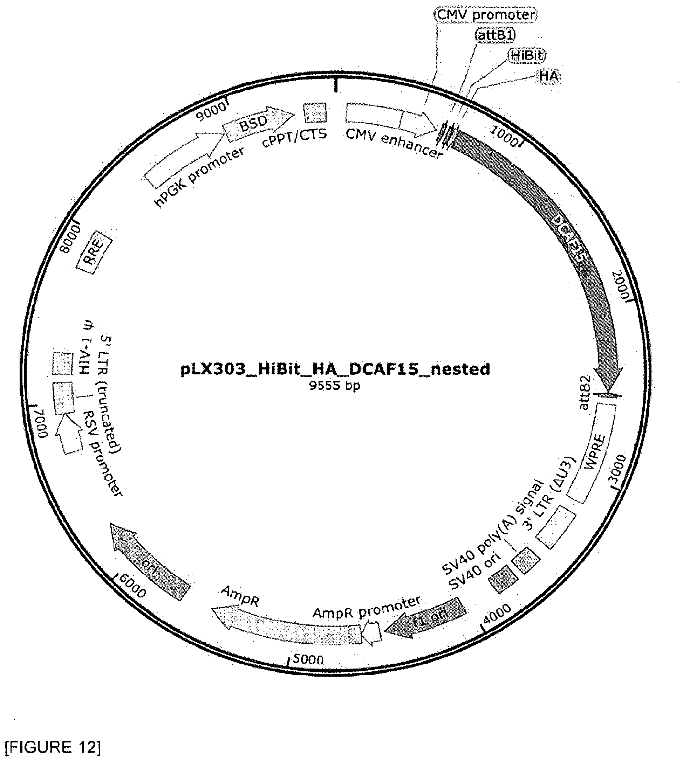

Generation of HiBit DCAF15 knock-in cells: HEK293t WT cells at 70 - 80% confluency were transiently transfected in a 6 well plate with a mix of 3 ug Cas9-sgRNA vector (PX458, addgene #48138, containing sgRNA 5'-ccggttgctccggaagtgga-3'), 6 ug of single stranded oligonucleotide donor (ssOND, 20 bp homology flanking the cut site) and 3.6 ug polyethylenimine (Figure 10 and 12). 72 hrs post-transfection cells were sorted (SH800 Cell sorter, SONY) for GFP positive cells and recovered for 24 hrs in media. Cells were subsequently seeded at a limiting dilution to generate single clones. Clones were tested after outgrowth for HiBit expression by seeding clonal cells in a black clear bottom 384 well plate and adding Nano-Glo HiBit Lytic buffer (Promega, N3030) reconstituted with recombinant LgBit and Nano-Glo HiBit Lytic Substrate in a 1:1 ratio. HiBit-expressing clones were validated by western blotting and detection of luciferase signal (Promega, N2410) at the correct size.

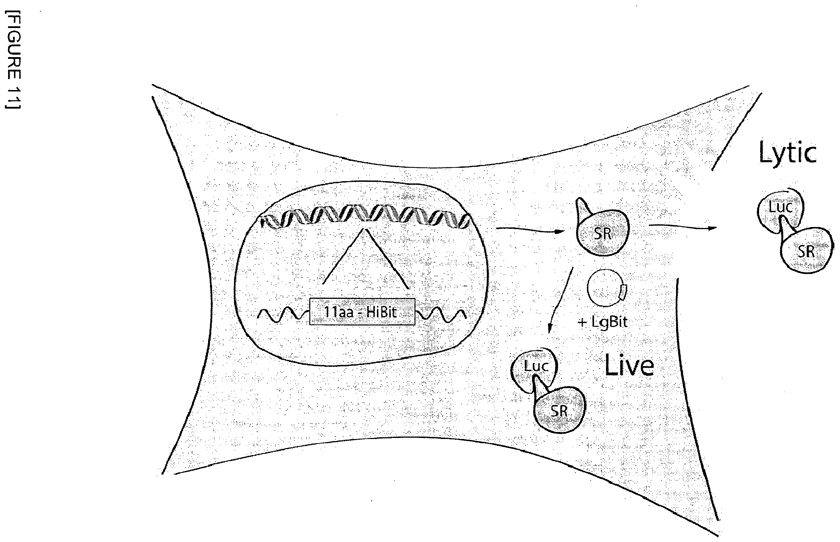

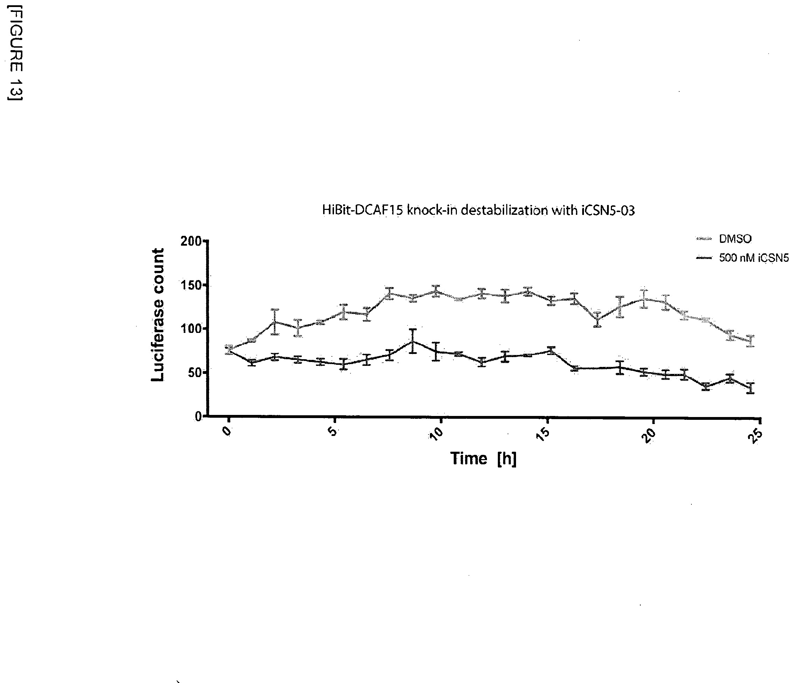

For live cell luciferase detection (Figure 11 and 13) 7000 HEK293t cells with a HiBit knock-in at the N-terminus of DCAF15 and stable ectopic expression of LgBit were seeded in 10 uL per well into a black, clear-bottom 384 well plate (Corning, #3764) and incubated over night. 20 uL of media containing 1 X Endurazine (Promega, N2570) was added to each well and incubated for 1 hour followed by a baseline luciferase measurement using an EnVision plate reader (Perkin Elmer).

Subsequently CSN5i-3 or an equal volume of DMSO was added in 10 uL of media to a final concentration of 500 nM in the assay volume of 40 uL. Luciferase signal was measured hourly for 24 hours.

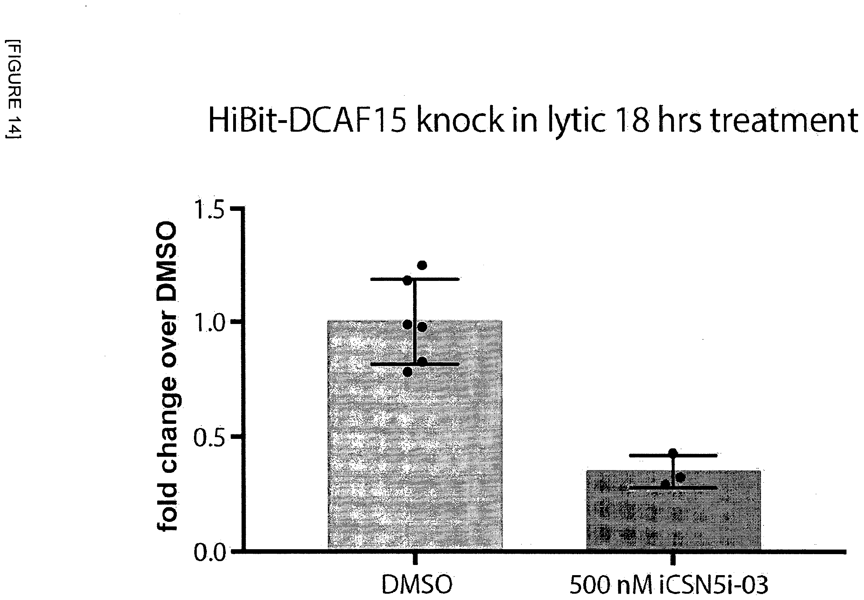

For lytic HiBit detection (Figure 11 and 14) 10000 cells were seeded in 10 uL per well into a black clear bottom 384 well plate. After recovery cells were treated by addition of iCSN5-3 or an equal volume of DMSO in 10 uL of media to the cells. After the indicated treatment time cells were lysed by adding 20 uL Nano-Glo HiBit Lytic buffer (Promega, N3030) reconstituted with recombinant LgBit and Nano-Glo HiBit Lytic Substrate. Luciferase signal of each well was normalized to the mean DMSO luciferase signal yielding fold-change over DMSO.

Luciferase signal was detected essentially as stated for Fig.13 with the exception that indisulam (Sigma-Aldrich, SML1225) was added together with CSN5i-3 to cells in 10 uL of media to reach the indicated final concentration (Figure 15). Luciferase signal of each well and timepoint was normalized to the mean DMSO luciferase signal yielding fold-change over DMSO. Of note, this data shows that indisulam treatment rescues DCAF15 from auto-degradation caused by COPS5 inhibition via CSN5i-3 .

Luciferase signal was detected essentially as stated for Fig. 15 with the exception that indisulam and dCEMM.1 were added together with iCSN5-3 to cells in the 10 uL treatment to a final concetration of 10 uM (Figure 17). Luciferase signal of each well and timepoint was normalized to the mean DMSO luciferase signal yielding fold-change over DMSO. Of note, this data shows that indisulam treatment, but also treatment with the novel compound dCeMM.1 rescues DCAF15 from auto-degradation caused by COPS5 inhibition via CSN5i-3 .

### EXAMPLE 8

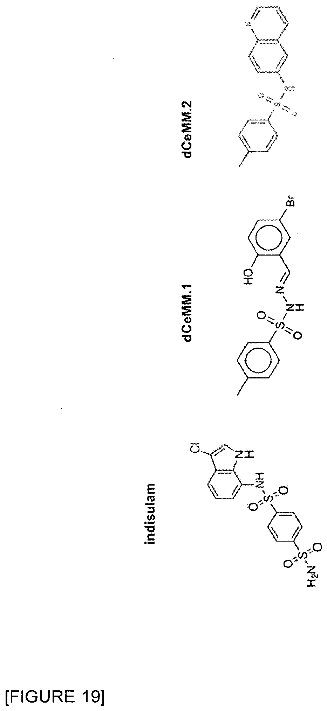

Subsequently, in this context, the E3 ligase substrate receptor (SR) stabilization approach was employed in order to identify novel chemical matter that can bind and chemically re-program the substrate receptor DCAF15. To be able to screen a collection of around 8000 small-molecules, a cellular system was developed that enabled monitoring of DCAF15 levels in live cells in multi-well format. To that end, DCAF15 was first knocked out in 293T cells via CRISPR/Cas9 technology. Then, DCAF15 was stably expressed as a HiBit^{®} fusion protein in these cells. After further transducing these cells with LgBit, this allowed live-cell tracing of DCAF15 levels in 384 well format by measuring bioluminescence (Figure 18). In brief, cells were seeded at a concentration of 250'000 cells/ml in DMEM+10%FCS+25mM Hepes. The luciferase substrate Endurazine was added at concentrations recommended by the manufacturer. Small-molecules were added at a concentration of 10 uM, and their effect on DCAF15 levels was assayed by continuous bioluminescent imaging (measurements were taken every 120 minutes). In total, 8000 compounds were tested (Figure 18 only shows only a subset of the 8000 tested molecules. In detail, 200 negative controls=compounds that did not appear to cause DCAF15 stabilization are displayed.). The known DCAF15 molecular glue indisulam (see Figure 7) was used as a positive control. In brief, indisulam is known to act as a DCAF15-dependent molecular glue that binds DCAF15 and induced proximity between DCAF15 and RBM39, leading to the degradation of the latter. As predicted, treatment with indisulam led to a pronounced stabilization of DCAF15 over time. Of note, two additional molecules (dCeMM.1 and dCeMM.2) were also identified to prompt significant DCAF15 stabilization. The chemical structure of dCeMM.1 and dCeMM.2 alongside indisulam are shown in Figure 19. Interestingly, both test compounds feature an aryl sulfonamide structure similar to indisulam.

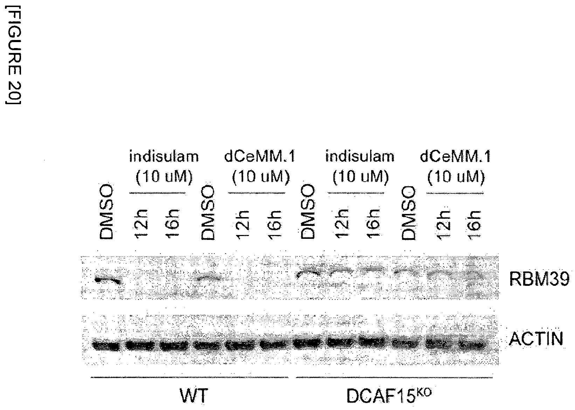

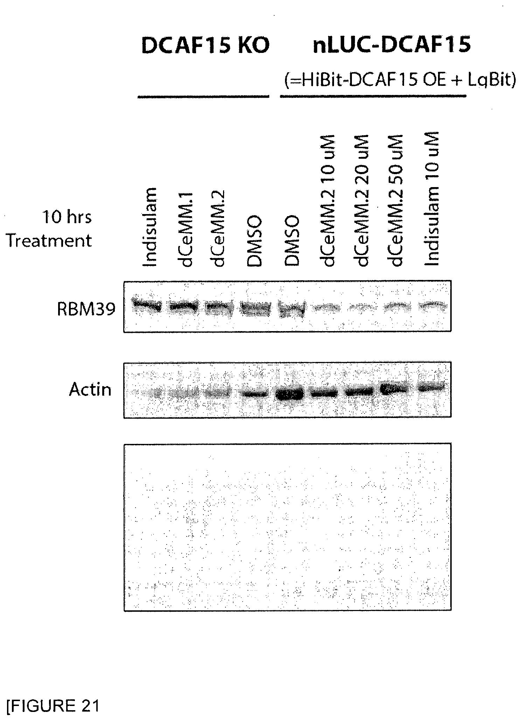

Given the structural similarity to indisulam, it was assayed if the two new test compounds would similarly lead to the degradation of RBM39 in a DCAF15-dependent manner. Towards that end, their effect on RBM39 levels in wildype cells as well as cells deficient for DCAF15 levels was tested. KBM7 (WT or knockout, Figure 20) as well as 293T cells (DCAF15-HiBit overexpression vs knockout, see Figure 21) were tested in various conditions of dCEMM.1 and/or dCEMM.2, comparing their effect to indisulam.

In sum, in a screening of multiple compounds for their ability to redirect the ligase DCAF15, a known positive control (such as indisulam) as well as compounds with unknown annotated function, e.g. the compounds denoted as dCeMM.1 and dCeMM.2, have been identified as positive hits, i.e. compounds that act in a DCAF15-dependent manner binding to DCAF15 and inducing proximity between DCAF15 and (a) target protein(s) such as RBM39, leading to the degradation of the target protein(s).

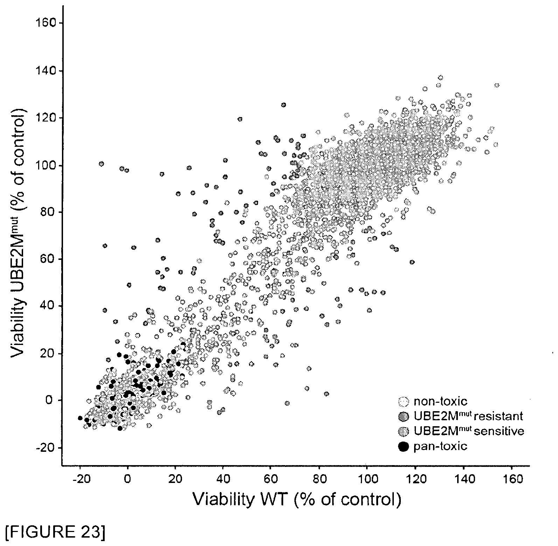

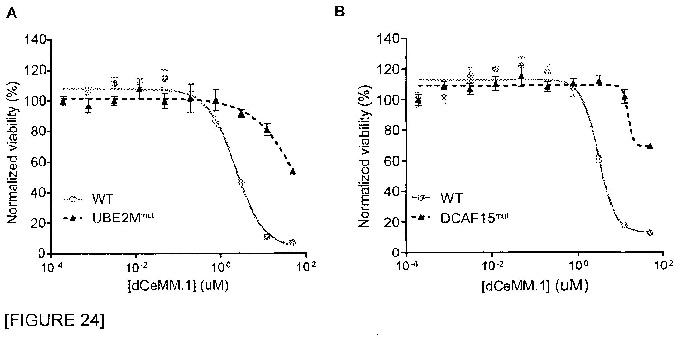

In another experiment, 2000 compounds were screened in a set up comparing their anti-proliferative effect in KBM7 cells modified in the UBE2M gene to wildtype KBM7 cells (Figure 23). The PROTAC^{®} ARV-771 was taken as positive control. Of note, among the identified hits was also dCeMM.1, which was similarly identified via the aforementioned DCAF15 restabilization assay. Figure 24 shows that dCeMM.1 loses anti-proliferative activity in KBM7 cells mutant for UBE2M as compared to KBM7 WT cells (24A). To further ascertain that the shift in viability is dependent on DCAF15, we also showed that inactivitation of DCAF15 in KBM7 cells lead to a pronounced resistance to dCeMM.1 as compared to KBM7 WT cells.

## Claims

1. A method for identifying a chemical compound or agent able to induce ubiquitination of a protein of interest,
the method comprising:
(i) contacting a chemical compound or agent of interest with an isolated eukaryotic cell comprising and/or expressing the protein of interest and manipulating said cell to comprise enhanced cullin-RING ubiquitin ligase (CRL) activity;
(ii) determining the level of at least one member of an E3 ligase complex present in the cell;
wherein said chemical compound or agent is identified to induce ubiquitination of the protein of interest if the level of the at least one member of the E3 ligase complex is increased compared to the level determined in the absence of said chemical compound or agent of interest.

2. The method of claim 1, wherein in step (i) said cullin-RING ubiquitin ligase (CRL) activity is enhanced compared to the cullin-RING ubiquitin ligase (CRL) activity in a control cell that is not manipulated to comprise enhanced CRL activity.

3. The method of claim 1 or 2, wherein said cell manipulated to comprise enhanced cullin-RING ubiquitin ligase (CRL) activity is a cell manipulated to comprise constantly neddylated cullin-RING ubiquitin ligases/CRLs and/or that is manipulated to comprise constant neddylation of cullin-RING ubiquitin ligases/CRLs.

4. The method of anyone of claims 1 to 3, wherein the cell manipulated to comprise enhanced cullin-RING ubiquitin ligase (CRL) activity and/or to comprise constantly neddylated cullin-RING ubiquitin ligases/CRLs is manipulated by inactivation of a target protein like a Cullin-associated NEDD8-dissociated protein selected from the group consisting of CAND1, CAND2 or CAND1 and CAND2, by inactivation of (a) target protein(s) being at least one member of the COP9 signalosome (CSN) and/or by inactivation of the COP9 signalosome (CSN), preferably wherein said inactivation of the COP9 signalosome (CSN) comprises the inactivation of the COPS5 subunit, the COPS8 subunit and/or of the COPS7B subunit.

5. The method of claim 4, wherein said inactivation comprises an inactivation by recombinant means and/or a hypomorphic mutation leading to a decrease of activity and/or abundance of the corresponding target protein or comprises the inactivation via a chemical compound/chemical inhibitor, preferably wherein said target protein is selected from the group consisting of CAND1, CAND2 or a protein member of the COP9 signalosome (CSN).

6. The method of any one of claims 4 or 5 wherein said inactivation comprises the step of contacting the cell to be manipulated to comprise enhanced cullin-RING ubiquitin ligase (CRL) activity and/or to comprise constantly neddylated cullin-RING ubiquitin ligases/CRLs with an inhibitor of the COP9 signalosome (CSN), preferably wherein said inhibitor of the COP9 signalosome (CSN) is an inhibitor of the COPS5 subunit, preferably CSN5i-3.

7. The method of anyone of claims 1 to 3, wherein the cell manipulated to comprise enhanced cullin-RING ubiquitin ligase (CRL) activity and/or to comprise constantly neddylated cullin-RING ubiquitin ligases/CRLs is manipulated by overexpressing at least one protein able to induce neddylation of the cullin-RING ubiquitin ligases/CRLs, preferably, whereby said at least one protein to be overexpressed is at least one member of the E3 ligase complex and is selected from
(i) a cullin/cullin backbone protein/cullin homolog selected from the group consisting of CUL4A, CUL4B, CUL1, CUL2, CUL3, CUL5, CUL6 and CUL7; preferably selected from human CUL4A, human CUL4B, human CUL1, human CUL2, human CUL3, human CUL5, and human CUL7, more preferably selected from the group consisting of human CUL4A and human CUL4B;
(ii) an adaptor protein selected from the group consisting of DBB1, SKP1, preferably DDB1;
(iii) a ligase substrate receptors selected from the group consisting of cereblon (CRBN), DCAF15, DCAF16, DCAF1, DCAF5, DCAF8, DET1, FBXO7, FBXO22, KDM2A, KDM2B, preferably cereblon (CRBN) or DCAF15;
(iv) an E1 activating enzyme that is NAE1, UBA3 subunit or a heterodimer of NAE1 and UBA3 subunit; and
(v) an E2 conjugating enzyme that is EBc12 or UBE2M, preferably UBE2M.

8. The method of anyone of claims 3 to 7, wherein said constantly neddylated cullin-RING ubiquitin ligases/CRLs and/or said constant neddylation of cullin-RING ubiquitin ligases/CRLs leads to destabilization and/or auto-degradation of at least one member of said CRL, preferably, wherein said constant neddylation leads to destabilization and/or auto-degradation of a substrate receptor of the CRL.

9. The method of claim any one of claims 1 to 8, wherein the protein of interest is selected from the group consisting DNA-binding proteins including transcription factors, RNA binding proteins, scaffolding proteins, GTPases, solute carriers, kinases, phosphatases, bromodomain- and chromodomain containing proteins, and G-protein coupled receptors.

10. The method of any of the preceding claims, wherein cullin-RING ubiquitin ligase (CRL) or the cullin-RING ubiquitin ligase complex comprises CUL4A, CUL4B, CUL1, CUL2, CUL3, CUL5, CUL6 and/or CUL7, preferably human CUL4A, human CUL4B, human CUL1, human CUL2, human CUL3, human CUL5 and/or human CUL7, more preferably human CUL4A and/or human CUL4B.

11. The method of any of the preceding claims, wherein the chemical compound or agent to be identified is a chemical compound or agent that has been predetermined to bind to the protein of interest or to bind to the protein of interest if the chemical compound or agent simultaneously binds to the protein of interest and a second protein, wherein the second protein preferably comprises a member of the E3 ligase complex.

12. The method of any of the preceding claims, wherein the chemical compound or agent to be identified is a peptide, a small molecule, or a molecule having a size between 0.5 and 2 kDa, preferably between 0.8 and 1.5 kDa,
wherein the small molecule or molecule having a size between 0.5 and 2 kDa, preferably between 0.8 and 1.5 kDa comprises a first moiety binding the protein of interest and a second binding moiety, wherein the first and the second binding moiety are optionally connected by a linker.

## Patentansprüche

1. Verfahren zur Identifizierung einer chemischer Verbindung oder eines Mittels, die/das fähig ist, die Ubiquitinierung eines Proteins von Interesse zu induzieren, wobei das Verfahren Folgendes umfasst:
(i) Inkontaktbringen einer chemischen Verbindung oder eines Mittels von Interesse mit einer isolierten eukaryotischen Zelle, die das Protein von Interesse umfasst und/oder exprimiert, und Behandeln dieser Zelle derart, dass sie eine verstärkte Aktivität der Cullin-RING-Ubiquitin-Ligase (CRL) aufweist;
(ii) Bestimmen des Gehalts an mindestens einem Bestandteil eines E3-Ligase-Komplexes, der in der Zelle vorliegt;
wobei die chemische Verbindung oder das Mittel als die Ubiquitinierung des Proteins von Interesse induzierend identifiziert wird, wenn der Gehalt an dem mindestens einen Mitglied des E3-Ligase-Komplexes im Vergleich zu dem Gehalt, wie er in Abwesenheit der chemischen Verbindung oder des Mittels von Interesse bestimmt wurde, erhöht ist.

2. Verfahren nach Anspruch 1, wobei im Schritt (i) die Aktivität der Cullin-RING-Ubiquitin-Ligase (CRL) im Vergleich zur Aktivität der Cullin-RING-Ubiquitin-Ligase (CRL) in einer Kontrollzelle, die nicht derart behandelt wird, dass sie eine verstärkte CRL-Aktivität aufweist, verstärkt ist.

3. Verfahren nach Anspruch 1 oder 2, wobei es sich bei der Zelle, die derart behandelt wird, dass sie eine verstärkte Aktivität der Cullin-RING-Ubiquitin-Ligase aufweist, um eine Zelle handelt, die derart behandelt wird, dass sie konstant neddylierte Cullin-RING-Ubiquitin-Ligasen/CRLs aufweist, und/oder derart behandelt wird, dass darin eine konstante Neddylierung von Cullin-RING-Ubiquitin-Ligasen/CRLs erfolgt.

4. Verfahren nach einem der Ansprüche 1 bis 3, wobei die Behandlung der Zelle, die derart behandelt wird, dass sie eine verstärkte Aktivität der Cullin-RING-Ubiquitin-Ligase (CRL) aufweist und/oder dass sie konstant neddylierte Cullin-RING-Ubiquitin-Ligasen/CRLs umfasst, erfolgt, indem ein Zielprotein wie ein Cullin-assoziiertes NEDD8-dissoziiertes Protein, das aus der Gruppe ausgewählt ist, bestehend aus CAND1, CAND2 oder CAND1 und CAND2, inaktiviert wird, indem (ein) Zielprotein(e), bei dem/denen es sich um mindestens einen Bestandteil des COP9-Signalosoms (CSN) handelt, inaktiviert wird/werden, und/oder indem das COP9-Signalosom (CSN) inaktiviert wird, wobei im Rahmen der Inaktivierung des COP9-Signalosoms (CSN) vorzugsweise die COPS5-Untereinheit, die COPS8-Untereinheit und/oder die COPS7B-Untereinheit inaktiviert werden.

5. Verfahren nach Anspruch 4, wobei im Rahmen der Inaktivierung eine Inaktivierung durch rekombinante Mittel und/oder durch eine hypomorphe Mutation erfolgt, was eine Abnahme der Aktivität und/oder Menge des entsprechenden Zielproteins bewirkt, oder die Inaktivierung mittels einer chemischen Verbindung / eines chemischen Hemmstoffs erfolgt, wobei das Zielprotein aus der Gruppe ausgewählt ist, bestehend aus CAND1, CAND2 oder einem Protein, das Bestandteil des COP9-Signalosoms (CSN) ist.

6. Verfahren nach einem der Ansprüche 4 oder 5, wobei die Inaktivierung den Schritt umfasst, in dem die Zelle, die derart behandelt werden soll, dass sie eine verstärkte Aktivität der Cullin-RING-Ubiquitin-Ligase (CRL) aufweist und/oder dass sie konstant neddylierte Cullin-RING-Ubiquitin-Ligasen/CRLs aufweist, mit einem Hemmer der COP9-Signalosoms (CSN) in Kontakt gebracht wird, wobei es sich bei dem Hemmer des COP9-Signalosoms (CSN) vorzugsweise um einen Hemmer der COPS5-Untereinheit, vorzugsweise von CSN5i-3, handelt.

7. Verfahren nach einem der Ansprüche 1 bis 3, wobei die Zelle, die derart behandelt wird, dass sie eine verstärkte Aktivität der Cullin-RING-Ubiquitin-Ligase aufweist und/oder dass sie konstant neddylierte Cullin-RING-Ubiquitin-Ligasen/CRLs aufweist, durch Überexpression mindestens eines Proteins behandelt wird, das dazu befähigt ist, eine Neddylierung der Cullin-RING-Ubiquitin-Ligasen/CRLs zu induzieren, wobei es sich bei mindestens einem Protein, das überexprimiert werden soll, vorzugsweise um mindestens einen Bestandteil des E3-Ligase-Komplexes handelt und es aus den folgenden ausgewählt ist
(i) einem Cullin- / Cullingerüst-Protein / Cullin-Homologon, das aus der Gruppe ausgewählt ist, bestehend aus CUL4A, CUL4B, CUL1, CUL2, CUL3, CUL5, CUL6 und CUL7; wobei es vorzugsweise aus menschlichem CUL4A, menschlichem CUL4B, menschlichem CUL1, menschlichem CUL2, menschlichem CUL3, menschlichem CUL5 und menschlichem CUL7 ausgewählt ist, wobei es insbesondere aus der Gruppe ausgewählt ist, bestehend aus menschlichem CUL4A und menschlichem CUL4B;
(ii) einem Adapter-Protein, das aus der Gruppe ausgewählt ist, bestehend aus DBB1, SKP1, wobei es sich vorzugsweise um DDB1 handelt;
(iii) einem Ligase-Substrat-Rezeptor, der aus der Gruppe ausgewählt ist, bestehend aus Cereblon (CRBN), DCAF15, DCAF16, DCAF1, DCAF5, DCAF8, DET1, FBXO7, FBXO22, KDM2A, KDM2B, wobei es sich vorzugsweise um Cereblon (CRBN) oder DCAF15 handelt;
(iv) einem E1-aktivierenden Enzym, wobei es sich um NAE1, eine UBA-Untereinheit oder einer Heterodimer aus NAE1 und einer UBA3-Untereinheit handelt; und
(v) einem E2-konjugierenden Enzym, wobei es sich um EBc12 oder UBE2M, vorzugsweise um UBE2M handelt.

8. Verfahren nach einem der Ansprüche 3 bis 7, wobei die konstant neddylierten Cullin-RING-Ubiquitin-Ligasen/CRLs und/oder die konstante Neddylierung von Cullin-RING-Ubiquitin-Ligasen/CRLs eine Destabilisierung und/oder einen selbsttätigen Abbau von mindestens einem Bestandteil der CRL bewirkt, wobei vorzugsweise die konstante Neddylierung eine Destabilisierung und/oder einen selbsttätigen Abbau eines Substrat-Rezeptors der CRL bewirkt.

9. Verfahren nach einem der Ansprüche 1 bis 8, wobei das Protein von Interesse aus der Gruppe ausgewählt ist, bestehend aus an DNA bindenden Proteinen, wobei dazu Transkriptionsfaktoren, an RNA bindende Proteine, gerüstbildende Proteine, GTPasen, Träger gelöster Stoffe, Kinasen, Phosphatasen, Proteine, die Bromodomänen und Chromodomänen umfassen, sowie G-Protein-gekoppelte Rezeptoren gehören.

10. Verfahren nach einem der vorhergehenden Ansprüche, wobei die Cullin-RING-Ubiquitin-Ligase (CRL) oder der Cullin-RING-Ubiquitin-Ligase-Komplex CUL4A, CUL4B, CUL1, CUL2, CUL3, CUL5, CUL6 und/oder CUL7, vorzugsweise menschliches CUL4A, menschliches CUL4B, menschliches CUL1, menschliches CUL2, menschliches CUL3, menschliches CUL5 und/oder menschliches CUL7, insbesondere menschliches CUL4A und/oder menschliches CUL4B umfassen.

11. Verfahren nach einem der vorhergehenden Ansprüche, wobei die Verbindung oder das Mittel, die/das identifiziert werden soll, eine chemische Verbindung oder ein Mittel ist, von der/dem im Vorfeld festgestellt worden ist, dass sie/es an das Protein von Interesse bindet oder an das Protein von Interesse bindet, wenn die chemische Verbindung oder das Mittel gleichzeitig an das Protein von Interesse und ein zweites Protein bindet, wobei das zweite Protein vorzugsweise einen Bestandteil des E3-Ligase-Komplexes umfasst.

12. Verfahren nach beliebigen der vorhergehenden Ansprüche, wobei die Verbindung oder das Mittel, die/das identifiziert werden soll, ein Peptid, ein kleines Molekül oder ein Molekül ist, dessen Größe zwischen 0,5 und 2 kDa, vorzugsweise zwischen 0,8 und 1,5 kDa liegt, wobei das kleine Molekül oder das Molekül, dessen Größe zwischen 0,5 und 2 kDa, vorzugsweise zwischen 0,8 und 1,5 kDa liegt, einen ersten Baustein, der an das Protein von Interesse bindet, und einen zweiten Bindungsbaustein umfasst, wobei der erste und der zweite Bindungsbaustein möglicherweise mittels eines Linkers verbunden sind.

## Revendications

1. Procédé d'identification d'un composé chimique ou d'un agent qui est capable d'induire l'ubiquitination d'une protéine d'intérêt,
le procédé comprenant :
(i) la mise en contact d'un composé chimique ou d'un agent d'intérêt avec une cellule eucaryote isolée comprenant et/ou exprimant la protéine d'intérêt et la manipulation de ladite cellule pour comprendre une activité culline-RING ubiquitine ligase (CRL) augmentée ;
(ii) la détermination du niveau d'au moins un membre d'un complexe E3 ligase présent dans la cellule ;
ledit composé chimique ou agent étant identifié comme induisant l'ubiquitination de la protéine d'intérêt si le niveau de l'au moins un membre du complexe d'E3 ligase est augmenté par rapport au niveau déterminé en absence dudit composé chimique ou agent d'intérêt.

2. Procédé selon la revendication 1, dans lequel dans l'étape (i) ladite activité culline-RING ubiquitine ligase (CRL) est augmentée par rapport à l'activité culline-RING ubiquitine ligase (CRL) dans une cellule témoin qui n'est pas manipulée pour comprendre une activité CRL augmentée.

3. Procédé selon la revendication 1 ou 2, dans lequel ladite cellule manipulée pour comprendre une activité culline-RING ubiquitine ligase (CRL) augmentée est une cellule manipulée pour comprendre des culline-RING ubiquitine ligases/CRL constamment neddylées et/ou qui est manipulée pour comprendre la neddylation constante des culline-RING ubiquitine ligases/CRL.

4. Procédé selon l'une quelconque des revendications 1 à 3, dans lequel la cellule manipulée pour comprendre une activité culline-RING ubiquitine ligase (CRL) augmentée et/ou pour comprendre des culline-RING ubiquitine ligases/CRL constamment neddylées est manipulée par l'inactivation d'une protéine cible comme une protéine associée à la culline dissociée de NEDD8 choisie dans le groupe constitué par CAND1, CAND2 ou CAND1 et CAND2, par inactivation d'une(de) protéine(s) cible(s) étant au moins un membre du signalosome COP9 (CSN) et/ou par inactivation du signalosome COP9 (CSN), de préférence ladite inactivation du signalosome COP9 (CSN) comprenant l'inactivation de la sous-unité COPS5, de la sous-unité COPS8 et/ou de la sous-unité COPS7B.

5. Procédé selon la revendication 4, dans lequel ladite inactivation comprend une inactivation par des moyens recombinants et/ou une mutation hypomorphe conduisant à une diminution de l'activité et/ou de l'abondance de la protéine cible correspondante ou comprend l'inactivation via un composé chimique/inhibiteur chimique, de préférence ladite protéine cible étant choisie dans le groupe constitué par CAND1, CAND2 ou un membre protéique du signalosome COP9 (CSN).

6. Procédé selon l'une quelconque des revendications 4 ou 5 dans lequel ladite inactivation comprend l'étape de mise en contact de ladite cellule à manipuler pour comprendre l'activité culline-RING ubiquitine ligase augmentée et/ou pour comprendre les culline-RING ubiquitine ligases/CRL constamment neddylées avec un inhibiteur du signalosome COP9 (CSN), de préférence ledit inhibiteur du signalosome COP9 (CSN) étant un inhibiteur de la sous-unité COPS5, de préférence CSN5i-3.

7. Procédé selon l'une quelconque des revendications 1 à 3, dans lequel la cellule manipulée pour comprendre l'activité culline-RING ubiquitine ligase (CRL) augmentée et/ou pour comprendre les culline-RING ubiquitine ligases/CRL constamment neddylées est manipulée par la surexpression d'au moins une protéine capable d'induire la neddylation des culline-RING ubiquitine ligases/CRL, de préférence, moyennant quoi ladite au moins une protéine à surexprimer est au moins un membre du complexe E3 ligase et est choisie parmi
(i) un homologue de protéine/culline à squelette culline/culline choisi dans le groupe constitué par CUL4A, CUL4B, CUL1, CUL2, CUL3, CUL5, CUL6 et CUL7 ; de préférence choisi parmi la CUL4A humaine, la CUL4B humaine, la CUL1 humaine, la CUL2 humaine, la CUL3 humaine, la CUL5 humaine et la CUL7 humaine, davantage de préférence choisi dans le groupe constitué par la CUL4A humaine et la CUL4B humaine ;
(ii) une protéine adaptatrice choisie dans le groupe constitué par DBB1, SKP1, de préférence DDB1 ;
(iii) un récepteur de substrat de ligase choisi dans le groupe constitué par le céréblon (CRBN), DCAF15, DCAF16, DCAF1, DCAF5, DCAF8, DET1, FBXO7, FBXO22, KDM2A, KDM2B, de préférence le céréblon (CRBN) ou DCAF15 ;
(iv) une enzyme activatrice d'E1 qui est NAE1, la sous-unité UBA3 ou un hétérodimère de NAE1 et de la sous-unité UBA3 ; et
(iv) une enzyme conjuguant E2 qui est Ebc12 ou UBE2M, de préférence UBE2M.

8. Procédé selon les revendications 3 à 7, dans lequel lesdites culline-RING ubiquitine ligases/CRL constamment neddylées et/ou ladite neddylation constante des culline-RING ubiquitine ligases/CRL conduit à la déstabilisation et/ou l'auto-dégradation d'au moins un membre de ladite CRL, de préférence, ladite neddylation constante conduisant à la déstabilisation et/ou l'auto-dégradation d'un récepteur de substrat de la CRL.

9. Procédé selon l'une quelconque des revendications 1 à 8, dans lequel la protéine d'intérêt est choisie dans le groupe constitué par les protéines de liaison à l'ADN comprenant les facteurs de transcription, les protéines de liaison à l'ARN, les protéines d'échafaudage, les GTPases, les transporteurs de soluté, les kinases, les phosphatases, les protéines contenant un bromodomaine et un chromodomaine et les récepteurs couplés à la protéine G.

10. Procédé selon l'une quelconque des revendications précédentes, dans lequel la culline-RING ubiquitine ligase (CRL) ou le complexe de culline-RING ubiquitine ligase comprend CUL4A, CUL4B, CUL1, CUL2, CUL3, CUL5, CUL6 et/ou CUL7, de préférence la CUL4A humaine, la CUL4B humaine, la CUL1 humaine, la CUL2 humaine, la CUL3 humaine, la CUL5 humaine et/ou la CUL7 humaine, davantage de préférence la CUL4A humaine ou la CUL4B humaine.

11. Procédé selon l'une quelconque des revendications précédentes, dans lequel le composé chimique ou l'agent à identifier est un composé chimique ou un agent qui a été prédéterminé pour se lier à la protéine d'intérêt ou pour se lier à la protéine d'intérêt si le composé chimique ou l'agent se lie simultanément à la protéine d'intérêt et à une seconde protéine, la seconde protéine comprenant de préférence un membre du complexe E3 ligase.

12. Procédé selon l'une quelconque des revendications précédentes, dans lequel le composé chimique ou l'agent à identifier est un peptide, une petite molécule ou une molécule ayant une taille comprise entre 0,5 et 2 kDa, de préférence entre 0,8 et 1,5 kDa, la petite molécule ou la molécule ayant une taille comprise entre 0,25 et 2 kDa, de préférence entre 0,8 et 1,5 kDa comprenant une première fraction se liant à la protéine d'intérêt et une seconde fraction de liaison, la première et la seconde fractions de liaison étant éventuellement connectées par un lieur.
